# EUROPEAN PATENT SPECIFICATION

(11) **EP 4 215 235 B1**
(45) Date of publication and mention of the grant of the patent: **08.10.2025**
(21) Application number: 23152560.1
(22) Date of filing: 20.01.2023
(51) Int. Cl.: A61M 16/00, A61M 16/06, A61M 16/08

(54) **STABILISING STRUCTURES FOR PATIENT INTERFACES**
STABILISIERUNGSSTRUKTUREN FÜR PATIENTENSCHNITTSTELLEN
STRUCTURES DE STABILISATION POUR INTERFACES PATIENT

(30) Priority: 20.01.2022 AU 2022900097; 06.05.2022 AU 2022901199; 22.12.2022 US 202218087532
(43) Date of publication of application: 26.07.2023
(73) Proprietor: ResMed Asia Pte. Ltd., Singapore 639443 (SG)
(72) Inventor: TAN, Beng Hai, 639443 Singapore (SG)
(74) Representative: Vossius & Partner Patentanwälte Rechtsanwälte mbB

(56) References cited:
- WO-A1-2019/183680
- WO-A1-2020/261139
- WO-A1-2021/012005

## Description

### 1 BACKGROUND OF THE TECHNOLOGY

### 1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hypoventilation Syndrome (OHS), Chronic Obstructive Pulmonary Disease (COPD), Neuromuscular Disease (NMD) and Chest wall disorders.

A range of therapies have been used to treat or ameliorate such conditions. Furthermore, otherwise healthy individuals may take advantage of such therapies to prevent respiratory disorders from arising. However, these have a number of shortcomings.

### 1.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 1.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

### 1.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 1.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

Certain masks may cause some patients a feeling of claustrophobia, unease and/or may feel overly obtrusive.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

Consequently, some masks suffer from being obtrusive, aesthetically undesirable, costly, poorly fitting, difficult to use, and/or uncomfortable especially when worn for long or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy, especially if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 1.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design is able to fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFTTM nasal pillows mask, SWIFTTM II nasal pillows mask, SWIFTTM LT nasal pillows mask, SWIFTTM FX nasal pillows mask and MIRAGE LIBERTYTM full-face mask. The following patent applications, assigned to ResMed Limited, describe examples of nasal pillows masks: International Patent Application WO2004/073,778 (describing amongst other things aspects of the ResMed Limited SWIFTTM nasal pillows), US Patent Application 2009/0044808 (describing amongst other things aspects of the ResMed Limited SWIFTTM LT nasal pillows); International Patent Applications WO 2005/063,328 and WO 2006/130,903 (describing amongst other things aspects of the ResMed Limited MIRAGE LIBERTYTM full-face mask); International Patent Application WO 2009/052,560 (describing amongst other things aspects of the ResMed Limited SWIFTTM FX nasal pillows).

### 1.2.3.1.2 Positioning and stabilising structure

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face. Several factors may be considered when comparing different positioning and stabilising techniques. These include: how effective the technique is at maintaining the seal-forming structure in the desired position and in sealed engagement with the face during use of the patient interface; how comfortable the interface is for the patient; whether the patient feels intrusiveness and/or claustrophobia when wearing the patient interface; and aesthetic appeal.

One technique is the use of adhesives. See for example US Patent Application Publication No. US 2010/0000534.

WO2020261139 describes a patient interface with a nasal seal. Another technique is the use of one or more straps and/or stabilising harnesses. Many such harnesses suffer from being one or more of ill-fitting, bulky, uncomfortable and awkward to use.

### 1.2.3.1.3 Pressurised Air Conduit

In one type of treatment system, a flow of pressurised air is provided to a patient interface through a conduit in an air circuit that fluidly connects to the patient interface at a location that is in front of the patient's face when the patient interface is positioned on the patient's face during use. The conduit may extend from the patient interface forwards away from the patient's face.

### 1.2.3.1.4 Pressurised Air Conduit used for Positioning / Stabilising the Seal-Forming Structure

Another type of treatment system comprises a patient interface in which a tube that delivers pressurised air to the patient's airways also functions as part of the headgear to position and stabilise the seal-forming portion of the patient interface at the appropriate part of the patient's face. This type of patient interface may be referred to as having "conduit headgear" or "headgear tubing". Such patient interfaces allow the conduit in the air circuit providing the flow of pressurised air from a respiratory pressure therapy (RPT) device to connect to the patient interface in a position other than in front of the patient's face. One example of such a treatment system is disclosed in US Patent Publication No. US 2007/0246043, in which the conduit connects to a tube in the patient interface through a port positioned in use on top of the patient's head.

It is desirable for patient interfaces incorporating headgear tubing to be comfortable for a patient to wear over a prolonged duration when the patient is asleep, form an air-tight and stable seal with the patient's face, while also able to fit a range of patient head shapes and sizes.

### 1.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

### 1.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 1.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

### 1.2.3.5 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

### 2 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

An aspect of certain forms of the present technology is to provide apparatus that improve the compliance of patients with respiratory therapy.

An aspect of the present technology is a patient interface for supplying a flow of air at a therapeutic pressure to a patient's airways. In certain forms, the patient interface may comprise a cushion module and a brace. The brace may be configured to brace against movement of portions of a seal-forming structure of the cushion module. Additionally, or alternatively, when the brace is mounted to a nasal portion of the cushion module, the nasal portion may be deformed relative to a natural shape of the nasal portion.

In certain forms, the brace may be constructed and configured to be more rigid than the nasal portion of the cushion. In certain forms, the brace is configured to mount to the nasal portion of the cushion and, in use, to brace against movement of portions of the seal-forming structure in sealing engagement with regions of the patient's face proximate the patient's nares. In certain forms, the brace is configured to deform a seal-forming surface of the nasal portion relative to a natural shape of the seal-forming surface of the nasal portion.

**In** certain forms, the cushion module may at least in part define a plenum chamber pressurisable to the therapeutic pressure and comprising a plenum chamber inlet port for receiving the flow of air into the plenum chamber. The cushion may comprise a seal-forming structure constructed and configured to form a seal with a region of the patient's face surrounding an entrance to the patient's nares, said seal-forming structure having an opening therein such that the flow of air at said therapeutic pressure is delivered to the patient's nares in use. The seal-forming structure may be constructed and configured to maintain said therapeutic pressure in the plenum chamber throughout the patient's respiratory cycle in use. The nasal portion may be comprised as part of the cushion and may be configured to be positioned, in use, proximate and anterior to a nasal and/or lip superior region of the patient's face. The cushion module may be configured to connect, in use, to a positioning and stabilising structure to provide a force to hold the seal-forming structure in a therapeutically effective position on the patient's head.

In certain forms, the brace may comprise a concave patient-facing surface facing towards the patient in use. The brace may be arc-shaped when viewed in a direction perpendicular to the transverse plane of the patient. The brace may be elongate and the brace may be configured to mount to the nasal portion of the cushion in an orientation such that the brace is elongate in the lateral direction with respect to the patient in use.

In certain forms, the seal-forming structure may be constructed and configured to form a seal with a region of the patient's face surrounding an entrance to the patient's mouth such that the flow of air is delivered to the patient's mouth in use.

In certain forms, the cushion module may comprise a frame, wherein the frame is constructed and configured to be more rigid than the nasal portion of the cushion. The frame may at least in part define the plenum chamber. The frame may comprise a frame opening forming the plenum chamber inlet port. The frame opening may be positioned in an anterior central region of the patient interface when worn.

In certain forms, the patient interface may comprise the positioning and stabilising structure to provide a force to hold the seal-forming structure in a therapeutically effective position on the patient's head. In certain forms, the positioning and stabilising structure may comprise one or more tubes configured to convey the flow of air at the therapeutic pressure from a connection port to the plenum chamber inlet port, wherein the connection port is positioned, in use, in a region of the patient's head superior to an otobasion superior of the patient's head.

An aspect of the technology is a patient interface for supplying a flow of air at a therapeutic pressure to a patient's airways. The patient interface may comprise a cushion module comprising a cushion, wherein the cushion is constructed and configured to be substantially flexible. The cushion module may at least in part define a plenum chamber pressurisable to the therapeutic pressure and comprising a plenum chamber inlet port for receiving the flow of air into the plenum chamber. The cushion may comprise a seal-forming structure constructed and configured to form a seal with a region of the patient's face surrounding an entrance to the patient's nares, said seal-forming structure having an opening therein such that the flow of air at said therapeutic pressure is delivered to the patient's nares in use, the seal-forming structure constructed and configured to maintain said therapeutic pressure in the plenum chamber throughout the patient's respiratory cycle in use. The cushion may comprise a nasal portion configured to be positioned, in use, proximate and anterior to a nasal and/or lip superior region of the patient's face, wherein the nasal portion comprises a seal-forming surface that is comprised as part of the seal-forming structure. The patient interface may further comprise a brace. In certain forms, when the brace is mounted to the nasal portion of the cushion module, the nasal portion may be deformed relative to a natural shape of the nasal portion. The nasal portion may be deformed so that an orientation of the seal-forming surface of the nasal portion is adjusted when the brace is mounted to the nasal portion.

Another aspect of the present technology is a patient interface system for supplying a flow of air at a therapeutic pressure to a patient's airways. In certain forms, the patient interface system may comprise a cushion module and a plurality of braces. The braces may be interchangeably mounted to the cushion module. The plurality of braces may be shaped and/or sized differently from each other. Each brace may be configured to brace against movement of portions of a seal-forming structure of the cushion module. In certain forms, when each brace is mounted to a nasal portion of the cushion module, the nasal portion is deformed relative to a natural shape of the nasal portion.

An aspect of the technology is a brace for use in a patient interface and/or patient interface system of another aspect of the technology. In certain forms, the brace may comprise a concave patient-facing surface facing towards the patient in use. The brace may be arc-shaped when viewed in a direction perpendicular to the transverse plane of the patient. The brace may be elongate and the brace may be configured to mount to the nasal portion of the cushion in an orientation such that the brace is elongate in the lateral direction with respect to the patient in use.

An aspect of the technology is a brace for a patient interface for supplying a flow of air at a therapeutic pressure to a patient's airways. The brace is elongate and is arc-shaped with a concave patient-facing surface and in use the brace is configured to mount to a nasal portion of a cushion in an orientation such that the brace is elongate in the lateral direction with respect to the patient, is arc-shaped when viewed in a direction perpendicular to the transverse plane of the patient, and the concave patient-facing surface faces towards the patient in use.

An aspect of the present technology is a patient interface for supplying a flow of air at a therapeutic pressure to a patient's airways. In certain forms, the patient interface may comprise a cushion module and an adaptor. When the adaptor is mounted to a nasal portion of the cushion module, the nasal portion may be deformed relative to a natural shape of the nasal portion. Additionally, the adaptor may be configured to brace against movement of portions of a seal-forming structure of the cushion module.

An aspect of one form of the present technology is a positioning and stabilising structure for a patient interface, the positioning and stabilising structure providing a force to hold a plenum chamber and a seal-forming structure in a therapeutically effective position on a patient's head in use.

In certain forms, the positioning and stabilising structure comprises an anterior engagement portion configured such that when, in use, a patient-facing surface of the anterior engagement portion contacts a non-patient-facing surface of the plenum chamber, a normal component of the contact force between the patient-facing surface of the anterior engagement portion and the non-patient-facing surface of the plenum chamber is in a direction superior to the direction of a tension force applied by side portions of the positioning and stabilising structure on the anterior engagement portion.

In examples, the patient-facing surface of the anterior engagement portion comprises a surface portion oriented such that a normal direction to the superior-facing surface portion is superior to the direction of the tension force. In other examples, a cross-section of the anterior engagement portion in the mid-sagittal plane is substantially wedge-shaped.

In certain forms the positioning and stabilising structure may comprise a superior portion configured to overlie a region of the patient's head superior to an otobasion superior of the patient's head in use. The positioning and stabilising structure may further comprise an anterior engagement portion configured to overlie an anterior portion of the plenum chamber in use. The positioning and stabilising structure may further comprise two side portions, each of the side portions being connected between the superior portion and the anterior engagement portion and being configured to overlie respective opposite cheek regions of the patient's head in use and to provide a tension force on the anterior engagement portion. A patient-facing surface of the anterior engagement portion is configured such that when, in use, the patient-facing surface of the anterior engagement portion contacts a non-patient-facing surface of the plenum chamber, a normal component of the contact force between the patient-facing surface of the anterior engagement portion and the non-patient-facing surface of the plenum chamber is in a direction superior to the direction of the tension force.

In examples: a) the patient-facing surface of the anterior engagement portion comprises a surface portion oriented such that a normal direction to the superior-facing surface portion is superior to the direction of the tension force; b) a cross-section of the anterior engagement portion in the mid-sagittal plane is substantially wedge-shaped; c) the positioning and stabilising structure further comprises a strap, wherein the anterior engagement portion comprises a central portion of the strap and a wedge mounted on a patient-facing side of the central portion of the strap, wherein the wedge comprises the patient-facing surface of the anterior engagement portion, and wherein the two side portions each comprise end portions of the strap; d) each of the two side portions comprises a first side portion connected between the superior portion and the plenum chamber and a second side portion extending to the anterior engagement portion; e) the first side portion connects to a region of the plenum chamber that is inferior in use to the anterior portion of the plenum chamber overlaid by the anterior engagement portion; f) the second side portions are configured to provide the tension force on a superior part of the plenum chamber to urge a superior part of the seal-forming structure into sealing contact with a region of the patient's face surrounding the patient's nasal airways in use; g) the first side portions are configured to provide a tension force on an inferior part of the plenum chamber to urge an inferior part of the seal-forming structure into sealing contact with a region of the patient's face surrounding the patient's mouth in use; h) an end of each of the second side portions is connected to a part of the respective first side portion at a connection; i) each of the connections between the end of each of the second side portion and the respective first side portion is located to overlie a region of the patient's head adjacently anterior to one of the patient's ears in use; j) the first side portions are configured to provide a tension force on an inferior part of the plenum chamber to urge an inferior part of the seal-forming structure into sealing contact with a region of the patient's face surrounding the patient's mouth in use; k) an end of each of the second side portions is connected to a part of the respective first side portion at a connection; l) each of the connections between the end of each of the second side portion and the respective first side portion is located to overlie a region of the patient's head adjacently anterior to one of the patient's ears in use; m) the positioning and stabilising structure is configured so that the ends of each of the second side portions may be connected to the respective first side portions at a plurality of different positions to adjust the direction of the tension force on the anterior engagement portion; n) the second side portions each comprise end portions of the strap; o) the first side portions each comprise a strap or rigidiser arm; p) the first side portions each comprise a tube configured to deliver pressurised air from the superior portion to the plenum chamber for delivery to the patient's airways, and wherein the superior portion comprises a connection port for receiving a supply of pressurised air and at least one tube for delivering the supply of pressurised air to the tubes of the second side portions; q) the superior portion comprises a crown portion that overlies the crown of the patient's head in use; r) the superior portion comprises a crown portion that overlies the crown of the patient's head in use; s) the crown portion and the second side portions are integrally formed; and/or t) the superior portion comprises a rear portion that overlies the posterior of the patient's head in use.

In certain forms the positioning and stabilising structure may comprise a superior portion configured to overlie a region of the patient's head superior to an otobasion superior of the patient's head in use. The positioning and stabilising structure may further comprise two first side portions, each of the first side portions being configured to overlie respective opposite cheek regions of the patient's head in use and having a superior end connected to the superior portion and an inferior end configured to engage with the plenum chamber at a first region of the plenum chamber to provide a first tension force to urge an inferior part of the seal-forming structure into sealing contact with a region of the patient's face surrounding the patient's airways in use. The positioning and stabilising structure may further comprise two second side portions, each of the second side portions being configured to overlie respective opposite cheek regions of the patient's head in use, being connected to a respective one of the first side portions at a connection and being configured to engage with the plenum chamber at a second region of the plenum chamber to provide a second tension force to urge a superior part of the seal-forming structure into sealing contact with a region of the patient's face surrounding the patient's airways in use. The second region of the plenum chamber is superior to the first region of the plenum chamber in use. The positioning and stabilising structure is configured so that the ends of each of the second side portions may be connected to the respective first side portions at a plurality of different positions to adjust the direction of the second tension force.

In examples: a) each of the connections between the second side portion and the respective first side portion is located to overlie a region of the patient's head adjacently anterior to one of the patient's ears in use; b) the first side portions each comprise a strap or rigidiser arm; c) the first side portions each comprise a tube configured to deliver pressurised air from the superior portion to the plenum chamber for delivery to the patient's airways, and wherein the superior portion comprises a connection port for receiving a supply of pressurised air and at least one tube for delivering the supply of pressurised air to the tubes of the second side portions; d) the positioning and stabilising structure further comprises a strap, wherein a central portion of the strap is configured to overlie an anterior portion of the plenum chamber in use, and wherein the two second side portions each comprise end portions of the strap; e) the central portion of the strap comprises a patient-facing surface configured such that when, in use, the patient-facing surface contacts a non-patient-facing surface of the plenum chamber, a normal component of the contact force between the patient-facing surface of the strap and the non-patient-facing surface of the plenum chamber is in a direction superior to the direction of the second tension force; f) the central portion of the strap comprises a patient-facing surface configured such that when, in use, the patient-facing surface contacts a non-patient-facing surface of the plenum chamber, a normal component of the contact force between the patient-facing surface of the strap and the non-patient-facing surface of the plenum chamber is in a direction superior to the direction of the second tension force; g) the patient-facing surface of the strap comprises a surface portion oriented such that a normal direction to the superior-facing surface portion is superior to the direction of the second tension force; h) the positioning and stabilising structure further comprises a wedge mounted on a patient-facing side of the central portion of the strap, wherein the wedge comprises the patient-facing surface of the strap; i) the superior portion comprises a crown portion that overlies the crown of the patient's head in use; j) the crown portion and the second side portions are integrally formed; and/or k) the superior portion comprises a rear strap that overlies the posterior of the patient's head in use.

An aspect of one form of the present technology is a patient interface for use in a respiratory therapy system. The patient interface may comprise a plenum chamber pressurisable to a therapeutic pressure of at least 6 cmH2O above ambient air pressure, said plenum chamber including a plenum chamber inlet port sized and structured to receive a flow of air at the therapeutic pressure for breathing by a patient. The patient interface may further comprise a seal-forming structure constructed and arranged to form a seal with a region of the patient's face surrounding an entrance to the patient's airways, said seal-forming structure having an opening therein such that the flow of air at said therapeutic pressure is delivered to the entrance to the patient's airways, the seal-forming structure constructed and arranged to maintain said therapeutic pressure in the plenum chamber throughout the patient's respiratory cycle in use. The patient interface further comprising a positioning and stabilising structure to provide a force to hold the seal-forming structure in a therapeutically effective position on the patient's head in use. The patient interface further comprising a vent structure to allow a continuous flow of gases exhaled by the patient from an interior of the plenum chamber to ambient, said vent structure being sized and shaped to maintain the therapeutic pressure in the plenum chamber in use. The patient interface is configured to allow the patient to breath from ambient through their mouth in the absence of a flow of pressurised air through the plenum chamber inlet port, or the patient interface is configured to leave the patient's mouth uncovered. The positioning and stabilising structure may comprise a positioning and stabilising structure according to another aspect of the technology.

In examples: a) the seal-forming structure is configured to seal around an entrance to the patient's nasal airways in use; b) the seal-forming structure is configured to seal around an entrance to the patient's nasal airways and the patient's mouth in use; c) the seal-forming structure forms first and second openings, wherein the first opening is configured to deliver the flow of air to the patient's nasal airways in use, and wherein the second opening is configured to deliver the flow of air to the patient's mouth in use; d) the plenum chamber comprises a groove in an anterior surface of the plenum chamber, wherein the groove comprises the non-patient-facing surface contacted in use by the patient-facing surface of the anterior engagement portion of the positioning and stabilising structure; e) the groove is sized to allow a substantial part of the wedge to be received within the groove; f) the groove is located in a part of the plenum chamber positioned, in use, inferior to a part of the seal-forming structure in sealing contact with a region of the patient's face surrounding the patient's nasal airways; g) the groove is located in a part of the plenum chamber positioned, in use, superior to a part of the seal-forming structure in sealing contact with a region of the patient's face surrounding the patient's mouth; h) the plenum chamber is flexible and a portion of the plenum chamber superior to the groove is able to move relative to a portion of the plenum chamber inferior to the groove; i) the plenum chamber inlet port is positioned in an anterior region of the plenum chamber and is configured to fluidly connect to an air circuit; and/or j) the plenum chamber comprises first and second plenum chamber inlet ports, each of the first and second plenum chamber inlet ports being fluidly connected to one of the tubes comprised as part of the second side portions of the positioning and stabilising structure.

Another aspect of one form of the present technology is a patient interface that is moulded or otherwise constructed with a perimeter shape which is complementary to that of an intended wearer.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a patient interface that may be washed in a home of a patient, e.g., in soapy water, without requiring specialised cleaning equipment.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 3 BRIEF DESCRIPTION OF THE DRAWINGS

The present technology is illustrated by way of example, and not by way of limitation, in the figures of the accompanying drawings, in which like reference numerals refer to similar elements including:
3.1 RESPIRATORY THERAPY SYSTEMS
   Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.
   Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.
   Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.
3.2 RESPIRATORY SYSTEM AND FACIAL ANATOMY
   Fig. 2A shows an overview of a human respiratory system including the nasal and oral cavities, the larynx, vocal folds, oesophagus, trachea, bronchus, lung, alveolar sacs, heart and diaphragm.
   Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.
   Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.
   Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.
   Fig. 2E is a further side view of a head. The approximate locations of the Frankfort horizontal and nasolabial angle are indicated. The coronal plane is also indicated.
   Fig. 2F shows a base view of a nose with several features identified including naso-labial sulcus, lip inferior, upper Vermilion, naris, subnasale, columella, pronasale, the major axis of a naris and the midsagittal plane.
   Fig. 2G shows a side view of the superficial features of a nose.
   Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
   Fig. 2I shows a medial dissection of a nose, approximately several millimeters from the midsagittal plane, amongst other things showing the septum cartilage and medial crus of greater alar cartilage.
   Fig. 2J shows a front view of the bones of a skull including the frontal, nasal and zygomatic bones. Nasal concha are indicated, as are the maxilla, and mandible.
   Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.
   Fig. 2L shows an anterolateral view of a nose.
   Fig. 2M shows a front view of a patient's face indicating an alar angle according to certain forms of the technology.
   Fig. 2N shows a side view of a patient's face indicating a nose-supramenton angle according to certain forms of the technology.
3.3 PATIENT INTERFACE
   Fig. 3A shows a patient interface in the form of a nasal mask in accordance with one form of the present technology.
   Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
   Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
   Fig. 3D shows a schematic of a cross-section through a structure at a point. An outward normal at the point is indicated. The curvature at the point has a value of zero.
   Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
   Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
   Fig. 3G shows a cushion for a mask that includes two pillows. An exterior surface of the cushion is indicated. An edge of the surface is indicated. Dome and saddle regions are indicated.
   Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.
   Fig. 3I shows a patient interface having conduit headgear, in accordance with one form of the present technology.
   Fig. 4A shows a perspective view of a cushion of a patient interface configured to be worn by a patient and convey pressurized air to the patient's nose and the patient's mouth.
   Fig. 4B shows a perspective view of a cushion of a patient interface configured to be worn by a patient and convey pressurized air to the patient's nose.
   Fig. 4C shows a perspective view of rigidiser arms usable with either the cushion of Fig. 4A of the cushion of Fig. 4B.
   Fig. 4D shows a perspective view of headgear straps usable with the cushion of Fig. 4B.
   Fig. 5 shows a front view of a cushion 3050 according to one form of the technology.
   Fig. 6A shows a front view of a brace 3900 according to one form of the technology.
   Fig. 6B shows a front view of the cushion 3050 of Fig. 5 with the brace 3900 of Fig. 6A mounted to it.
   Fig. 6C shows a side cross-sectional view of the cushion 3050 of Fig. 5 with the brace 3900 of Fig. 6A mounted to it.
   Fig. 6D shows a top cross-sectional view of the cushion 3050 of Fig. 5 with the brace 3900 of Fig. 6A mounted to it.
   Fig. 7A shows a front view of a brace 3900 according to another form of the technology.
   Fig. 7B shows a front view of a cushion 3050 similar to that shown in Fig. 5 with the brace 3900 of Fig. 7A mounted to it.
   Fig. 7C shows a side cross-sectional view of the cushion 3050 and brace 3900 of Fig. 7B.
   Fig. 7D shows a top cross-sectional view of the cushion 3050 and brace 3900 of Fig. 7B.
   Fig. 8A shows a front view of a brace 3900 according to another form of the technology.
   Fig. 8B shows a front view of the cushion 3050 similar to that shown in Fig. 5 with the brace 3900 of Fig. 8A mounted to it.
   Fig. 8C shows a side cross-sectional view of the cushion 3050 and brace 3900 of Fig. 8B.
   Fig. 8D shows a top cross-sectional view of the cushion 3050 and brace 3900 of Fig. 8B.
   Fig. 9A shows a top view of a cushion 3050 and brace 3200 according to another form of the technology.
   Fig. 9B shows a top view of a cushion 3050 and brace 3200 according to another form of the technology.
   Fig. 9C shows a top view of a cushion 3050 and brace 3200 according to another form of the technology.
   Fig. 10A shows a top view of a patient interface 3000 according to another form of the technology.
   Fig. 10B shows a top view of a patient interface 3000 according to another form of the technology.
3.4 RPT DEVICE
   Fig. 11A shows an RPT device in accordance with one form of the present technology.

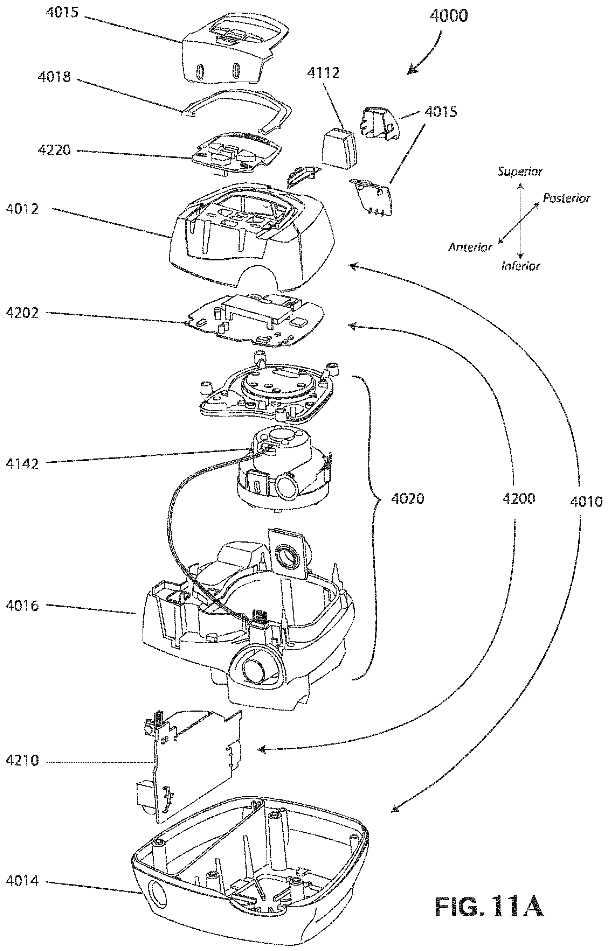

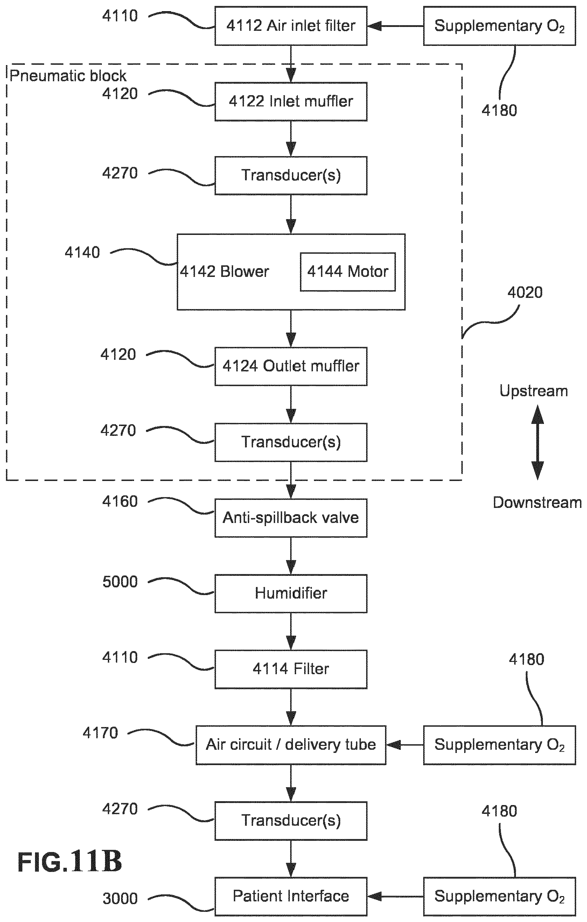

   Fig. 11B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.
3.5 HUMIDIFIER
   Fig. 12A shows an isometric view of a humidifier in accordance with one form of the present technology.
   Fig. 12B shows an isometric view of a humidifier in accordance with one form of the present technology, showing a humidifier reservoir 5110 removed from the humidifier reservoir dock 5130.
3.6 POSITIONING AND STABILISING STRUCTURE
   Fig. 13A shows a perspective view of a portion of a patient interface including part of a positioning and stabilising structure in accordance with one form of the present technology.
   Fig. 13B shows a front view of the portion of the patient interface of Fig. 13A with part of the positioning and stabilising structure not shown.
   Fig. 13C shows a side view of the portion of the patient interface of Fig. 13A with an additional part of the positioning and stabilising structure shown.
   Fig. 14A shows a perspective view of a portion of a patient interface including part of a positioning and stabilising structure in accordance with another form of the present technology.
   Fig. 14B shows a front view of the portion of the patient interface of Fig. 14A.
   Fig. 14C shows a side view of the portion of the patient interface of Fig. 13A with an additional part of the positioning and stabilising structure shown.
   Fig. 15A shows a side view of a patient interface in accordance with another form of the present technology with a portion of the positioning and stabilising structure in a first position.
   Fig. 15B shows a side view of the patient interface of Fig. 15A with a portion of the positioning and stabilising structure in a second position.
   Fig. 16A shows a side view of a patient interface in accordance with another form of the present technology.
   Fig. 16B shows a side view of region A of Fig. 16A with a portion of an anterior engagement portion shown in cross-section.
   Fig. 16C shows a perspective view of an anterior engagement portion of the positioning and stabilising structure of Fig. 16A.
   Fig. 16D shows a side cross-sectional view of region A of Fig. 16A with the anterior engagement portion in a first position.
   Fig. 16E shows a side cross-sectional view of region A of Fig. 16A with the anterior engagement portion in a second position.
   Fig. 17A shows a side view of a patient interface in accordance with another form of the present technology.
   Fig. 17B shows a front view of the patient interface of Fig. 17A.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 4.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 4.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 3800.

### 4.3 PATIENT INTERFACE

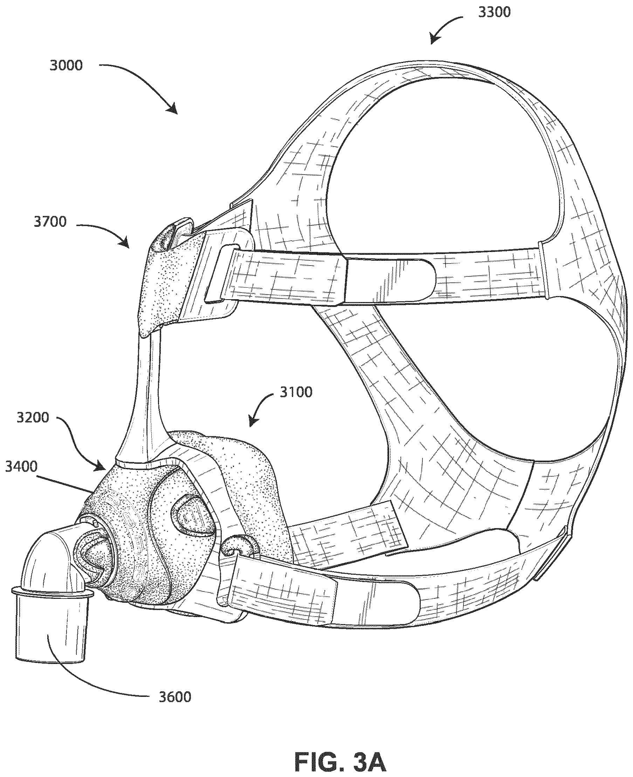

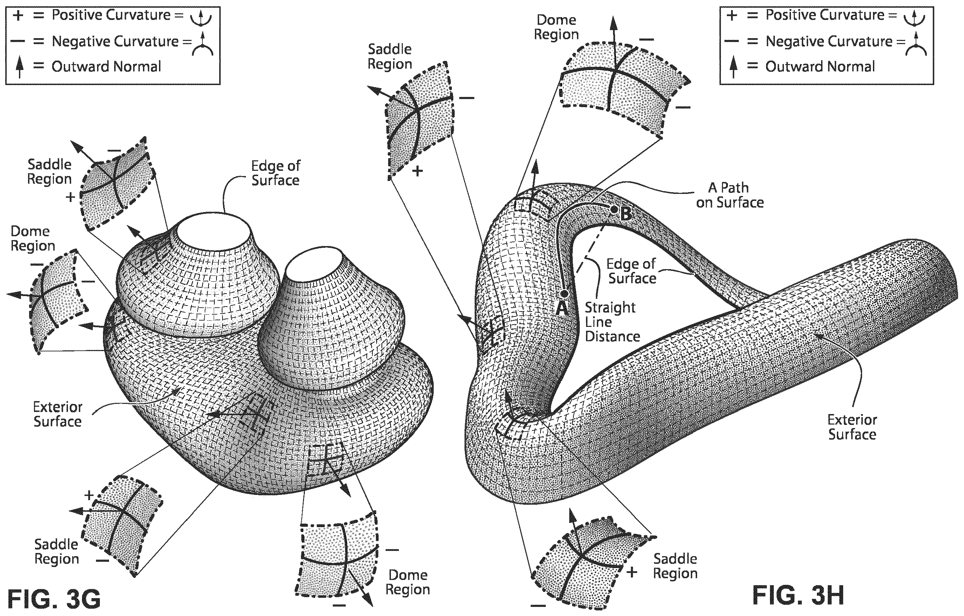

A non-invasive patient interface 3000, such as that shown in Fig. 3A, in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

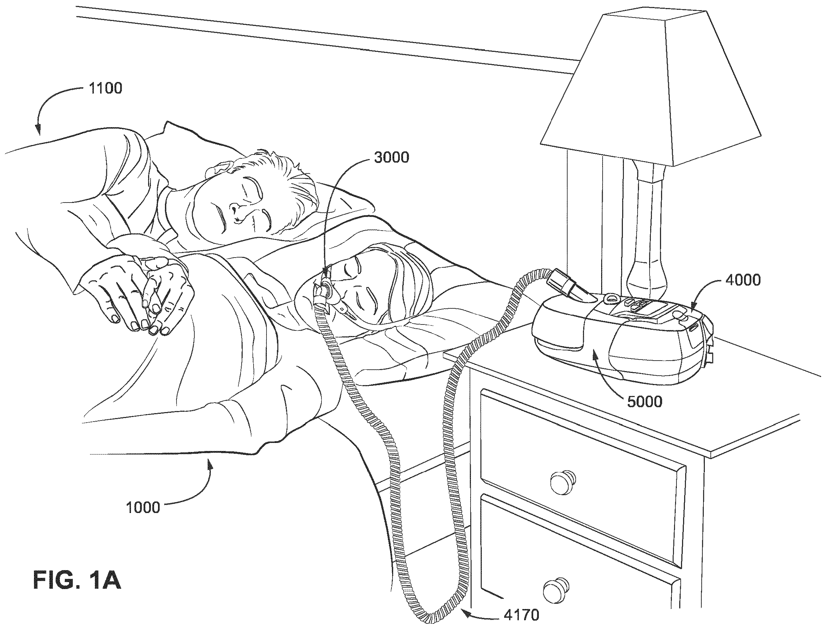

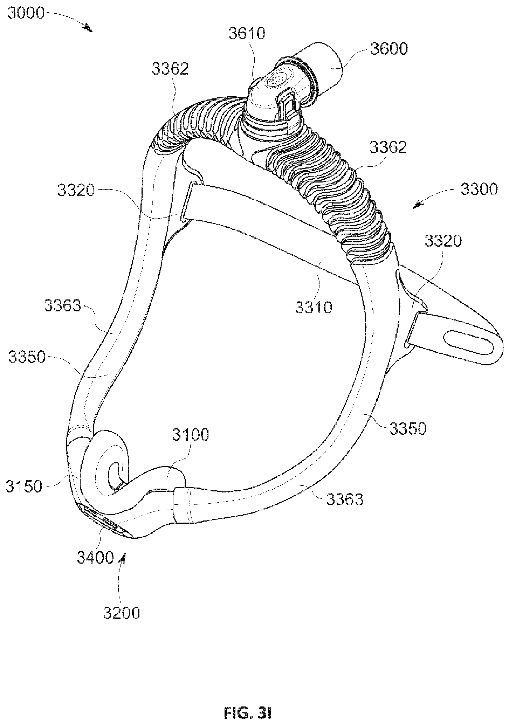

As shown in Fig. 3I, a non-invasive patient interface 3000 in accordance with another aspect of the present technology comprises the following functional aspects: a seal-forming structure 3000, a plenum chamber 3200, a positioning and stabilising structure 3300, a vent 3400 and one form of connection port 3600 for connection to an air circuit (such as the air circuit 4170 shown in Figs. 1A-1C). The plenum chamber 3200 may be formed of one or more modular components in the sense that it or they can be replaced with different components, for example components of a different size.

An unsealed patient interface 3800, in the form of a nasal cannula, includes nasal prongs 3810a, 3810b which can deliver air to respective nares of the patient 1000 via respective orifices in their tips. Such nasal prongs do not generally form a seal with the inner or outer skin surface of the nares. This type of interface results in one or more gaps that are present in use by design (intentional) but they are typically not fixed in size such that they may vary unpredictably by movement during use. This can present a complex pneumatic variable for a respiratory therapy system when pneumatic control and/or assessment is implemented, unlike other types of mask-based respiratory therapy systems. The air to the nasal prongs may be delivered by one or more air supply lumens 3820a, 3820b that are coupled with the nasal cannula-type unsealed patient interface 3800. The lumens 3820a, 3820b lead from the nasal cannula-type unsealed patient interface 3800 to a respiratory therapy device via an air circuit. The unsealed patient interface 3800 is particularly suitable for delivery of flow therapies, in which the RPT device generates the flow of air at controlled flow rates rather than controlled pressures. The "vent" or gap at the unsealed patient interface 3800, through which excess airflow escapes to ambient, is the passage between the end of the prongs 3810a and 3810b of the nasal cannula-type unsealed patient interface 3800 via the patient's nares to atmosphere.

In certain forms of the technology, the patient interface 3000 may comprise a cushion module 3150. The cushion module may be a physical component, or assembly of components, that provides the functional aspects of a seal-forming structure 3100 and/or a plenum chamber 3200. These functional aspects may be provided by the cushion module 3150 alone or in combination with one or more other physical components. The cushion module 3150 may comprise a cushion 3050 and a frame 3270. The cushion 3050 may alone or in combination with one or more other components provide the functional aspects of a seal-forming structure 3100 and/or a plenum chamber 3200. The frame 3270 may alone or in combination with one or more other components provide the functional aspect of a plenum chamber 3200.

In certain forms of the technology, the patient interface 3000 may comprise a brace 3900. A further explanation of brace 3900 is provided below.

In certain forms, the patient interface 3000 may comprise a positioning and stabilising structure 3300 to hold the seal-forming structure 3100 in a sealing position in use. More details on positioning and stabilising structures 3000 that may be provided in different forms of the technology are described below.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure above ambient, for example of at least 2, 4, 6, 10 or 20 cmH₂O with respect to ambient.

### 4.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs - the actual sealing surface - may change within a given treatment session, from day to day, and from patient to patient, depending on a range of factors including for example, where the patient inte-face was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 4.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. The support flange may be relatively thicker than the sealing flange. The support flange is disposed between the sealing flange and the marginal edge of the plenum chamber 3200, and extends at least part of the way around the perimeter. The support flange is or includes a spring-like element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface. An adhesive surface may be used to adhere the seal-forming structure to the patient's face.

In certain forms of the present technology, a seal-forming structure may comprise one or more of a pressure-assisted sealing flange, a compression sealing portion, a gasket sealing portion, a tension portion, and a portion having a tacky or adhesive surface.

### 4.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 4.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 4.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 4.3.1.5 Forehead region

In one form, the seal-forming structure that forms a seal in use on a forehead region of the patient's face. In such a form, the plenum chamber may cover the eyes in use.

### 4.3.1.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 4.3.1.7 Nose-only Masks

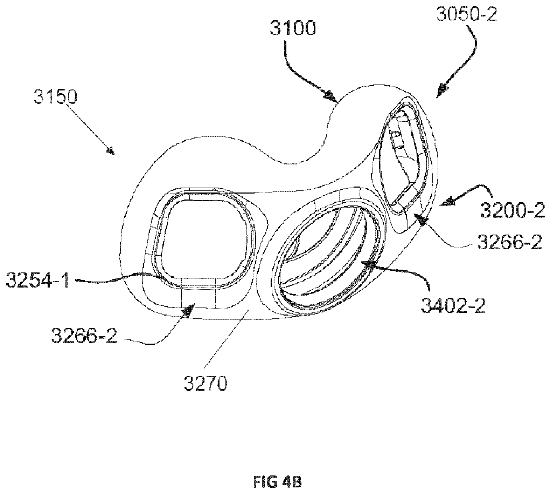

In certain forms, such as shown in Figs. 3I and 4B, the patient interface 3000 comprises a seal-forming structure 3100 configured to seal around an entrance to the patient's nasal airways but not around the patient's mouth. The seal-forming structure 3100 may be configured to seal to the patient's lip superior. The patient interface 3000 may leave the patient's mouth uncovered. This patient interface 3000 may deliver a supply of air or breathable gas to both nares of patient 1000 and not to the mouth. This type of patient interface may be identified as a nose-only mask.

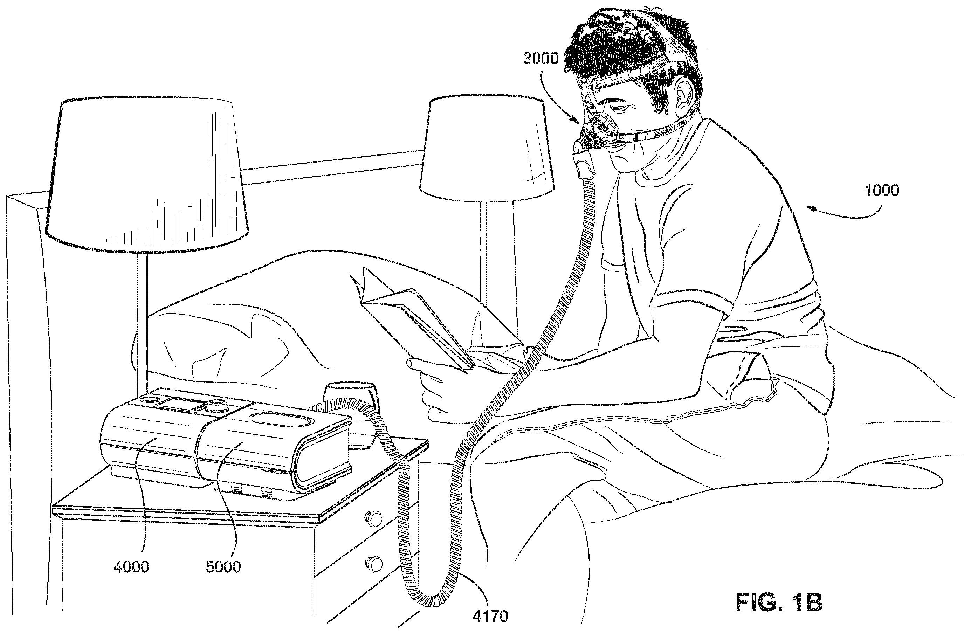

One form of nose-only mask according to the present technology is what has traditionally been identified as a "nasal mask", having a seal-forming structure 3100 configured to seal on the patient's face around the nose and over the bridge of the nose. A nasal mask may be generally triangular in shape. In one form, the non-invasive patient interface 3000 comprises a seal-forming structure 3100 that forms a seal in use to an upper lip region (e.g. the lip superior), to the patient's nose bridge or at least a portion of the nose ridge above the pronasale, and to the patient's face on each lateral side of the patient's nose, for example proximate the patient's nasolabial sulci. The patient interface 3000 shown in Fig. 1B has this type of seal-forming structure 3100. This patient interface 3000 may deliver a supply of air or breathable gas to both nares of patient 1000 through a single orifice.

Another form of nose-only mask may seal around an inferior periphery of the patient's nose without engaging the user's nasal ridge. This type of patient interface 3000 may be identified as a "nasal cradle" mask and the seal-forming structure 3100 may be identified as a "nasal cradle cushion", for example. In one form, for example as shown in Fig. 3I, the seal-forming structure 3100 is configured to form a seal in use with inferior surfaces of the nose around the nares. The seal-forming structure 3100 may be configured to seal around the patient's nares at an inferior periphery of the patient's nose including to an inferior and/or anterior surface of a pronasale region of the patient's nose and to the patient's nasal alae. The seal-forming structure 3100 may seal to the patient's lip superior. The shape of the seal-forming structure 3100 may be configured to match or closely follow the underside of the patient's nose and may not contact a nasal bridge region of the patient's nose or any portion of the patient's nose superior to the pronasale. In one form of nasal cradle cushion, the seal-forming structure 3100 comprises a bridge portion dividing the opening into two orifices, each of which, in use, supplies air or breathable gas to a respective one of the patient's nares. The bridge portion may be configured to contact or seal against the patient's columella in use. Alternatively, the seal-forming structure 3100 may comprise a single opening to provide a flow or air or breathable gas to both of the patient's nares.

In some forms, a nose-only mask may comprise nasal pillows, described above.

The seal-forming structure of a nose-only mask may be provided by a nasal portion 3050-2 of a cushion 3050, such as is shown in Figs. 3I, 4B and 9A to 9C.

### 4.3.1.8 Nose and Mouth Masks

In certain forms, such as shown in Figs. 3A, 4A, 5, 6B to 6D, 7B to 7D, 8B to 8D, 13A to 13C, 14A to 14C, 15A to 15B, 16A to 16E and 17A to 17B, the patient interface 3000 comprises a seal-forming structure 3100 configured to seal around an entrance to the patient's nasal airways and also around the patient's mouth. The seal-forming structure 3100 may be configured to seal to the patient's face proximate a chin region. This patient interface 3000 may deliver a supply of air or breathable gas to both nares and to the mouth of patient 1000. This type of patient interface may be identified as a nose and mouth mask.

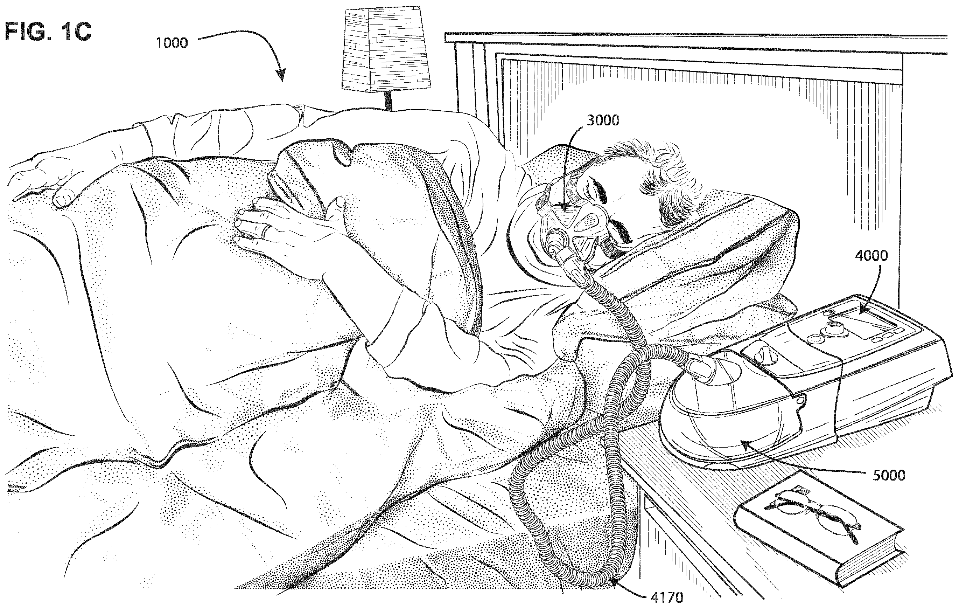

One form of nose and mouth mask according to the present technology is what has traditionally been identified as a "full-face mask", having a seal-forming structure 3100 configured to seal on the patient's face around the nose, below the mouth and over the bridge of the nose. A full-face mask may be generally triangular in shape. In one form the patient interface 3000 comprises a seal-forming structure 3100 that forms a seal in use to a patient's chin-region (which may include the patient's lip inferior and/or a region directly inferior to the lip inferior), to the patient's nose bridge or at least a portion of the nose ridge superior to the pronasale, and to cheek regions of the patient's face. The patient interfaces 3000 shown in Fig. 1C and 3A are of this type. This patient interface 3000 may deliver a supply of air or breathable gas to both nares and mouth of patient 1000 through a single orifice. This type of seal-forming structure 3100 may be referred to as a "full face cushion".

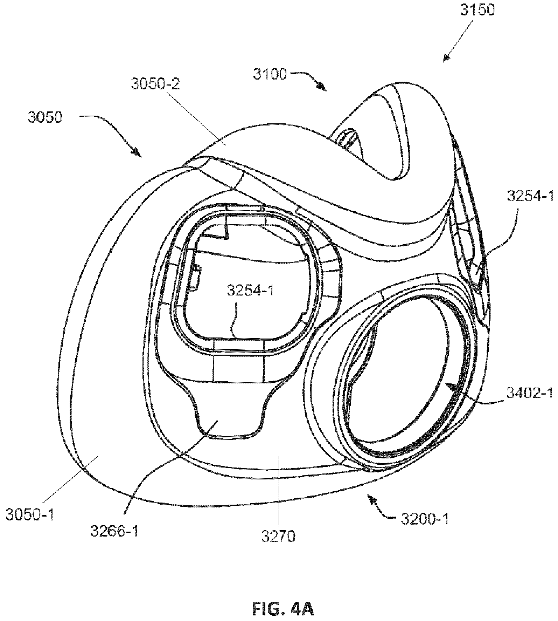

In other forms, for example as shown in Figs. 4A, 5, 6B to 6D, 7B to 7D, 8B to 8D, 13A to 13C, 14A to 14C, 15A to 15B, 16A to 16E and 17A to 17B, the patient interface 3000 comprises a seal-forming structure 3100 that forms a seal in use on a patient's chin region (which may include the patient's lip inferior and/or a region directly inferior to the lip inferior), to an inferior and/or an anterior surface of a pronasale portion of the patient's nose, to the alae of the patient's nose and to the patient's face on each lateral side of the patient's nose, for example proximate the nasolabial sulci. The seal-forming structure 3100 may also form a seal against a patient's lip superior. A patient interface 3000 having this type of seal-forming structure may have a single opening configured to deliver a flow of air or breathable gas to both nares and mouth of a patient, may have an oral hole configured to provide air or breathable gas to the mouth and a nasal hole configured to provide air or breathable gas to the nares, or may have an oral hole for delivering air to the patient's mouth and two nasal holes for delivering air to respective nares. This type of patient interface 3000 may have a nasal portion and an oral portion, the nasal portion sealing to the patient's face at similar locations to a nasal cradle mask.

In a further form of nose and mouth mask, the patient interface 3000 may comprise a seal-forming structure 3100 having a nasal portion comprising nasal pillows and an oral portion configured to form a seal to the patient's face around the patient's mouth.

In some forms, the seal-forming structure 3100 may have a nasal portion that is separate and distinct from an oral portion. In other forms, a seal-forming structure 3100 may form a contiguous seal around the patient's nose and mouth.

The seal-forming structure of a nose and mouth mask may be provided by a nasal portion 3050-2 and a mouth portion 3050-1 of a cushion 3050, such as is shown in Figs. 3A, 4A, 5, 6B to 6D, 7B to 7D, 8B to 8D, 13A to 13C, 14A to 14C, 15A to 15B, 16A to 16E and 17A to 17B. Further information on the relative orientations of the seal-forming regions of the nasal portion 3050-2 and the mouth portion 3050-1 is provided below.

It is to be understood that the above examples of different forms of patient interface 3000 do not constitute an exhaustive list of possible configurations. In some forms a patient interface 3000 may comprise a combination of different features of the above-described examples of nose-only and nose and mouth masks.

### 4.3.2 Plenum chamber

In certain forms, the plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms, the plenum chamber 3200 may be formed, at least in part, by a cushion module 3150. As explained in more details below, the cushion module 3150 may comprise a cushion 3050 and optionally a frame 3270.

In certain forms of the present technology, the plenum chamber 3200 is constructed from a transparent or translucent material, e.g. the frame 3270 may be formed a transparent or translucent polycarbonate and/or the cushion 3050 may be formed from a transparent or translucent silicone. The use of a transparent or translucent material can reduce the obtrusiveness of the patient interface, and help improve compliance with therapy. The use of a transparent material can aid a clinician to observe how the patient interface is located and functioning.

### 4.3.2.1 Multiple Openings

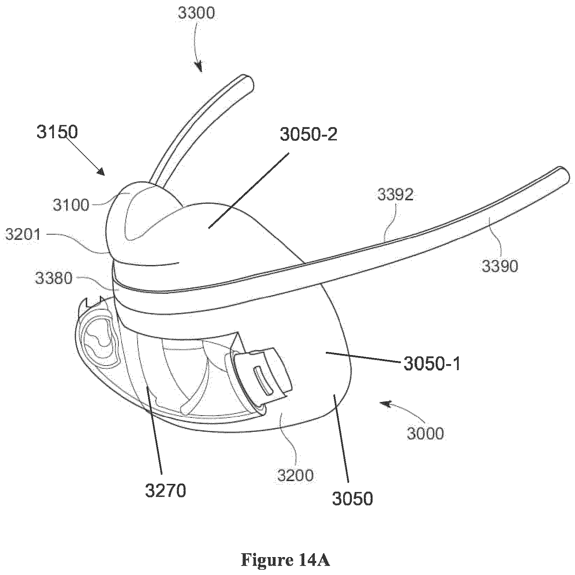

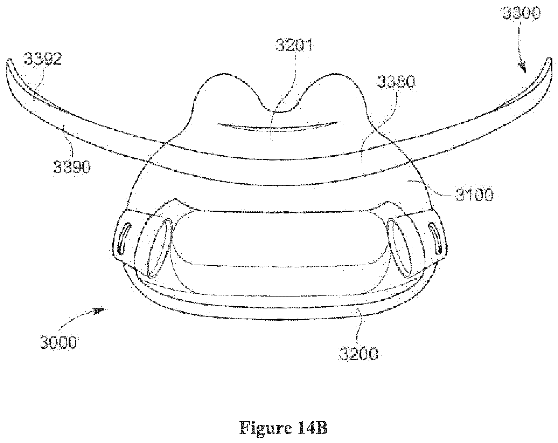

As shown in Figs. 4A, 4B, 14A and 14B, different plenum chambers 3200-1, 3200-2 may be formed as part of a multi-opening cushion module 3150. In the illustrated examples, the cushion modules 3150 each include three openings, although an alternate cushion may be formed with greater or fewer openings.

In some forms, the different openings may serve different functions. For example, some openings may be exclusively inlet openings, while other openings may be exclusively outlet openings.

In other forms, at least one opening may serve two different functions. For example, one opening may operate as both an inlet and an outlet during the same breathing cycle.

The plurality of openings may allow for a variety of configurations of air delivery to the plenum chamber 3200-1, 3200-2. For example, depending on patient need and/or patient comfort, the patient may use a given cushion 3050 in a configuration using conduit headgear (described below) or a configuration using a single conduit in front of the patient's face, for example.

### 4.3.2.1.1 Nose and Mouth Mask

As shown in Fig. 4A, the plenum chamber 3200-1 may include a pair of plenum chamber inlet ports 3254-1, which may be used to convey gas into and/or out of the plenum chamber 3200-1. The plenum chamber inlet ports 3254-1 may be disposed on opposite sides (e.g., left and right sides) of the plenum chamber 3200-1.

In some forms, the plenum chamber 3200-1 may also include at least one vent opening 3402-1 (see e.g., Fig. 4A). The vent opening 3402-1 may be disposed in a center of the plenum chamber 3200-1. For example, the vent opening 3402-1 may be disposed between the plenum chamber inlet ports 3254-1.

In some forms, the plenum chamber 3200-1 may include a pair of grooves 3266-1. Each groove 3266-1 may be disposed proximate to one of the plenum chamber inlet ports 3254-1. Each groove 3266-1 may form a partially recessed surface.

### 4.3.2.1.2 Nose-only Mask

The plenum chamber 3200-2 of a nasal only cushion module 3150 may be similar to the plenum chamber 3200-1 of the mouth and nose cushion modules 3150. Only some similarities and differences between the plenum chambers 3200-1, 3200-2 may be described below.

As shown in Fig. 4B, the plenum chamber 3200-2 includes a pair of plenum chamber inlet ports 3254-2, which may be used to convey gas into and/or out of the plenum chamber 3200-2. The plenum chamber inlet ports 3254-2 may be disposed on opposite sides (e.g., left and right sides) of the plenum chamber 3200-2.

In some forms, the plenum chamber 3200-2 may also include at least one vent opening 3402-2 (see e.g., Fig. 4B). The vent opening 3402-2 may be disposed in a centre of the plenum chamber 3200-2. For example, the vent opening 3402-2 may be disposed between the plenum chamber inlet ports 3254-2.

In some forms, the plenum chamber 3200-2 may include a pair of grooves 3266-2. Each groove 3266-2 may be disposed proximate to one of the plenum chamber inlet ports 3254-2. Each groove 3266-2 may form a partially recessed surface.

### 4.3.3 Cushion Module

In certain forms of the technology, the patient interface 3000 comprises a cushion module 3150. The cushion module may be a physical component, or assembly of components, that provides the functional aspects of a seal-forming structure 3100 and/or a plenum chamber 3200. These functional aspects may be provided by the cushion module 3150 alone or in combination with one or more other physical components. The cushion module 3150 may comprise a cushion 3050 and a frame 3270. The cushion 3050 may alone or in combination with one or more other components provide the functional aspects of a seal-forming structure 3100 and/or a plenum chamber 3200. The frame 3270 may alone or in combination with one or more other components provide the functional aspect of a plenum chamber 3200.

Examples of cushion modules 3150 according to certain forms of the technology are illustrated in Figs. 3A, 3I, 4A, 4B, 5 to 9C, and 13A to 17B.

### 4.3.3.1 Cushion

The cushion module 3150 may comprise a cushion 3050. The cushion 3050 may be a component, or assembly of components, that provide a cushioning function when the patient interface 3000 is worn. That is, the cushion 3050 may act to create a soft, or relatively soft, feel of the patient interface 3000 against the patient's skin when worn.

The cushion 3050 may provide the functional aspect of the seal-forming structure 3100. For example, the cushion 3050 may comprise a seal-forming structure 3100 such as described in more detail earlier. In some forms, the seal-forming structure 3100 may form parts of the cushion 3050 located on a patient-facing side during use of the patient interface 3000.

The cushion 3050 may additionally provide the functional aspect of the plenum chamber 3200, either alone or in combination with one or more other components, for example with the frame 3270.

In some forms, the cushion 3050 is, at least in part, constructed and configured to be flexible. For example, the cushion 3050 may be constructed from a soft, flexible, resilient material like silicone, textile, foam, etc., and/or be configured in a shape that allows flexibility. In some forms the cushion 3050 may be formed from a material which has a Young's modulus of 0.4 Gpa or lower, for example foam. In some forms of the technology the cushion 3050 may be made from a material having Young's modulus of 0.1Gpa or lower, for example rubber. In other forms of the technology the cushion 3050 may be made from a material having a Young's modulus of 0.7Mpa or less, for example between 0.7Mpa and 0.3Mpa. An example of such a material is silicone.

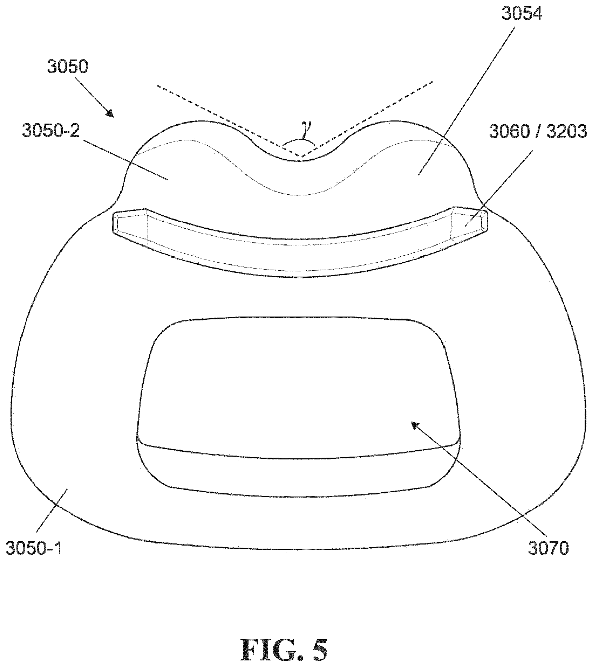

The cushion 3050 may comprise one or more openings 3070 for receiving a flow of breathable gas to be supplied to the patient's airways. Such an opening is illustrated in Fig. 5, for example, where the cushion 3050 comprises a single such opening 3070. The opening 3070 may be located in an anterior wall of the cushion 3050.

The cushion 3050 may comprise one or more openings for delivering the flow of breathable gas to the patient's airways, e.g. to an entrance to the patient's nares and/or mouth. This opening may be located in a posterior wall of the cushion 3050.

In certain forms of the technology, the cushion 3050 may comprise a nasal portion 3050-2. In certain forms of the technology, the cushion 3050 may comprise both a nasal portion 3050-2 and a mouth portion 3050-1.

### 4.3.3.1.1 Nasal portion

In certain forms of the technology, such as illustrated in Figs. 3A, 3I, 4A, 4B, 5 to 9C and 13A to 17B, the cushion 3050 may comprise a nasal portion 3050-2. The nasal portion 3050-2 is a portion of the cushion 3050 that is configured to be positioned, in use, proximate to a nasal region and/or a lip superior region of the patient's face. In certain forms, the nasal portion 3050-2 is configured to be positioned, in use, proximate and anterior to a nasal region and/or lip superior region of the patient's face.

The nasal portion 3050-2 may comprise a part of the seal-forming structure 3100 that is configured, in use, to form a seal with a region of the patient's face surrounding, or part surrounding, one or both entrances to the patient's nares. For example, in the case of the cushion 3050 illustrated in Figs. 5 to 9C, the nasal portion 3050-2 may seal at least partly around an inferior periphery of the patient's nose. The nasal portion 3050-2 illustrated in Figs. 5 to 9C may be identified as a "nasal cradle cushion" as explained above. It should be understood that the nasal portion 3050-2 may be configured to partly (as opposed to entirely) surround the entrances to the patient's nares in some forms because, in such forms, the cushion 3050 may additionally comprise a mouth portion 3050-1 that, in combination with the nasal portion 3050-2, surround the entrances to the nares in use, and the nasal portion 3050-2 itself may only surround the nares partly, for example around superior and lateral regions of the nares. In other forms, the nasal portion 3050-2 may be configured to form a seal with a region of the patient's face entirely surrounding one or both entrances to the patient's nares.

The nasal portion 3050-2 may also form at least part of the plenum chamber 3200. Accordingly, at least part of the plenum chamber 3200 may be situated, in use, proximate the nasal region and/or lip superior region of the patient's face, e.g. proximate and anterior to the nasal region and/or the lip superior region. In some forms, for example the forms illustrated in Figs. 5 to 9C, the nasal portion 3050-2 of the cushion 3050 entirely forms, or forms a substantial part of, the part of the plenum chamber 3200 proximate to the region of the patient's face around the nares, e.g. the part of the plenum chamber 3200 proximate and anterior to the nasal region and/or lip superior region. Consequently, the volume of space with which the patient's nares directly exchange air as the patient breathes in an out may be entirely or substantially enclosed by a component or assembly of components constructed and configured to be substantially flexible in use.

In order to form at least part of the plenum chamber 3200, the nasal portion 3050-2 may comprise a posterior wall portion 3052 and an anterior wall portion 3054. In use, when the patient interface 3000 is worn by the patient, the posterior wall portion 3052 is closer to the patient's face than the anterior wall portion 3054. The posterior wall portion 3052 comprises the part of the seal-forming structure 3100 on the nasal portion 3050-2. The posterior wall portion 3052 and the anterior wall portion 3054 may be directly connected, or may be connected by lateral wall portions and a superior wall portion of the nasal portion 3050-2. The posterior wall portion 3052 and the anterior wall portion 3054 may form the walls enclosing the volume of space enclosed within part of the plenum chamber 3200.

In certain forms, the cushion 3050 may comprise only a nasal portion 3050-2. For example, in the forms of the technology shown in Figs. 3I, 4B and 9A to 9C, the cushion comprises a nasal portion 3050-2 but no mouth portion.

In certain forms of the technology, the nasal portion 3050-2 of the cushion 3050 may be configured to receive a brace 3900, such as braces 3900 of the kind described in more detail below.

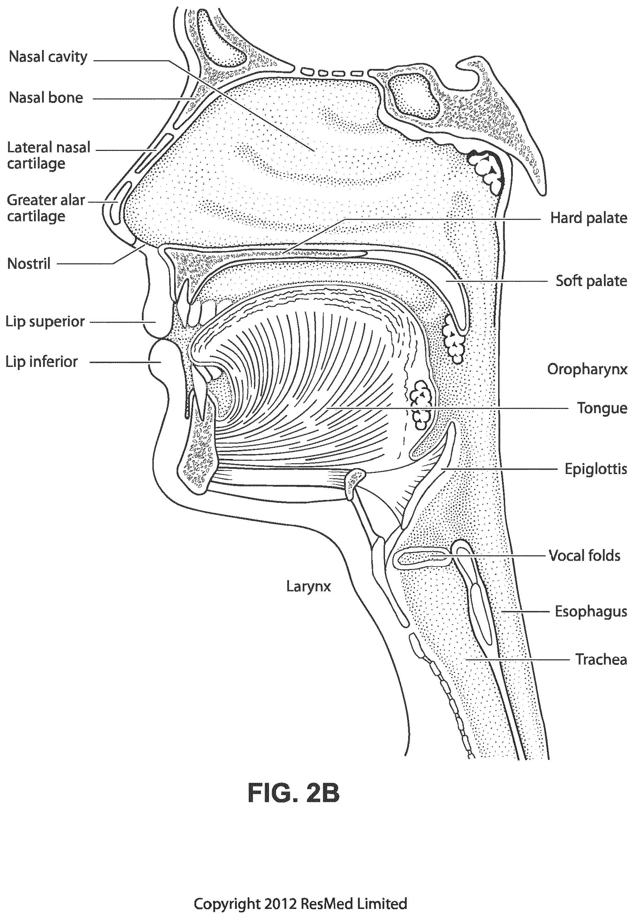

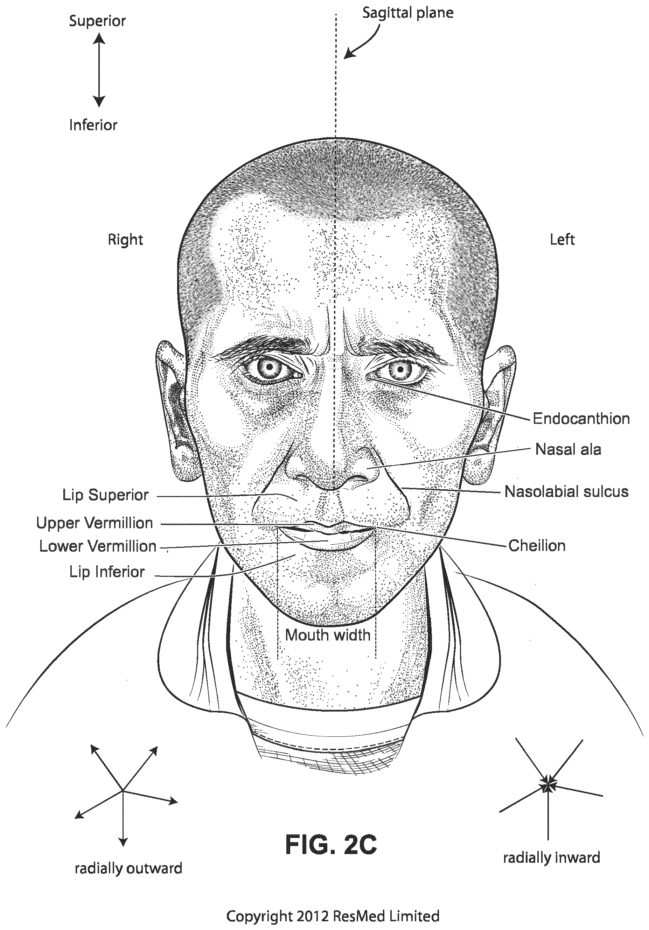

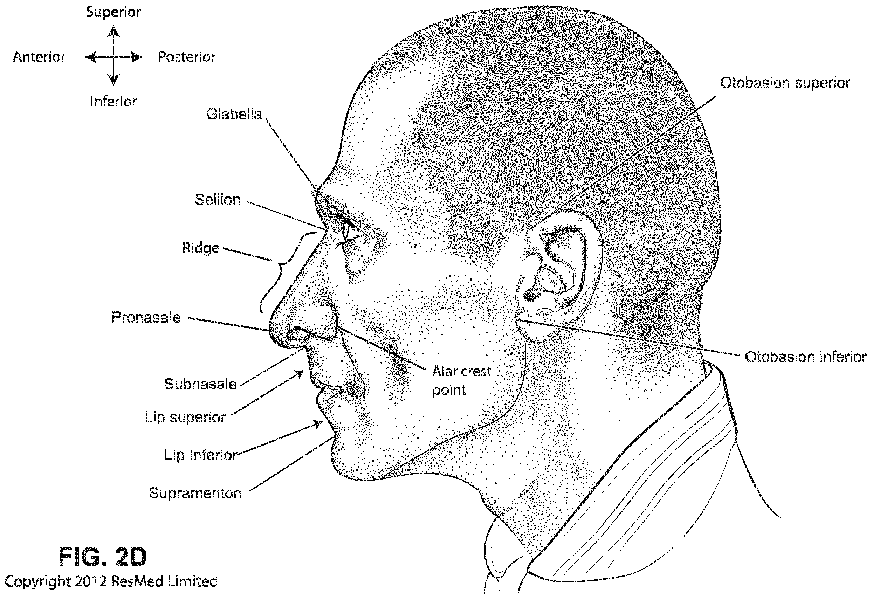

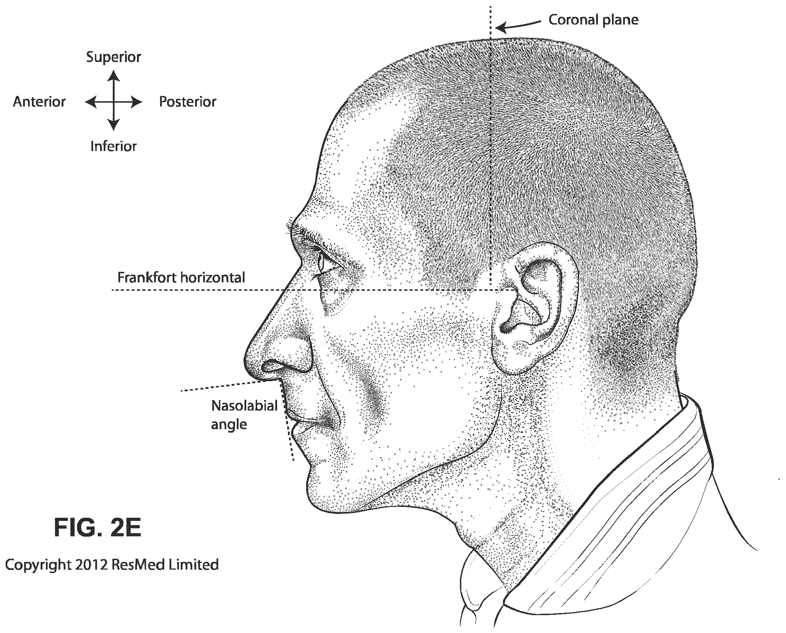

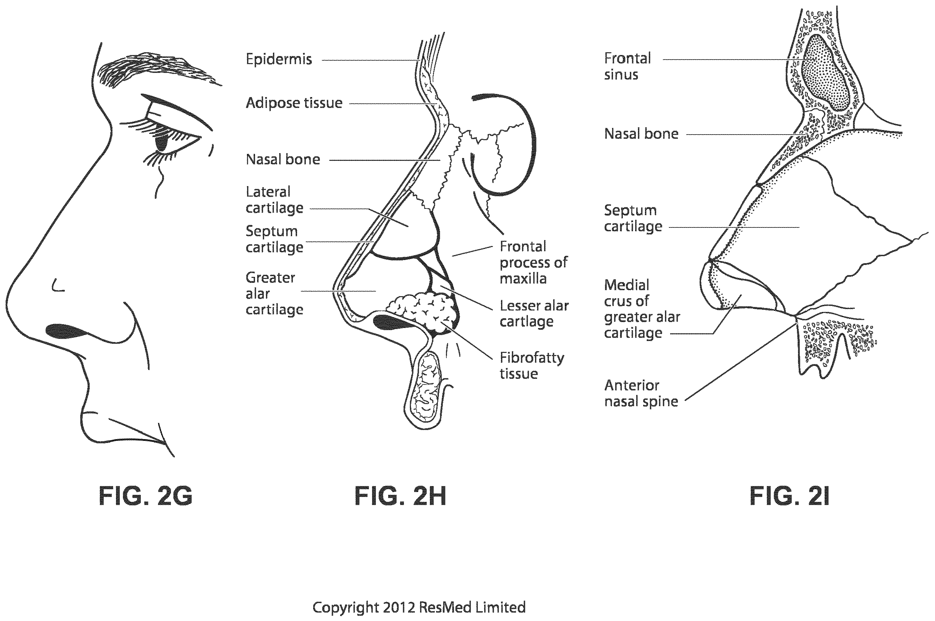

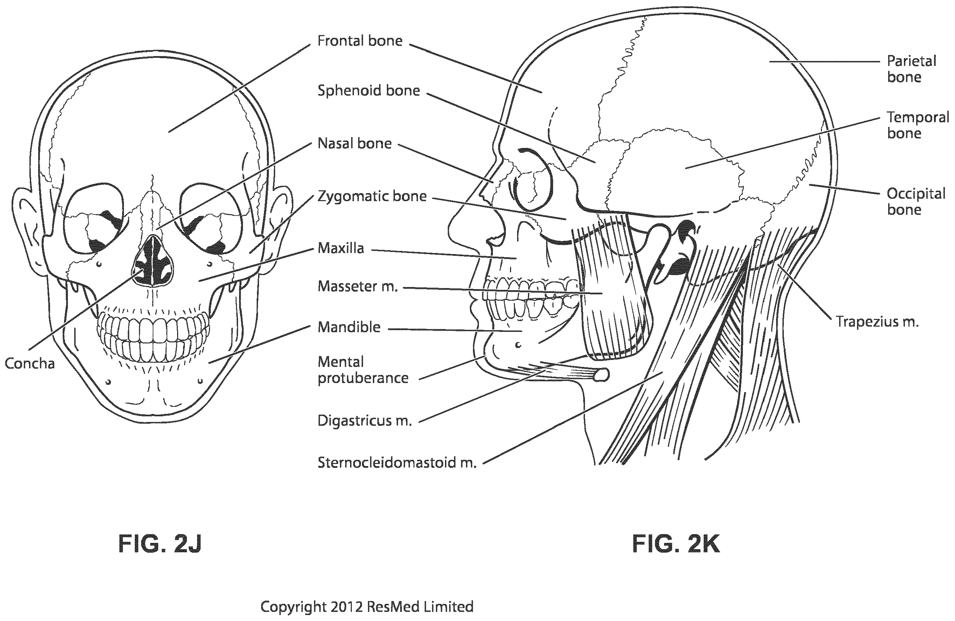

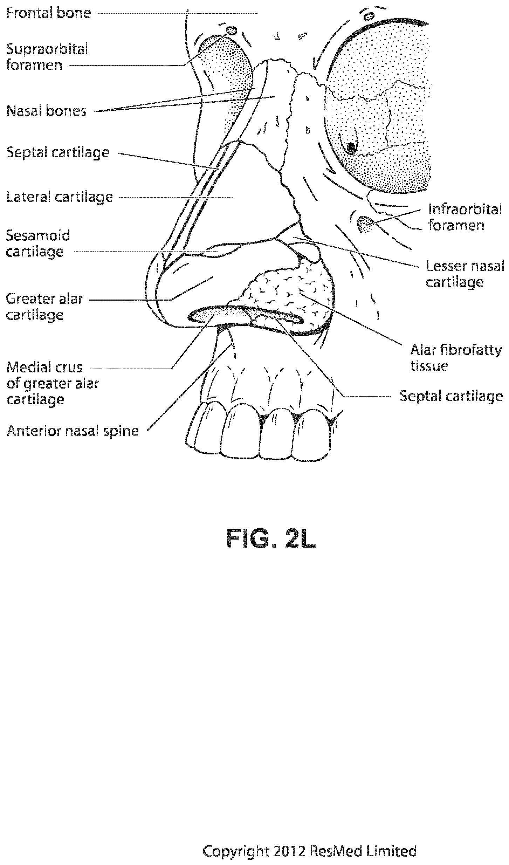

In the form of the technology shown in Figs. 5, 6B to 6D, 7B to 7D and 8B to 8D, the nasal portion 3050-2 of the cushion 3050 forms a recess 3060 suitable for receiving brace 3900. For example, the recess 3060 may have a shape and size suitable for brace 3900, or part of brace 3900, to fit into the recess 3060. The recess 3060 may have a shape that is complementary to the shape of the brace 3900 or a part of the brace 3900 that, in use, fits into the recess 3060. For example, the recess 3060 may form a negative space that is the same or similar to the shape of the brace 3900 or part thereof. In the form of Fig. 5, the recess 3060 is elongate, i.e. it has a length that is substantially longer than its width and depth. The elongated length of the recess 3060 in this form is oriented laterally, i.e. the length of the recess 3060 extends in the lateral direction compared to the patient's body when in use, while the shorter width of the recess 3060 is oriented in the superior-inferior direction and the depth of the recess 3060 is oriented in the anterior-posterior direction. In some forms, the recess 3060 may be arc-shaped to complement the shape of an arc-shaped brace 3900. For example, in the form of Fig. 5, the anterior part of the recess 3060 is formed by a wall of the nasal portion 3050-2 which is arc-shaped when viewed in a direction perpendicular to the transverse plane of the patient. This is equivalent to viewing in a superior-inferior direction. In some forms, the recess 3060 may be parallel to the Frankfort horizontal (see Fig. 2E). In other forms, the recess may deviate (e.g. curve) along its length in a superior-inferior direction such that the recess is not entirely parallel to the Frankfort horizontal. For example, in the case of the recess 3060 shown in Fig. 5, lateral ends of the recess 3060 are superior to a central region of the recess 3060 that lies on the mid-sagittal plane of the patient during use. In this way, the recess 3060 forms a U-shape when viewed from the front (i.e. in an anterior view). The U-shape in certain forms may be shallow, i.e. the curvature of the recess 3060 when viewed from an anterior direction may be low.

The recess 3060 may be located in an anterior wall portion 3054 of the nasal portion 3050-2. The anterior wall portion 3054 in which the recess 3060 is located may be positioned directly in front of (i.e. anterior to) the nasal region and/or lip superior region of the patient's face. In the form of Fig. 5, the recess 3060 is formed in a surface of the anterior wall portion 3054 of the nasal portion 3050-2 facing away from the patient during use, i.e. an anterior surface. In other forms, the recess 3060 may be formed in a surface of the anterior wall portion 3054 of the nasal portion 3050-2 facing towards the patient during use, i.e. a posterior surface. In certain forms, the recess 3060 may create a protrusion on the opposite side of the anterior wall portion 3054 of the nasal portion 3050-2 to the recess 3060, while in other forms the nasal portion 3050-2 may be formed such that the surface on the opposite side from the side in which the recess 3060 is formed does not have a protrusion and may be substantially flush with adjacent areas of the nasal portion 3050-2.

In some forms, the recess 3060 may be sized and shaped in such a way that it is suitable to receive any one brace 3900 of a plurality of braces at a time, where each of the plurality of braces 3900 has a different shape and/or size to the others. Differing braces 3900 are described in more detail below.

In some forms, the recess 3060 may be located in an inferior region of the nasal portion 3050-2 or, in some forms, between the nasal portion 3050-2 and the mouth portion 3050-1 of the cushion 3050. For example, as shown in Fig. 5, the lateral outer edge of the cushion 3050 may have a substantial change in gradient between the nasal portion 3050-2 and the mouth portion 3050-1. The nasal portion 3050-2 may be narrower in lateral width than the mouth portion 3050-1 and the outer profile of the cushion 3050 may bend outwardly from the nasal portion 3050-2 moving down to the mouth portion 3050-1. The recess 3060 may be positioned level, or approximately level, with the substantial change in gradient between the nasal portion 3050-2 and the mouth portion 3050-1.

In other forms, as described in more detail below in section 4.3.5.4.2, the plenum chamber 3200 may additionally or alternatively comprise a groove 3203 which receives a portion of the positioning and stabilising structure 3300 in use. In some forms, the same structural form of the plenum chamber 3200 may act as both a groove 3203 and the recess 3060 as they are described in this specification, i.e. a single groove 3203/recess 3060 may be configured and constructed to provide the function of contacting a part of the positioning and stabilising structure 3300 or for receiving brace 3900 depending on the particular patient's 1000 requirements or setup, for instance to achieve the best fit of the patient interface 3000.

### 4.3.3.1.2 Mouth portion

In certain forms of the technology, such as illustrated in Figs. 3A, 4A and 5, 6B to 6D, 7B to 7D,8B to 8D, and 13A to 17B, the cushion 3050 may comprise a mouth portion 3050-1. The mouth portion 3050-1 is a portion of the cushion 3050 that is configured to be positioned, in use, proximate to the patient's mouth. In certain forms, the mouth portion 3050-1 is configured to be positioned, in use, proximate and anterior to the patient's mouth.

The mouth portion 3050-1 may comprise a part of the seal-forming structure 3100 that is configured, in use, to form a seal with a region of the patient's face surrounding, or part surrounding, the entrance to the patient's mouth. It should be understood that the mouth portion 3050-1 may be configured to partly (as opposed to entirely) surround the entrance to the patient's mouth in some forms because, in such forms, the cushion 3050 may additionally comprise a nasal portion 3050-2 that, in combination with the mouth portion 3050-1, surround the entrance to the mouth in use, and the mouth portion 3050-1 itself may only surround the mouth partly, for example around inferior and lateral regions of the mouth. In other forms, the mouth portion 3050-1 may be configured to form a seal with a region of the patient's face entirely surrounding the entrance to the patient's mouth.

The mouth portion 3050-1 may also form at least part of the plenum chamber 3200. Accordingly, at least part of the plenum chamber 3200 may be situated, in use, proximate the mouth region of the patient's face, e.g. proximate and anterior to the mouth region. In some forms, the mouth portion 3050-1 may form the plenum chamber 3200 along with the nasal portion 3050-2 and, if present, the frame 3270.

In some forms, such as those illustrated in Figs. 3A, 4A and 5, 6B to 6D, 7B to 7D and 8B to 8D, the mouth portion 3050-1 and the nasal portion 3050-2 of the cushion 3050 may be integrally formed together. For example, a cushion 3050, comprising both a mouth portion 3050-1 and a nasal portion 3050-2, may be injection moulded from silicone.

In the forms of the technology illustrated in Figs. 5, 6B to 6D, 7B to 7D and8B to 8D, the opening 3070 is provided in the mouth portion 3050-1 of the cushion.

### 4.3.3.1.3 Angles of seal-forming regions of nasal and mouth portions

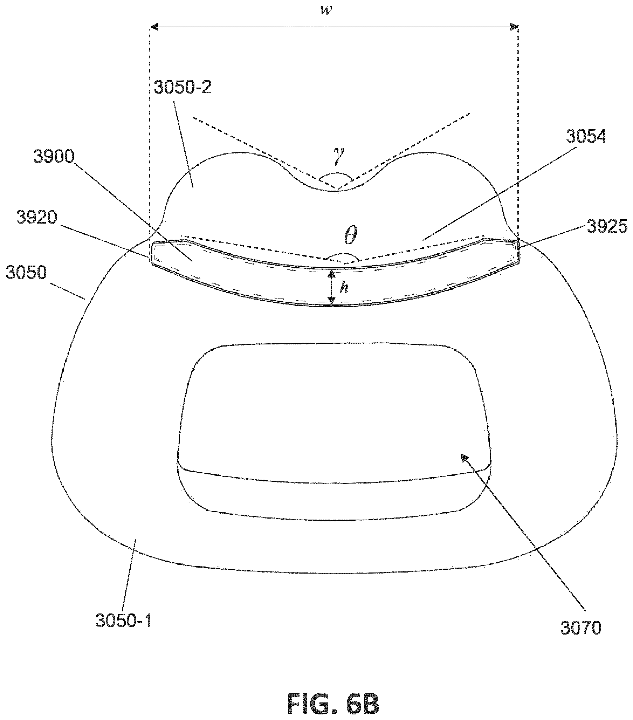

Figs. 6B to 6D show a cushion 3050 comprising a seal-forming structure 3100. As has been described, the cushion 3050 comprises a nasal portion 3050-2 and a mouth portion 3050-1. Both the nasal portion 3050-2 and the mouth portion 3005-1 comprise part of the seal-forming structure 3100. The seal-forming structure 3100 that is comprised as part of the nasal portion 3050-2 is configured to form a seal with a region of the patient's face around the nares and the seal-forming structure 3100 that is comprised as part of the mouth portion 3050-1 is configured to form a seal with a region of the patient's face around the mouth.

In certain forms of the technology, the shape of the nasal portion 3050-2, the shape of the mouth portion 3050-2 and the orientation of the nasal portion 3050-2 relative to the mouth portion 3050-1 complement the shape of the regions of the patient's face that they seal against in use, i.e. the regions around the patient's nares and mouth. This may make the patient interface 3000 comfortable to wear and provide an effective seal with the patient's face in use.

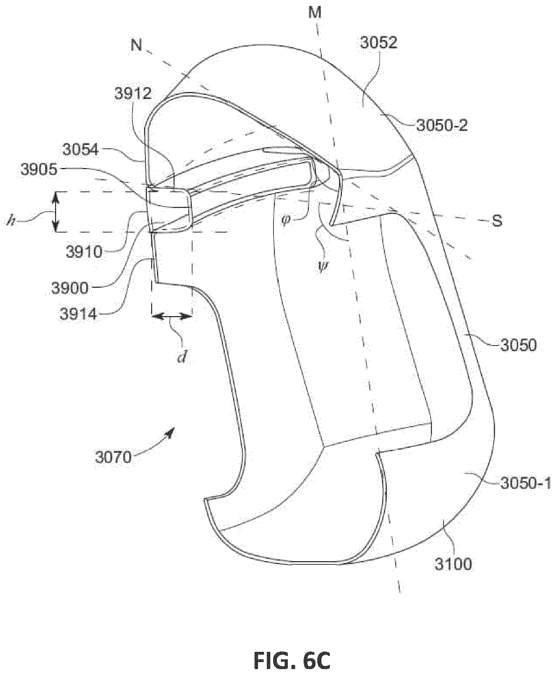

The side cross-sectional view of the cushion 3050 shown in Fig. 6C is a cross-sectional view through a plane corresponding to the mid-sagittal plane of the patient when the patient interface 3000 is appropriately positioned in use, i.e. the cross-section is a central slice through the cushion 3050 in a superior-inferior direction. **In** this plane, the parts of each of the nasal portion 3050-2 and mouth portion 3050-1 that form the target seal-forming region (i.e. those parts of the seal-forming structure 3100 that are configured to contact the patient's face in use) may lie on respective lines labelled as N and M in Fig. 6C. The lines N and M represent the orientation of the target seal-forming regions of the nasal portion 3050-2 and mouth portion 3050-1 respectively that are presented for contact with the patient's face that lie in the mid-sagittal plane in use. In the form shown in Fig. 6C, line M is tangential to a part of the target seal-forming region of the mouth portion 3050-1 that is positioned superior to an oral hole in the mouth portion in use, and is also tangential to a part of the target seal-forming region of the mouth portion 3050-1 that is positioned inferior to an oral hole in the mouth portion in use. In the form shown in Fig. 6C, line N is tangential to a relatively planar region of the target seal-forming region of the nasal portion 3050-2. Lines N and M may be oriented at an angle *φ* with respect to each other.

It will be appreciated that, in other cross-sections through the cushion 3050 in a superior-inferior direction, the parts of each of the nasal portion 3050-2 and mouth portion 3050-1 that form the target seal-forming region differ. In some forms, the relative orientations of the target seal-forming regions of the nasal portion 3050-2 and the mouth portion 3050-1 (i.e. the angle between lines equivalent to lines N and M) in these other cross-sections may also be the angle *φ* or a similar angle. In other forms, the relative angle between lines equivalent to these lines may differ.

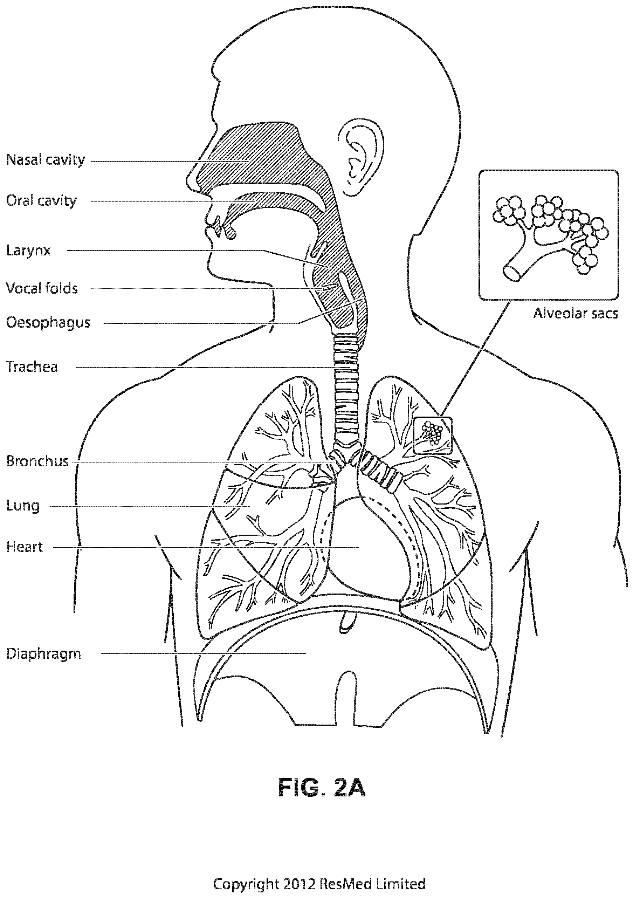

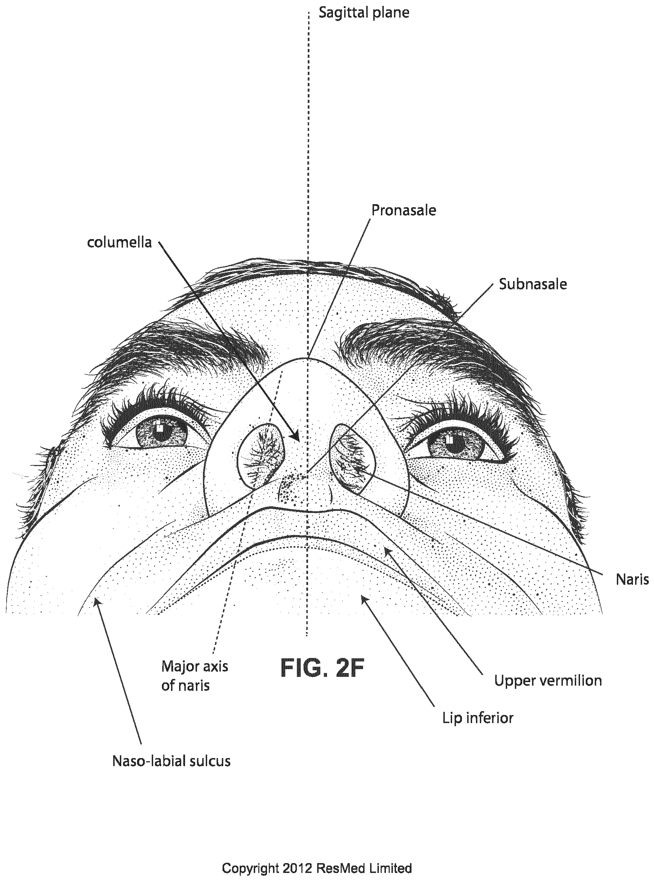

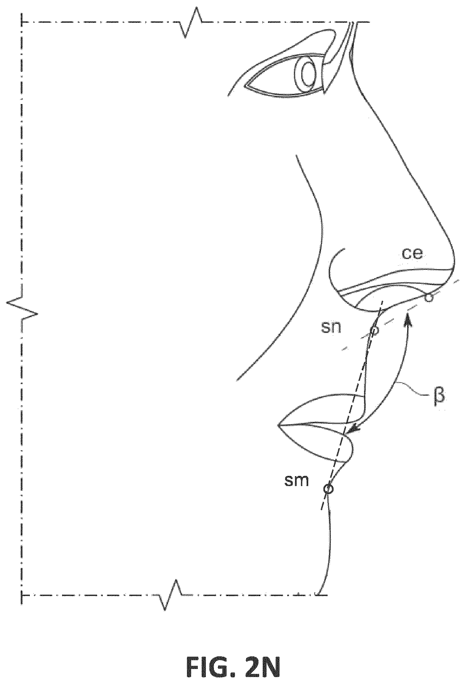

As shown in Figs. 2F and 2N, a human face may define three points: the superior-most point of the columella ce, the subnasal point sn and the supramenton sm. An angle *β* may be defined as the angle between a line joining ce and sn, and a line joining sn and sm when viewed from a lateral direction (as in Fig. 2N). The angle *β* may be referred to as the nose-supramenton angle. **In** certain forms, the cushion 3050 may be configured so that the angle *φ* (as described above and shown in Fig. 6C) is substantially similar to the angle *β* of the patient. This may promote a comfortable fit and/or a reliable sealing engagement of the seal-forming structure 3100 with the patient's face during use. In exemplary forms, the angle *φ* may be in the range of approximately 90° to approximately 150°, e.g. the angle *φ* may be obtuse.

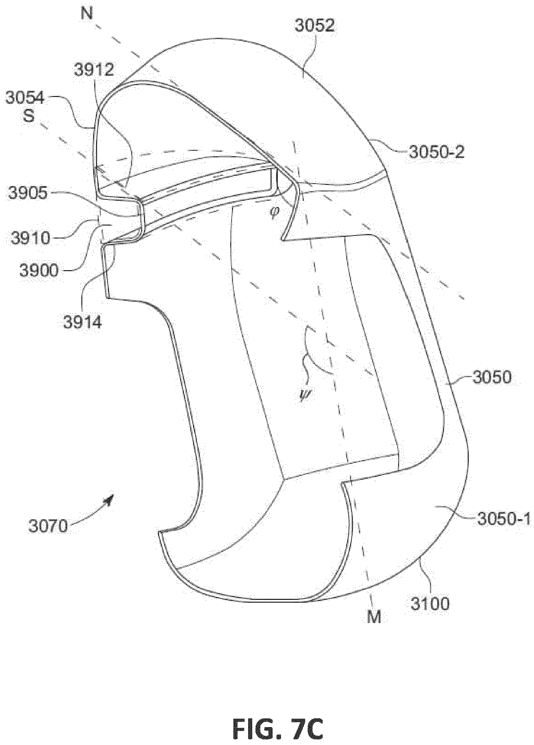

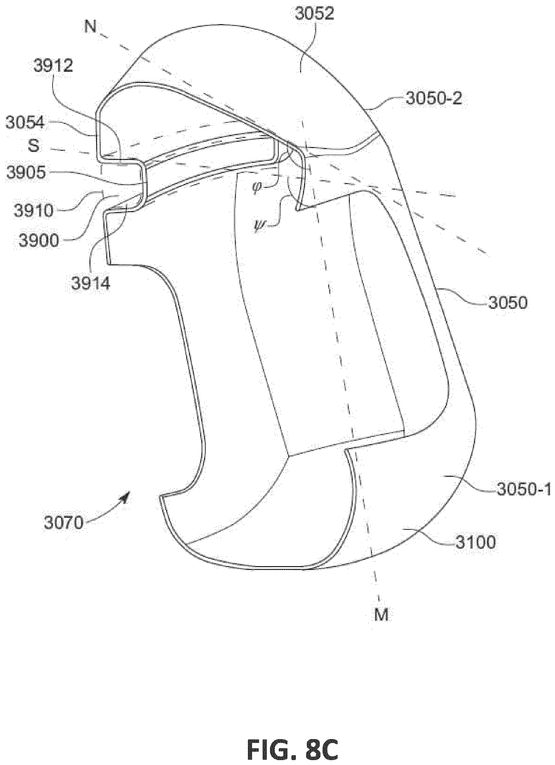

Figs. 7C and 8C show forms of cushion 3050 in which the angle *φ* between lines N and M differs from the angle shown in Fig. 6C. In Fig. 7C the angle *φ* is greater than the angle *φ* in Fig. 6C while in Fig. 8C the angle *φ* is less than the angle *φ* in Fig. 6C. A patient may select a patient interface 3000 with a cushion 3050 having an angle *φ* that meets certain preferences, for example is comfortable and/or provides an effective seal in use. For example, the patient may select a patient interface 3000 with a cushion 3050 having an angle *φ* that is similar to the nose-supramenton angle *β* of the patient's face.

The nasal portion 3050-2 of the cushion comprises a posterior wall portion 3052 that seals against regions of the patient's face surrounding the patient's nares during use, for example an inferior periphery of the patient's nose including an inferior and/or anterior surface of a pronasale region of the patient's nose and the patient's nasal alae. The posterior wall portion 3052 may be concave in at least one direction in order to provide an effective seal with these regions of the patient's face. In some forms, the posterior wall portion 3052 may be saddle-shaped with a one-dimensional positive curvature in the lateral direction (see Figs. 3G to 3I).

Figs. 5 and 6B mark an angle γ that may be generally indicative of the degree of positive curvature of the posterior wall portion 3052 in the lateral direction. Angle γ may be the angle subtended at the centre of the posterior wall portion 3052 (i.e. the part of the posterior wall portion 3052 lying in the mid-sagittal plane when the patient interface 3000 is worn) by the tips of the posterior wall portion 3052 that protrude towards the patient, are positioned laterally compared to the centre of the posterior wall portion 3052 and are configured to seal against the patient's nasal ala and/or cheek regions in use. It will be appreciated that, while Figs. 5 and 6B illustrate the cushion 3050 from the anterior direction, this view may not correspond to the plane of greatest curvature of the posterior wall portion 3052. In some forms, the angle γ may be understood to be the defined angle in the plane of greatest curvature of the posterior wall portion 3052 while in other forms the angle γ may be the defined angle in the plane shown in Figs. 5 and 6B.

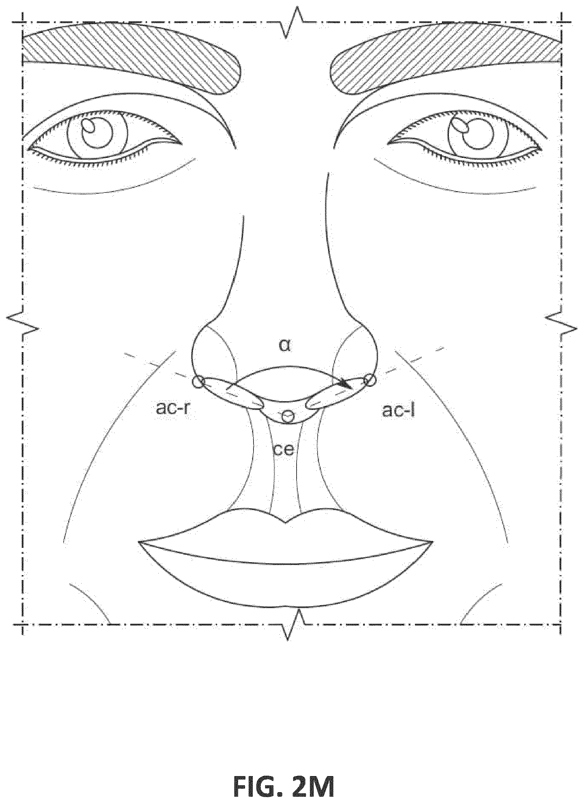

As shown in Figs. 2F and 2M, a human nose may define three points: the left alar curvature ac-l and the right alar curvature ac-r, and the superior-most point of the columella ce. An angle *α* may be defined as the angle between a line joining ac-l and ce, and a line joining ac-r and ce when viewed from an anterior direction (as in Fig. 2M). The angle *α* may be referred to as the alar angle. In certain forms, the nasal portion 3050-2 is configured so that the angle γ (which may be the angle may be generally indicative of the degree of positive curvature of the posterior wall portion 3052 in the lateral direction, as shown in Figs. 5 and 6B) is substantially similar to the angle *α* of the patient. This may promote a comfortable fit and/or a reliable sealing engagement of the seal-forming structure 3100 with the patient's face during use. In exemplary forms, the angle γ may be in the range of approximately 60° to approximately 160°, e.g. the angle γ may be obtuse.

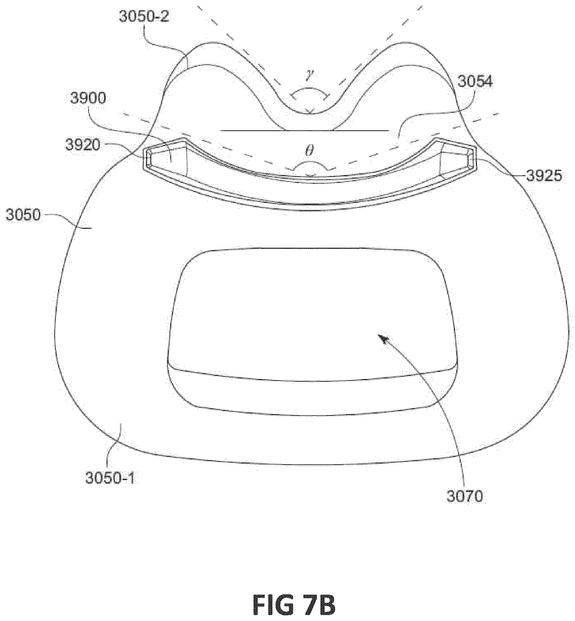

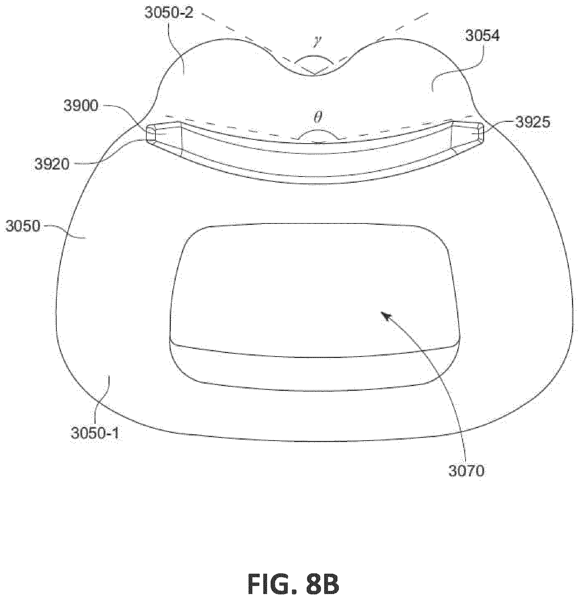

Figs. 7B and 8B show forms of cushion 3050 in which the angle γ differs from the angle shown in Figs. 5 and 6B. In Fig. 7B the angle γ is less than the angle γ in Fig. 6B while in Fig. 8B the angle γ is less than the angle γ in Fig. 6B. A patient may select a patient interface 3000 with a cushion 3050 having an angle γ that meets certain preferences, for example is comfortable and/or provides an effective seal in use. For example, the patient may select a patient interface 3000 with a cushion 3050 having an angle γ that is similar to the alar angle *α* of the patient's face.

### 4.3.3.2 Frame

The cushion module 3150 may comprise a frame 3270. The frame 3270 may be a component that provides structural shape, and may provide rigidity, to the cushion module 3150.

The frame 3270 may be constructed and configured to be more rigid than the cushion 3050. For example, the frame 3270 may be formed from a material having a higher Young's modulus than the material used to form the cushion 3050. Additionally, or alternatively, the frame 3270 may be configured in a shape that allows less flexibility than the cushion 3050.

The frame 3270 may, in combination with the cushion 3150, form the plenum chamber 3200. Accordingly, the frame 3270 may take the form of a component that defines in part the volume of space inside the patient interface 3000 that is pressurised in use and with which the patient exchanges air when respirating. As shown in Figs. 3A, 3I, 4A, 4B, 13A, and 14A the frame 3270 may take the form of a shell that partially encloses the volume inside the plenum chamber 3200 to varying degrees. In the case of the frames 3270 shown in Figs. 4A, 4B, 13A, and 14A the frame 3270 forms an anterior wall of the plenum chamber 3200 with the cushion 3050 forming substantial parts of the superior, inferior, lateral and posterior walls. In comparison, the frame 3270 of Fig. 3I encloses a greater part of the volume within the plenum chamber 3200 and the frame 3270 in this form defines the anterior wall and some regions of the superior and inferior walls of the plenum chamber 3200.

The frame 3270 may form a plenum chamber inlet port 3254 through which the flow of breathable gas is supplied to the plenum chamber 3200. The plenum chamber inlet port 3254 may be located in an anterior wall portion of the frame 3270, and may be located centrally, e.g. the mid-sagittal plane of the patient may pass through the plenum chamber inlet port 3254 when the patient interface 3000 is being worn. The plenum chamber inlet port 3254 may be circular. The air circuit 4170 may fluidly connect to the plenum chamber inlet port 3254, for example through a direct mechanical connection or via another component, for example an elbow or swivel.

Figs. 5, 6B to 6D, 7B to 7Dand 8B to 8D illustrate a cushion 3050 but do not illustrate a frame 3270. However, it should be understood that forms of the technology incorporating the cushions 3050 of Figs. 5, 6B to 6D, 7B to 7D and8B to 8D may additionally include a frame 3270. The frame 3270 in such forms may be sized and shaped to mount to the anterior part of the cushion 3050 and to span across the opening 3070. This may be similar to the way the frame 3270 of the forms illustrated in Figs. 4A and 4B are provided to the respective cushions in those forms.

In certain forms, for example the form illustrated in Fig. 5, the frame 3270 is connected to the mouth portion 3050-1 of the cushion 3050 and does not directly connect to the nasal portion 3050-2 of the cushion 3050. Consequently, the part of the plenum chamber 3200 formed by the nasal portion 3050-2 may be substantially flexible. For example, it may be entirely, or substantially entirely, formed from a material having a relatively low modulus of elasticity or Young's modulus.

### 4.3.3.3 Connection to positioning and stabilising structure

In certain forms, the cushion module 3150 is configured to connect, in use, to a positioning and stabilising structure 3300.

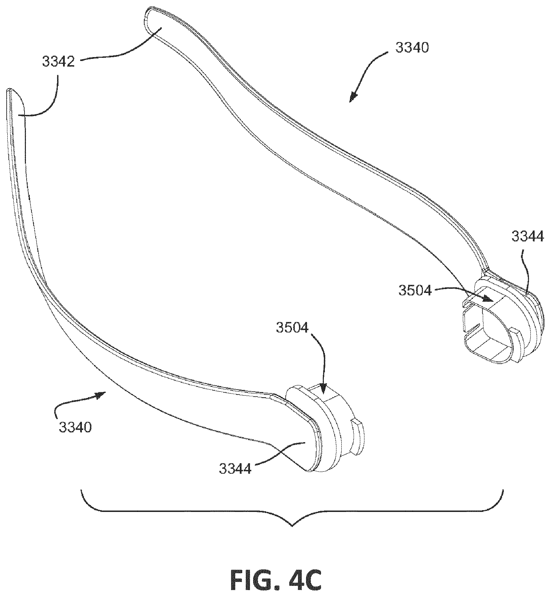

For example, in certain forms of the technology, the cushion module 3150 comprises a plurality of connectors to facilitate a connection of the cushion module to the positioning and stabilising structure 3300. The connectors may take the form of hooks, loops, buckles, clips, tabs or the like that are configured to connect to a part of the positioning and stabilising structure 3300, for example straps or rigidiser arms 3340. The cushion module 3150 may have one connector on each side lateral so that the positioning and stabilising structure 3300 can connect to the cushion module 3150 on each side of the patient's face. In some forms, the connectors are provided to, or are part of, the frame 3270. In other forms, the connectors are provided to, or are part of, the cushion 3050. In certain forms, the frame 3270 of the cushion module 3150 may comprise one or more openings configured to receive part of the positioning and stabilising structure 3300, for example an arm connection structure 3504 of the rigidiser arms 3340. An exemplary arm connection structure 3504 is illustrated in Fig. 4C.

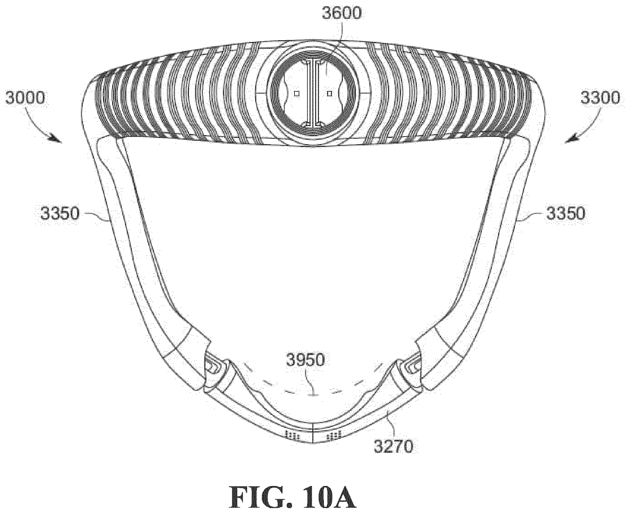

In certain forms of the technology, for example as illustrated in Figs. 3I and 10A, the cushion module 3150 comprises one or more openings that are configured to fluidly and mechanically connect to respective headgear tubes 3350 of the positioning and stabilising structure 3300. The openings in the cushion module 3150 may be located on each lateral side of the cushion module 3150 so that the positioning and stabilising structure 3300 can connect to the cushion module 3150 on each side of the patient's face. The connection between the openings and the tubes 3350 in such forms may serve the dual purpose of providing a mechanical connection to enable the positioning and stabilising structure 3300 to position and stabilise the seal-forming structure 3100 on the patient's face and facilitating delivery of the flow of air under therapeutic pressure to the patient's airways via the plenum chamber 3200.

In certain forms, the positioning and stabilising structure 3300 may comprise a frame configured to connect to the cushion module 3150. In some such forms, the cushion module 3150 may comprise no frame 3270 itself and the frame of the positioning and stabilising structure 3300 may serve the function of the frame 3270 of the cushion module 3150 in other forms. In other forms, the cushion module 3150 may comprise a frame 3270 in addition to the frame of the positioning and stabilising structure 3300. The frame of the positioning and stabilising structure 3300 may be positioned, in use, anterior to the cushion module 3150.

### 4.3.4 Brace

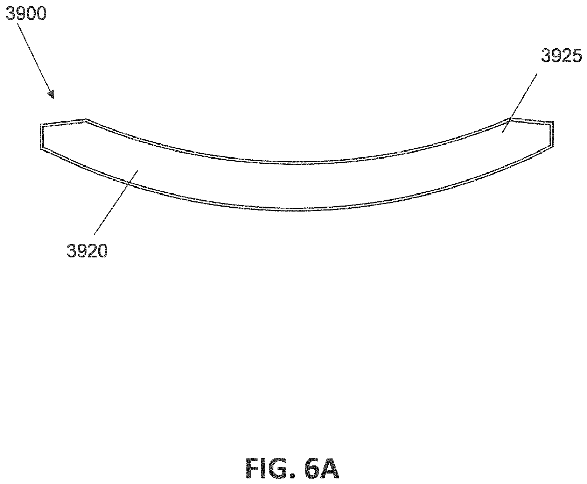

In certain forms of the technology, patient interface 3000 comprises a brace 3900. Brace 3900 may be configured to brace against movement of a certain part or certain parts of the patient interface 3000, during use. For example, the brace 3900 may brace against movement of a certain part or certain parts of the cushion module 3150 during use. For example, the forms of brace 3900 illustrated in Figs. 6A to 9C are configured to brace against movement of portions of the seal-forming structure 3100 in sealing engagement with regions of the patient's face proximate the patient's nares. The brace 3900 may additionally, or alternatively, be configured to brace against movement of portions of the cushion 3050 adjacent to the brace 3900 when the brace 3900 is mounted to the cushion 3050. These portions of the cushion 3050 may be adjacent to the seal-forming structure 3100 (i.e. those parts of the cushion 3050 in contact with the patient's face). In other examples, the patient interface 3000 may comprise a brace that braces against movement of other portions of the seal-forming structure 3100.

Additionally, or alternatively, the brace 3900 may have a different shape to the natural (i.e. undeformed) shape of the region of the cushion 3050 to which the brace 3900 is mounted in use. Therefore, in certain forms, when the brace 3900 is mounted to the cushion 3050, for example the nasal portion 3050-2, the portion of the cushion 3050 to which the brace 3900 is mounted deforms relative to its natural shape. This may assist in improving the sealing engagement between the seal-forming structure 3100 and the patient's face for any given cushion 3050. Consequently, the brace 3900 may be considered to brace the cushion 3050 into a new shape. The brace 3900 may alternatively be referred to as an "adaptor". It should be understood that, unless the context clearly indicates otherwise, when referred to as an adaptor, the properties may be the same as the described properties of the brace.

It should be appreciated that, in forms of the technology in which the cushion module 3150 comprises a frame 3270 in addition to a brace 3900, the frame 3270 and the brace 3900 may be functionally and/or physically distinct components. For example, in the forms of the technology illustrated in Figs. 6A to 8D, the brace 3900 is not directly in contact with the frame 3270 (when the frame is mounted to the cushion 3050). In certain forms, the brace 3900 may function to brace against movement of the nasal portion 3050-2 during use while the frame 3270 may provide structural shape, and optionally rigidity, to the mouth portion 3050-1.

Features of exemplary forms of brace 3900 will now be described with reference to Figs. 6A to 6D. The brace 3900 in Fig. 6A is illustrated from an anterior view perspective when the brace 3900 is in the orientation it would be in during use. Figs. 6B to 6D show the brace 3900 mounted to the cushion 3050 of Fig. 5.

### 4.3.4.1 Shape of brace

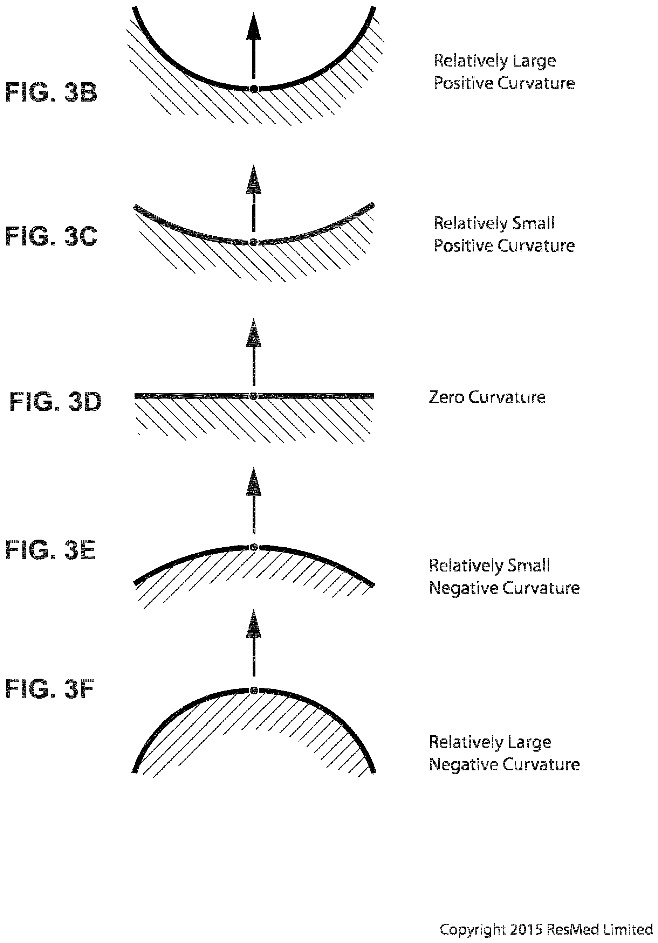

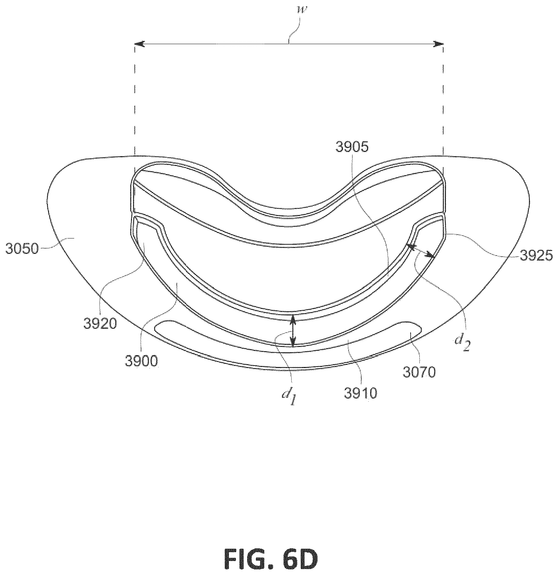

The brace 3900 may comprise a patient-facing surface 3905 that, in use, faces towards the patient. The brace 3900 may also comprise a non-patient-facing surface 3910 that, in use, faces away from the patient during use. The patient-facing surface 3905 may be concave, as illustrated in Fig. 6D. That is, when oriented in the intended position during use, the one-dimensional curvature of the patient-facing surface 3905 in cross-section through a plane parallel to the Frankfort horizontal (see Fig. 2E) is positive (see Figs. 3B and 3C). This cross-section is the top (or superior) cross-sectional view of the cushion 3050 and brace 3900 illustrated in Fig. 6D. In some forms, for example, the forms illustrated in Fig. 6A to 6D, the non-patient-facing surface 3910 may be convex, as illustrated in Fig. 6D. That is, when oriented in the intended position during use, the one-dimensional curvature of the non-patient-facing surface 3910 in cross-section through a plane parallel to the Frankfort horizontal (see Fig. 2E) is negative (see Figs. 3E and 3F). This cross-section is the top (or superior) cross-sectional view of the cushion 3050 and brace 3900 illustrated in Fig. 6D. In other forms, the non-patient-facing surface may have a different shape, for example it may be substantially planar (i.e. having a substantially zero curvature in cross-section when viewed from in a top cross-sectional view).

In forms in which the non-patient-facing surface 3910 of the brace 3900 is convex and the patient-facing surface 3905 is concave, the brace 3900 may be arc-shaped when viewed in a direction perpendicular to the transverse plane of the patient (or equivalently when viewed in a direction perpendicular to the Frankfort horizontal). For example, the brace 3900 illustrated in Figs. 6A to 6D is arc-shaped. In some forms, for example as illustrated in Figs. 6A to 6D, the curvature of the non-patient-facing surface 3910 of the brace 3900 may be similar in shape, but opposite, to the concave curvature of the patient-facing surface 3905. Consequently, the brace 3900 may have a relatively constant depth along its width (the depth and width of brace 3900 is explained further below).

In certain forms of the technology, for example the form illustrated in Figs. 6A to 6D, the brace 3900 is elongate. That is, the brace 3900 may be significantly longer in one direction than in another direction. In the case of the brace 3900 illustrated in Figs. 6A to 6D, the brace 3900 has a significantly longer width than its depth or width. The brace 3900 may be configured to mount to the nasal portion 3050-2 of the cushion 3050 in an orientation such that the brace is elongate in the lateral direction with respect to the patient when the patient interface 3000 is being used. That is, the longest dimension of the brace 3900, i.e. the width, may be arranged to extend laterally with respect to the patient. That is, the longest dimension of the brace 3900 may be perpendicular to the mid-sagittal plane of the patient. The brace 3900 may have a first end 3920 and a second end 3925 at the opposite end from the first end 3920.

The brace 3900 of certain forms of the technology, for example as shown in Figs. 6A to 6C, is elongate and has a cross-sectional shape that is substantially rectangular. As such, the brace 3900 may form a shape that may be described as an arc-shaped rectangular prism. The edges of the prism (i.e. the corners of the rectangular cross-section) may be rounded. A brace 3900 having this form may have a patient-facing surface 3905, a non-patient-facing surface 3910, a superior surface 3912 and an inferior surface 3914 (see Figs. 6C, for example). In other forms, the cross-sectional shape of the brace 3900 may be different, for example, it may be trapezoidal, triangular, circular, or oval. The cross-sectional shape of the brace 3900 may have rounded corners in these forms so that the brace 3900 has rounded edges.

### 4.3.4.2 Parameters indicative of shape of brace

Figs. 6B to 6D indicate certain parameters that are indicative of the shape of the brace 3900 in certain forms of the technology. These parameters will now be described in more detail. These parameters will be described with respect to the intended orientation of the brace 3900 with respect to the patient when the brace 3900 is mounted to the cushion 3050 in the intended orientation (i.e. as shown in Figs. 6B to 6D) and when the cushion 3050, which is part of patient interface 3000, is worn in the intended position, i.e. with the nasal portion 3050-2 in sealing engagement with an inferior region of the patient's nose and the mouth portion 3050-1 in sealing engagement with a region around the patient's mouth. It will be appreciated that, despite this orientation being used for ease of description, the brace 3900 may be formed separately to the rest of the patient interface 3000 and, as described later, may be detached and re-attached to the cushion 3005, therefore the described features of the brace are present whether or not the brace is in the described position and orientation.

In the illustrated form, the width w of the brace 3900 is the dimension of the brace 3900 in the lateral direction across the patient's face. This may be the straight-line distance from a first end 3920 of the brace 3900 to the second end 3920 of the brace. As explained earlier, the width may be the longest dimension of the brace 3900. In the illustrated form, the brace 3900 is oriented so that the width extends laterally with respect to the patient, i.e. perpendicular to the mid-sagittal plane of the patient. In certain forms, the width w may be in the range of approximately 40 mm to approximately 80 mm, for example approximately 60 mm.

In the illustrated form, the height h of the brace 3900 is the dimension of the brace 3900 in the superior-inferior direction with respect to the patient. This may also be the dimension of the brace 3900 in a direction that is perpendicular to a transverse plane of the patient's body. The height of the brace 3900 may be the distance between the superior surface 3912 and the inferior surface 3914. For the form of brace 3900 illustrated in Figs. 6A to 6D, the height of the patient-facing surface 3905 of the brace 3900 is approximately equal to the height of the non-patient-facing surface 3910 of the brace 3900. However, in other forms, such as described later, these heights may differ. For the form of brace 3900 illustrated in Figs. 6A to 6D, the height of the brace 3900 is approximately equal along the width of the brace 3900. In other forms, the height may vary along the width. For example, the height of the brace 3900 may be greater or narrower at or proximate the first end 3920 and/or the second end 3925 of the brace 3925. In certain forms, the height h may be in the range of approximately 5 mm to approximately 9 mm.

In the illustrated form, the depth d of the brace 3900 may be the dimension of the brace in a direction that is perpendicular to the width and the height of the brace 3900. For example, the depth of the brace 3900 may be the dimension of the brace in the anterior-posterior direction, or the dimension in a direction that is perpendicular to the frontal plane of the patient's body. In the form illustrated in Fig. 6D, the brace 3900 comprises a central region that lies on or near the mid-sagittal plane of the body during use. This central region has a depth *d₁* that is oriented as just explained. The depth of the brace 3900 may differ in orientation and/or magnitude along the width of the brace 3900. For example, the depth of the brace 3900 may be greater or narrower at or proximate the first end 3920 and/or the second end 3925 of the brace 3925. As another example, as illustrated in Fig. 6D, since the brace 3900 is arc-shaped, the depth *d₂* of the brace 3900 proximate the second end 3925 extends at a non-zero angle to the depth *d₁* of the brace 3900 at the central region, for example in the range of approximately 10° to approximately 80°. The magnitude of the angle between the direction of extent of the depth *d₂* of the brace 3900 proximate the second end 3925 compared to the depth *d₁* of the brace 3900 at the central region may be considered an indicator of the amount of curvature in the arc-shaped brace when viewed in a direction perpendicular to the transverse plane of the patient (i.e. the curvature of the patient-facing surface 3905 and the non-patient-facing surface 3910 as shown in Fig. 6D). In certain forms, the depth *d* may be in the range of approximately 3 mm to approximately 8 mm, for example approximately 5 mm.

In some forms, the brace 3900 may lie in a plane substantially parallel to the Frankfort horizontal along its width. In other forms, the superior surface 3912 of the brace 3900 may have a positive curvature in one dimension (see Figs. 3A and 3B) when viewed in a posterior direction, i.e. in an anterior or front view as shown in Figs. 6A and 6B. For example, as also shown in Figs. 6A and 6B, the brace 3900 may be U-shaped when viewed in a posterior direction. The 'U' may be oriented with the superior surface 3912 being the concave side of the 'U' and the inferior surface 3914 being the convex side of the 'U'. In such forms, an angle *θ* may be defined as an angle representative of the curvature of the U-shape of the brace 3900 when viewed in the stated direction. The angle *θ* may be the angle subtended at the centre of the superior surface 3912 by the superior parts of the first and second ends 3920 and 3925 of the brace 3900. The greater the curvature, the lesser the curvature. In some forms the angle *θ* may be in the range of approximately 100° to approximately 150°, e.g. the angle *θ* may be obtuse. The brace may have an angle *θ* of 180° when the brace is flat when viewed from the anterior side.

Another angle associated with the brace 3900 may be the angle of the superior surface 3912 of the brace 3900 relative to a plane of the target seal-forming region of the mouth portion 3050-1 of the cushion 3050. In certain forms of the technology, for example as shown in Fig. 6C, the superior surface 3912 of the brace 3900 slopes downwards towards the patient, i.e. downwards from the non-patient-facing surface of the brace to the patient-facing surface of the brace 3900. Consequently, the height *h* of the non-patient-facing surface 3910 of the brace may be greater than the height *h* of the patient-facing surface 3905 of the brace 3900. It will be understood that the superior surface 3912 of the brace 3900 and the target seal-forming region of the mouth portion 3050-1 may have non-planar surfaces, therefore this angle may be the angle between any two portions of these surfaces. For example, the angle in question may be the angle of a central region of the superior surface 3912 of the brace 3900 (i.e. a region of the superior surface 3912 lying on the mid-sagittal plane relative to the patient when the patient interface 3000 is worn) relative to a plane of the target seal-forming region of the mouth portion 3050-1 in a central region of the cushion 3050 (i.e. a region of the cushion 3050 lying on the mid-sagittal plane relative to the patient when the patient interface 3000 is worn). Such an angle is marked as *ψ* in the exemplary form illustrated in Fig. 6C. In this figure, *ψ* is the angle between line S and line M, where line S is a line indicating the orientation of the superior surface 3912 of the brace 3900 in the cross-section in the mid-sagittal plane shown in Fig. 6C, and line M is as defined earlier. In exemplary forms, the angle *ψ* may be in the range of approximately 90° to approximately 150°, e.g. the angle *ψ* may be obtuse.

It will be appreciated that angle *ψ* depends on the orientation in which the brace 3900 is mounted to cushion 3050, and also the orientation of the plane of the target seal-forming region of the mouth portion 3050-1 relative to the rest of the cushion 3050. Therefore, there may be another angle (not marked in Fig. 6C that is the angle between the patient-facing surface 3905 and the superior surface 3912 of the brace 3900. In some forms, the brace 3900 may be mounted such that the patient-facing surface 3905 of the brace 3900 is oriented substantially in the superior-inferior direction. Alternatively, the patient-facing surface 3905 of the brace 3900 may be oriented parallel to line M.

While *ψ* as shown in Fig. 6C is the angle between the orientation of the superior surface 3912 of the brace 3900 and the orientation of the plane of the target seal-forming region of the mouth portion 3050-1 in the mid-sagittal plane cross-section of the cushion 3050, there is also an angle between this surface and plane in other cross-sections in the superior-inferior direction, i.e. proximate the first end 3920 and second end 3925 of the brace 3900. The equivalent angle at the ends of the brace 3900 may, in some forms, be similar or the same as the angle *ψ* as shown in Fig. 6C in the mid-sagittal plane cross-section of the cushion 3050. In other forms, the angle may differ at the ends of the brace 3900.

### 4.3.4.3 Shape and position of brace compared to patient's face

The brace 3900 may be configured to be mounted to the cushion 3050 in a position so that the brace 3900 is positioned anterior to the patient's nares. The brace 3900 may, in some forms, also be positioned inferior to the patient's nares (as compared to the superior-inferior axis of the patient's body), for example directly anterior to the patient's lip-superior region. The mounting of the brace 3900 to the cushion 3050 will be described in more detail later.

The brace 3900 may have a width *w* that is substantially as wide as, or slightly wider than, the patient's nose. That is, the width w may be at least as wide as the distance between the lateral surfaces of the patient's nasal ala. In other forms, the width w may be at least as wide as the distance between the left alar curvature (or crest point) ac-l and the right alar curvature (or crest point) ac-r (see Fig. 2M). The brace may be positioned on the cushion 3050 so that, when the patient interface 3000 is worn, the brace 3900 is positioned so that the first end 3920 and the second end 3925 of the brace 3900 are positioned proximate the respective lateral surfaces of the patient's nasal ala and/or the respective left alar curvature and the right alar curvature. For example, the first end 3920 and the second end 3925 of the brace 3900 may be positioned slightly anterior to, slightly inferior to, and/or slightly lateral to these regions of the patient's face.

In certain forms, the brace 3900 is configured so that the angle *θ* (which may be the angle subtended at the centre of the superior surface 3912 by the superior parts of the first and second ends 3920 and 3925 of the brace 3900, as shown in Fig. 6B) is substantially similar to the alar angle *α* of the patient.

In the form of technology shown in Fig. 6C, the angle *φ* is different from the angle *ψ.* However, in some forms, these angles are substantially similar, i.e. the orientation of the superior surface 3912 of the brace 3900 may be substantially similar to the orientation of adjacent target seal-forming regions of the nasal portion 3050-2, e.g. a region of the nasal portion 3050-2 in the same superior-inferior plane. In certain forms, the orientation of the superior surface 3912 of the brace 3900 at or proximate the ends of the brace 3900 (i.e. first end 3920 and second end 3925) is substantially similar to the orientation of adjacent target seal-forming regions of the nasal portion 3050-2, i.e. lateral regions of the nasal portion 3050-2 positioned to seal against the nasal ala. Furthermore, as has been described earlier, these angles may in some forms be substantially similar to the nose-supramenton angle *β* of the patient as described earlier. As is described in more detail later in this specification, the two angles *φ* and *ψ* may be related, for example a patient interface 3000 having a brace 3900 with a larger angle *ψ* may result in a larger angle *φ* of the nasal portion 3050-2.

### 4.3.4.4 Rigidity / material of brace

In certain forms of the technology, the brace 3900 is constructed and configured to be relatively rigid when compared to the nasal portion 3050-2 of the cushion 3050. Consequently, the brace 3900 provides additional rigidity to the inherent relatively low level of rigidity of the nasal portion 3050-2 which, as has been explained, is constructed and configured to be flexible.

The relative rigidity of the brace 3900 compared to the nasal portion 3050-2 of the cushion 3050 may be achieved through the shape of the brace 3900, the material from which the brace 3900 is formed, or a combination of these two factors.

In some forms, the brace 3900 is made from a material having an elastic modulus (e.g. Young's modulus) that is greater than the respective modulus of the material used to form the nasal portion 3050-2. In certain forms, the brace 3900 is made from a material having a Young's modulus of approximately 500 Mpa to approximately 1800 Mpa. In examples, the brace 3900 may be made from high-duro silicone, plastic (e.g. polypropylene, hytrel), foam or gel providing a suitable level of higher rigidity compared to the nasal portion 3050-2.

In certain forms, the brace 3900 may be configured to be deformable, for example plastically deformable. This may enable a patient (or their health professional) to adjust the shape of the brace 3900 to suit their preferences (e.g. comfort and/or sealing fit). In one example, the brace 3900 may be formed of a plastic material in combination with an elongate metal member (e.g. a wire). The metal member may be attached to, for example embedded inside, the plastic material. The metal member may be plastically deformable so that the brace 3900 retains a new shape when deformed.

### 4.3.4.5 Mounting of brace to cushion

The brace 3900 may be mounted to the cushion 3050, for example to the nasal portion 3050-2 of the cushion 3050. In certain forms, the brace 3900 is mounted to an anterior wall portion of the cushion 3050, for example in the form illustrated in Figs. 6B to 6D, the brace 3900 is mounted to the anterior wall portion 3054 of the nasal portion 3050-2.

In some forms, the brace 3900 may be mounted to an inferior region of the nasal portion 3050-2 of the cushion 3050. In other forms, the brace 3900 may be mounted between the nasal portion 3050-2 and the mouth portion 3050-1. Again, the brace 3900 may be mounted to an anterior wall portion of the cushion 3050 when mounted in such locations.

In certain forms, the brace 3900 is mounted to the cushion 3050 in a manner whereby the brace 3900 has a bracing effect on regions of the cushion 3050 adjacent the brace 3900 when it is mounted to the cushion 3050. This may be achieved, for example, by mounting the brace 3900 to the cushion 3050 in a manner which holds substantially all parts of the brace 3900 in a fixed position relative to regions of the cushion 3050 adjacent to the respective parts of the brace 3900. For example, the brace 3900 may be mounted in a manner whereby substantially all of the patient-facing surface 3905 of the brace 3900 may be in contact with the cushion 3050 and each part of the patient-facing surface 3905 of the brace 3900 is held in fixed position relative to the part of the cushion 3050 with which it is in contact.

In certain forms, the brace 3900 is mounted to a patient-facing (posterior) surface of the anterior wall portion of the cushion 3050. In other forms, the brace 3900 is mounted to a non-patient-facing (anterior) surface of the anterior wall portion of the cushion 3050. In still other forms, the cushion 3050, may comprise one or more pockets for receiving the brace 3900 and holding the brace 3900 in place relative to the cushion 3050. The pocket may have an opening into which the brace 3900 may be inserted, and in some forms the pocket may be provided in a patient-facing (posterior) surface of the anterior wall portion of the cushion 3050 while in other forms the pocket may be provided in a non-patient-facing (anterior) surface of the anterior wall portion of the cushion 3050.

It has been described earlier in this specification that the nasal portion 3050-2 of the cushion 3050 may form a recess 3060 into which the brace 3900 may be positioned in order to mount the brace 3900 to the nasal portion 3050-2.

In some forms, the brace 3900 may be mounted to the cushion 3050 via a friction fit. For example, in forms in which the nasal portion 3050-2 forms a recess 3060 into which the brace 3900 is positioned, the recess 3060 may be sized so that the brace 3900 fits securely with a friction fit. In other forms, the brace 3900 may be mounted to the cushion 3050 using an alternative mounting mechanism, for example adhesive, hook-and-loop connectors, clips, interlocks, etc. In some forms the mounting mechanism is one that enables the brace 3900 to be readily removed and subsequently re-mounted to the cushion 3050.

In certain forms, the brace 3900 may have a different shape to the natural (i.e. undeformed) shape of the region of the cushion 3050 to which the brace 3900 is mounted in use. Therefore, in certain forms, when the brace 3900 is mounted to the cushion 3050, for example the nasal portion 3050-2, the portion of the cushion 3050 to which the brace 3900 is mounted deforms relative to its natural shape. In particular, a seal-forming surface of the cushion 3050 may be deformed, for example its orientation may be adjusted. This may improve the sealing engagement between the cushion 3050 and the patient's face, e.g. the deformation may alter the shape of the seal-forming surface to be more similar to a part of the patient's face than when undeformed. For example, in some forms the brace 3900 may have a greater or lesser degree of curvature across its patient-facing surface 3905 than the degree of curvature in the same plane as the part of the cushion 3050 to which the brace 3900 mounts (which may be, for example, the recess 3060 described earlier) when the cushion 3050 is undeformed. Consequently, when the brace 3900 is mounted to a part of the cushion 3050, since the cushion 3050 is flexible, that part of the cushion is deformed by the brace 3900. This may enable the position and/orientation of the seal-forming surfaces of the cushion 3050 to be altered when the brace 3900 is mounted to the cushion 3050 compared to when it is not. For example, the relative orientation of the seal-forming surface of the nasal portion 3050-2 (i.e. the posterior wall portion 3052) and the seal-forming surface of the mouth portion 3050-1, for example as indicated by angle *φ*, may be altered by selecting a brace 3900 having a particular orientation of superior surface 3912 and mounting it to the cushion 3050 (e.g. because the brace 3900 may have the effect of pushing upwards on and distorting the nasal portion 3050-2 so as to change the orientation of its seal-forming surface). In another example, the amount of curvature of the posterior wall portion 3052 of the nasal portion 3050-2, for example as indicated by angle y, may be altered by selecting a brace 3900 having a particular degree of curvature of its patient-facing surface 3905 and/or its superior surface 3912 (for example, as indicated by the degree of curvature from the perspective of the superior view of brace 3900, as shown in Fig. 6D, or as indicated by angle *θ*) and mounting it to the cushion 3050. In other examples, a brace 3900 with a suitable width, height and/or depth may be selected and, when mounted to the cushion 3050, the brace 3900 may alter the size and shape of the cushion 3050, which may include the seal-forming region of nasal portion 3050-2, accordingly.

A good fit, e.g. a good sealing engagement between the seal-forming structure 3100 and the patient's face, may be able to be achieved by selecting an appropriately sized and shaped brace 3900 and mounting it to the cushion 3050. This may be a factor in achieving a good fit in addition to selecting an appropriately sized and shaped cushion 3050. In some forms, it may be a more important factor than selecting an appropriately sized and shaped cushion 3050.

### 4.3.4.6 Function of brace

It has already been explained that, in certain forms of the technology, the brace 3900 may be configured to brace against movement of a certain part or certain parts of the patient interface 3000 during use, for example the portions of the seal-forming structure 3100 in sealing engagement with regions of the patient's face proximate the patient's nares. Consequently, the brace 3900 may function to maintain the seal-forming structure 3100 in sealing engagement with the patient's face during use. In particular, the brace 3900 may function to maintain the seal-forming parts of the nasal portion 3050-2 in sealing engagement with regions of the patient's face proximate the patient's nares. The brace 3900 may promote the maintenance of this sealing engagement despite movement of the patient's face or despite the cushion 3050 having a size and/or shape that may make maintaining sealing engagement with the patient's face during use difficult, for example if the seal-forming structure 3100 is of a size and/or shape that does not fit the patient's face well.

Additionally, or alternatively, as has been described, the brace 3900 may function to alter the shape of the cushion 3050 when the brace 3900 is mounted to the cushion 3050. This may mean that a single size and/or shape of cushion 3050 may be suitable for use with patients having different facial shapes. Alternatively, a manufacturer may be able to produce fewer different size/shape cushions while being able to accommodate the facial shapes of the same number of people as would otherwise be the case.

Additionally, or alternatively, the brace 3900 may tend to decouple movement of the mouth portion 3050-1 of the cushion 3050 from the nasal portion 3050-2. Therefore, distortion to one of the mouth portion 3050-1 or nasal portion 3050-2 during use, which may affect the sealing engagement between the respective portion and the patient's face, may not result in a disturbance between the sealing engagement of the other portion and the patient's face.

### 4.3.4.7 Alternative brace shapes

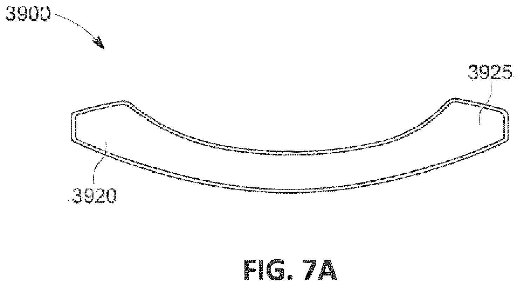

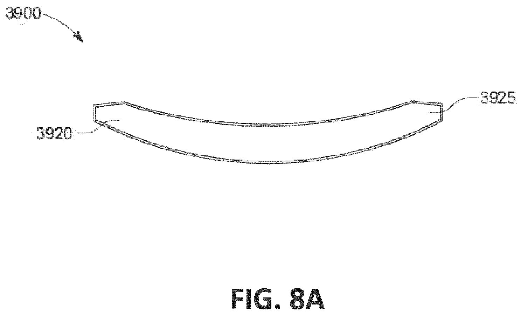

Figs. 7A and 8A show two braces 3900 according to other forms of the technology. The braces 3900 shown in these figures are different in shape, and may be different in size, to the brace 3900 shown in Figs. 6A to 6D but they may be configured in a similar way and serve a similar function. Consequently, except where differences are explicitly pointed out, the earlier description of features of the brace 3900 shown in Figs. 6A to 6D will be understood to similarly apply to the braces in Figs. 7A and 8A. Figs. 7B to 7D and Figs. 8B to 8D illustrate cushions 3050 with the respective braces of Figs. 7A and 8A mounted to them.

The shape and size of the brace 3900 may be selected to suit a particular patient. Examples of ways in which properties of a brace 3900 may be selected to suit patients are provided below.

The three different braces 3900 shown in Figs. 6A to 8D may differ in any one or more of the width w, height *h* and depth *d* explained above. A brace 3900 that is relatively wide may be more suitable for a patient having a relatively wide nose compared to a brace 3900 that has a relatively low width, for example. The relative proportions of these quantities may also differ between braces in different forms of the technology, as well as the relative proportions of these quantities between different regions of the braces.

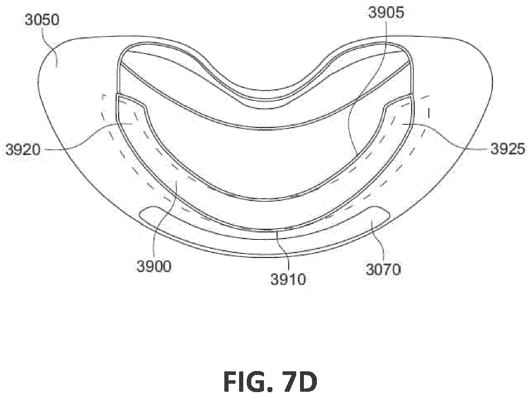

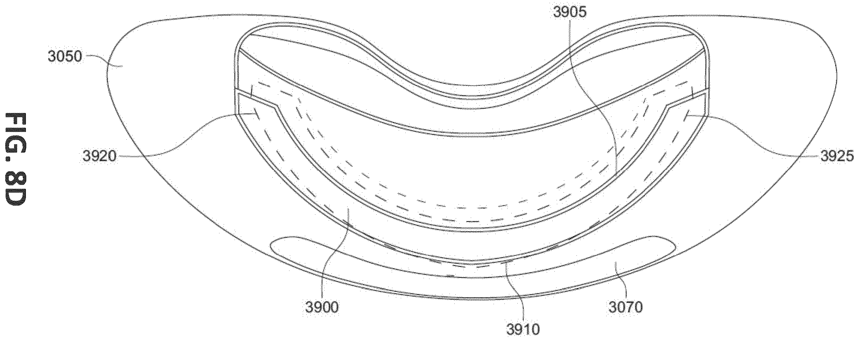

As can be seen in Figs. 6D, 7D and 8D, the braces 3900 have different amounts of curvature of the patient-facing surfaces 3905 of the braces 3900. The brace 3900 in Fig. 7D has a larger curvature of the patient-facing surface 3905 compared to the brace 3900 in Fig. 6D, while the brace 3900 of Fig. 8D has a lower curvature. This differing amount of curvature may be achieved by forming the braces 3900 with generally parallel patient-facing and non-patient facing surfaces 3905 and 3910 but with differing degrees of curvature to these surfaces between the braces. Alternatively, or additionally, the depths of the braces may be configured to achieve the desired difference in curvature. For example, one brace 3900 may have a first end 3920 and a second end 3925 that have a greater depth *d₂* than the first and second ends of another brace 3900. Additionally, or alternatively, the depth *d₂* of the first end 3920 and the second end 3925 of one brace 3900 may be greater relative to the depth *d₁* of a central region of the brace to a greater extent than the relative depths of the same regions of another brace 3900. Put another way, one brace 3900 may have deeper ends relative to the rest of the brace than another brace 3900. A brace 3900 with a larger curvature of the patient-facing surface 3905 may be more suitable for use by a patient having a thin or pointy nose, while a brace 3900 with a smaller curvature of the patient-facing surface 3905 may be more suitable for use by a patient having a wider or flatter nose.

Another difference between the three different braces 3900 shown in Figs. 6A to 8D is the degree of curvature of the superior surface 3912 of the brace 3900 when viewed in a posterior direction (as in Figs. 6B, 7B and 8B), as indicated by the angle *θ* labelled in these figures and explained earlier. The brace 3900 in Fig. 7B has a higher curvature of the patient-facing surface 3905 compared to the brace 3900 in Fig. 6D, while the brace 3900 of Fig. 8B has a lower curvature. This difference between the braces may be achieved in a number of ways, including altering the relative height *h* of a brace at its central region compared to at its first and second ends 3920 and 3925. The degree of curvature of the superior surface 3912 of a brace 3900 to be used by a particular patient may be selected based on the alar angle *α* (i.e. the angle between a line joining ac-l and ce, and a line joining ac-r and ce when viewed from an anterior direction for the patient, as shown in Fig. 2M). For example, the brace 3900 may be selected so that the angle *θ* of the brace 3900 is substantially similar to the alar angle *α* of the patient.

Another difference between the three different braces 3900 shown in Figs. 6A to 8D is the orientation of the superior surfaces 3912 compared to other surfaces of the brace 3900. For example, as shown in Figs. 6C, 7C and 8C, the superior surfaces 3912 may be oriented by a different amount compared to a plane of the target seal-forming region of the mouth portion 3050-1 in a central region of the cushion 3050 (i.e. a region of the cushion 3050 lying on the mid-sagittal plane relative to the patient when the patient interface 3000 is worn), as indicated by the angles labelled as *ψ* between lines S and M in each illustration. The angle *ψ* of the form shown in Fig. 7C is greater than the angle *ψ* of the form shown in Fig. 6B while the angle *ψ* of the form shown in Fig. 8C is less than the angle *ψ* of the form shown in Fig. 6B. The relative orientation of the superior surface 3912 may be selected based on the nose-supramenton angle *β* between a line joining ce and sn, and a line joining sn and sm when viewed from a lateral direction for the patient (as shown in Fig. 2N). For example the brace 3900 may be selected so that the angle *ψ* is substantially similar to the nose-supramenton angle *β* of the patient. Alternatively, the angle *ψ* may not be similar to the nose-supramenton angle *β* of the patient but may be greater or lesser depending on the magnitude of the nose-supramenton angle *β* of the patient. For example, there may be a positive correlation between the angles *ψ* and β for achieving good level of comfort and/or sealing engagement in use. In certain examples, a brace 3900 may be selected so that the orientation of the superior surface 3912 of the brace may cause the orientation of the seal-forming structure of the nasal portion 3050-2 to be substantially similar to the nose-supramenton angle *β* of the patient when the brace 3900 is mounted to the cushion 3050. It has already been explained that the orientation of the seal-forming structure of the nasal portion 3050-2 may be affected by the shape of the brace 3900, and in particular the orientation of the superior surface 3912 of the brace, so this relationship may be used to create a good fit with the patient's nose. For example, a brace 3900 may be selected that has an angle *ψ* where, when the brace 3900 is mounted to the cushion 3050, causes the angle *φ* of the nasal portion 3050-2 to be substantially similar to the nose-supramenton angle *β* of the patient.

### 4.3.4.8 Interchangeability of brace

In certain forms, the brace 3900 may be mounted to the cushion 3050 in a manner that allows the brace 3900 to be removed from the cushion 3050 and subsequently re-mounted to the cushion. Furthermore, the brace 3900 may be replaced with a replacement brace 3900. The brace and the replacement brace may be different in size and/or shape. In other words, the cushion module 3150 may be configured such that a plurality of different braces 3900 may be interchangeably mounted to the cushion 3050.

Ways of mounting the brace 3900 to the cushion 3050 in a removable and interchangeable manner have been described earlier.

In this way, a patient may be able to choose from a range of braces 3900, and optionally from a range of cushions 3050, in order to select the brace and/or the cushion (and thereby the combination of brace and cushion) that suits them, for example provides a comfortable fit and/or a good seal during use. This selection may be made in consultation with an appropriate health professional.

In certain forms, fewer different types of cushion 3050 may be provided compared to the number of braces 3900 that may be provided. This may be because a brace 3900 may be quicker and easier to manufacture than a cushion 3050.

In certain forms, a brace 3900 may be able to be 3D-printed. The shape of the brace 3900 to be 3D-printed may be selected to be customised to a particular patient. The patient's nasal and/or mouth region may initially be scanned by a suitable shape detection device and a suitable shape of brace 3900 may be determined from the resulting scan.

### 4.3.4.9 Types of mask

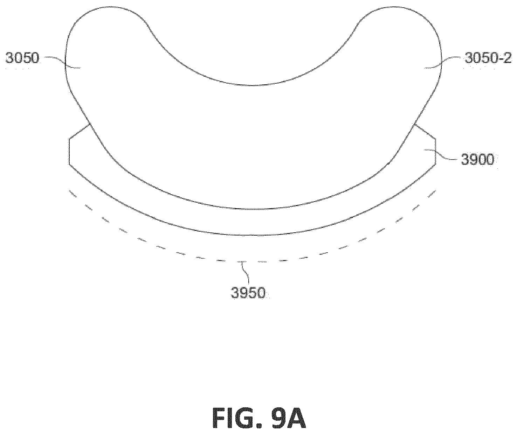

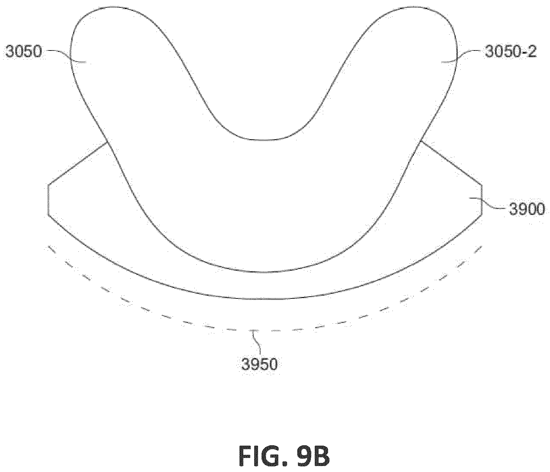

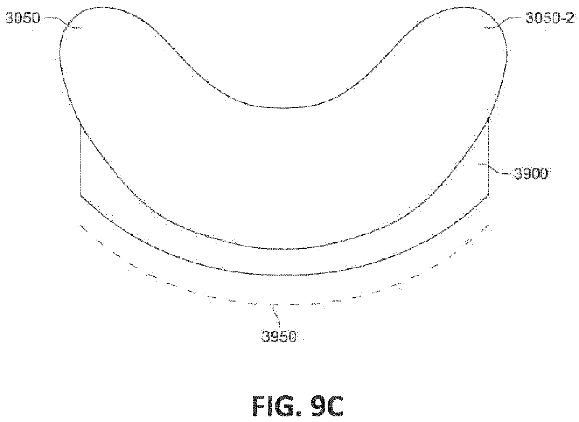

The exemplary forms of patient interfaces 3000 comprising braces 3900 illustrated in Figs. 6A to 8D are nose and mouth masks. However, in other forms of the technology, for example those illustrated in Figs. 9A to 9C, a brace 3900 may be provided to nose only masks, for example masks that may be identified as "nasal masks" or "nasal cradle masks" (as explained earlier).

The nose only masks in Figs. 9A to 9C comprise a cushion 3050 comprising a nasal portion 3050-2 and a brace 3900. Features of these components may be the same or similar to those described earlier in relation to the nose and mouth masks of Figs. 6A to 8D and the earlier disclosure will be understood to apply to these forms of the technology as well, although it will be appreciated that the cushions 3050 of the forms shown in Figs. 9A to 9C do not comprise mouth portions.

The braces 3900 of the forms shown in Figs. 9A to 9C may differ in a similar way to what is described earlier, and again a brace 3900 may be selected to suit a particular patient's needs and/or preferences.

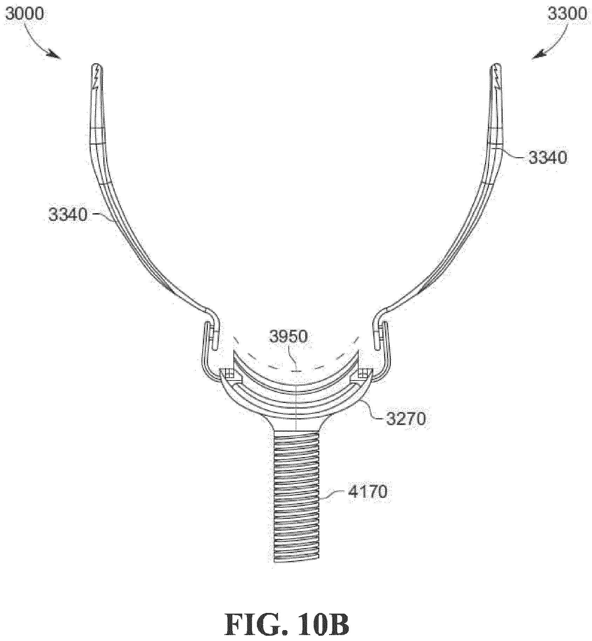

Figs. 10A and 10B show other components of patient interfaces 3000 in the form of nose only masks according to certain forms of the technology.

For example, the patient interface 3000 of Fig. 10A comprises a positioning and stabilising structure 3300 in the form of "conduit headgear", i.e. comprising tubes 3350 to deliver pressurised air from a connection port 3600 to the patient's airways (such as described elsewhere in this specification). The connection port 3600 may be positioned, in use, in a region of the patient's head superior to an otobasion superior of the patient's head. The patient interface 3000 of Fig. 10A further comprises a cushion module 3150 comprising a frame 3270 which, together with cushion 3050 (not shown in Fig. 10A), forms plenum chamber 3200. The tubes 3350 fluidly connect to openings on lateral sides of the frame 3270.

The patient interface 3000 of Fig. 10B comprises a positioning and stabilising structure 3300 comprising rigidiser arms 3340 (such as described elsewhere in this specification). The positioning and stabilising structure 3300 may also comprise one or more straps (not shown in Fig. 10B). The patient interface 3000 of Fig. 10A further comprises a cushion module 3150 comprising a frame 3270 which, together with cushion 3050 (not shown in Fig. 10B), forms plenum chamber 3200. An air circuit 4170 connects to an opening in an anterior wall of the plenum chamber 3200, for example through an opening in the frame 3270.

Marked in each of Figs. 10A and 10B is a line 3950. This line is indicative of an interfacing region of the patient interface 3000. Similar lines 3950 are marked in each of Figs. 9A to 9C and these mark interfacing regions of the assembly of cushion 3050 and brace 3900 of these figures. The interfacing regions of any of the assemblies shown in Figs. 9A to 9C may be interfaced with the interfacing regions of any of the patient interfaces 3000 of Figs. 10A and 10B to provide an assembled patient interface 3000 incorporating a brace 3900 of the type described earlier. Forms of the technology are not limited by the nature of the interface and any suitable manner of connection may be used. In some forms, the connection between the interfacing regions may be achieved by one or more clips. In other forms, the connection between the interfacing regions may be achieved by one or more magnetic members creating a magnetic connection.

In some forms, the cushion 3050 may connect directly to the frame 3270 and the brace 3900 may be connected only to the cushion 3050 and not the frame 3270. In other forms, the brace 3900 may be directly connected to both the cushion 3050 and the frame 3270, for example the brace 3900 may be sandwiched between the cushion 3050 and the frame 3270.

### 4.3.5 Positioning and stabilising structure

The seal-forming structure 3100 of the patient interface 3000 of the present technology may be held in sealing position in use by the positioning and stabilising structure 3300. The positioning and stabilising structure 3300 may comprise and function as "headgear" since it engages the patient's head in order to hold the patient interface 3000 in a sealing position.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

**In** one form of the present technology, a positioning and stabilising structure 3300 is provided that is configured in a manner consistent with being worn by a patient while sleeping. **In** one example the positioning and stabilising structure 3300 has a low profile, or cross-sectional thickness, to reduce the perceived or actual bulk of the apparatus. **In** one example, the positioning and stabilising structure 3300 comprises at least one strap having a rectangular cross-section. **In** one example the positioning and stabilising structure 3300 comprises at least one flat strap.

**In** one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another suitable for a small sized head, but not a large sized head.

Certain forms of a positioning and stabilising structure 3300 in accordance with aspects of the technology will be described below with particular reference to Figs. 3A, 4D, 5 to 10B, and 13A to 17B. In these forms, a positioning and stabilising structure 3300 provides a force to hold the plenum chamber 3200 and seal-forming structure 3100 in a therapeutically effective position on the patient's head in use, i.e. the positioning and stabilising structure 3300 may be configured to assist the seal-forming structure 3100 in contacting and forming a seal with the patient's face.

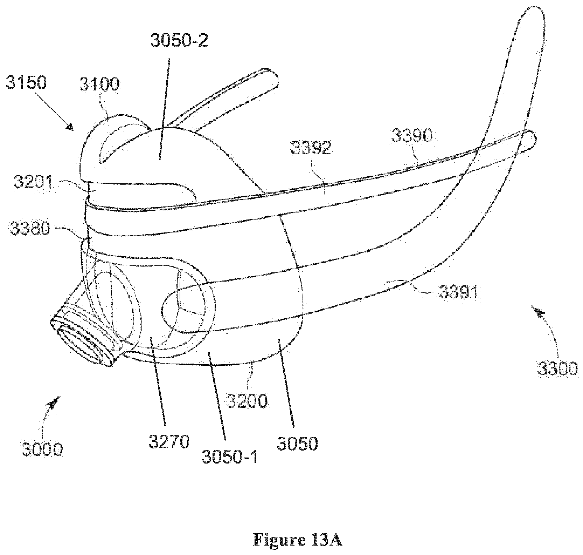

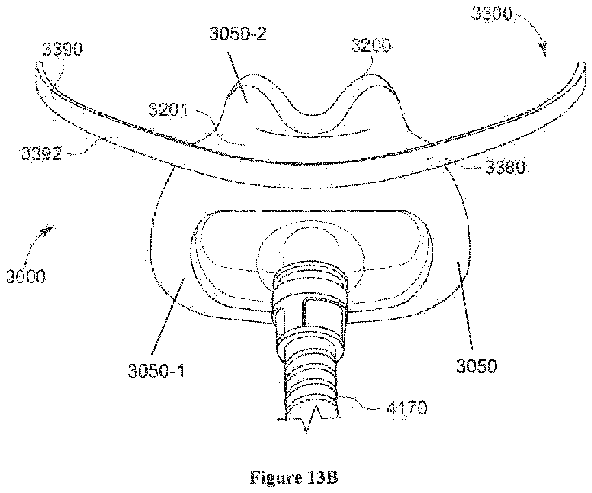

In the forms of the technology shown in Figs. 13A to 17B, the positioning and stabilising structure 3300 may comprise a superior portion 3370, an anterior engagement portion 3380, and two side portions 3390 connected between the superior portion 3370 and the anterior engagement portion 3380, as described below in more detail.

The superior portion 3370, anterior engagement portion 3380 and side portions 3390 may each be provided by one or more physical components. In some forms, one physical component may comprise one or more of the superior portion 3370, anterior engagement portion 3380 and/or side portions 3390.

In use the positioning and stabilising structure 3300 may be symmetrical about the sagittal plane of the patient 1000.

### 4.3.5.1 Headgear straps

In some forms such as those shown in Figs. 3A, 4D, 5 to 10B, and 13A to 17B, the positioning and stabilising structure 3300 may include headgear 3302 with at least one strap which may be worn by the patient in order to assist in properly orienting the seal-forming structure 3100 against the patient's face (e.g., in order to limit or prevent leaks).

As described above, some forms of the headgear 3302 may be constructed from a textile material, which may be comfortable against the patient's skin. The textile may be flexible in order to conform to a variety of facial contours. Although the textile may include rigidizers along a selected length, which may limit bending, flexing, and/or stretching of the headgear 3302.

In certain forms, the headgear 3302 may be at least partially extensible. For example, the headgear 3302 may include elastic, or a similar extensible material. For example, the entire headgear 3302 may be extensible or selected portions may be extensible (or more extensible than surrounding portions). This may allow the headgear 3302 to stretch while under tension, which may assist in providing a sealing force for the seal-forming structure 3100.

As mentioned above, in some forms, such as those shown in Figs. 13A to 17B, the positioning and stabilising structure 3300 may comprise a superior portion 3370, an anterior engagement portion 3380, and two side portions 3390. The superior portion 3370, an anterior engagement portion 3380, and side portions 3390 may form headgear 3302 and may include one or more straps as described above.

### 4.3.5.1.1 Four-point connection

In some forms of the technology, the headgear may be a four-point connection headgear. This means that the headgear may connect to four separate places, and therefore may include four different straps providing a tensile force to help maintain the seal-forming structure 3100 in a sealing position. The positioning and stabilising structure 3300 of Fig. 3A may be considered a four-point connection headgear.

Another four-point headgear arrangement is described below in section 4.3.5.5.1.

### 4.3.5.1.2 Two-point connection

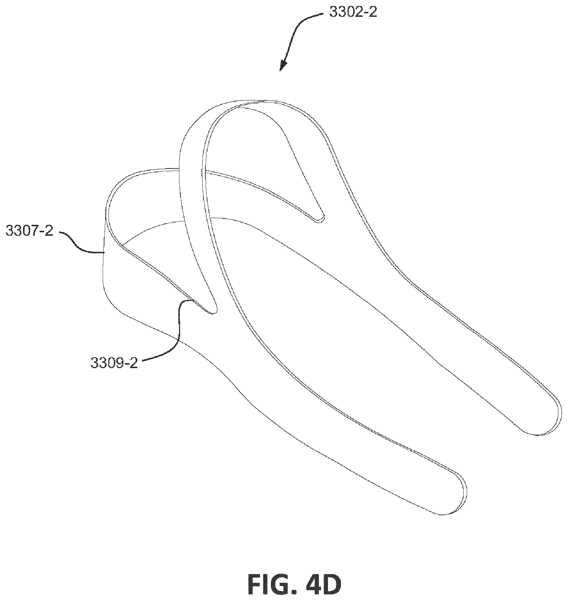

As shown in Fig. 4D, some forms of the headgear 3302-2 may be a two-point connection headgear. This means that the headgear 3302-2 may connect to two separate places, for example two separate connection points on a cushion module 3150.

In some forms, the headgear 3302-2 may be formed from a continuous piece of material. In other words, the headgear 3302-2 may not be formed from multiple straps connected (e.g., stitched) together. This may be comfortable for a patient as they will not be in contact with any seams or joints connecting different straps. In other forms, the headgear 3302-2 may be formed from multiple straps (e.g., two superior straps, a rear strap, etc.) that are connected together (e.g., with stitching, ultra-sonic welding, etc.).

As shown in Fig. 4D, some forms of the headgear 3302-2 may be at least partially bifurcated. For example, a rear strap 3307-2 of the headgear 3302-2 (e.g., configured to contact the posterior portion of the patient's head) may be wider than the surrounding portions of the headgear 3302-2. An intermediate section of the rear strap 3307-2 may include a slit 3309-2. A superior section of the rear strap 3307-2 may therefore be movable relative to the inferior section as a result of the slit 3309-2. This may allow the patient to have a larger strap coverage on the posterior region of their head, which may assist in better anchoring the headgear 3302-2 to the patient's head since there is no inferior strap.

In some forms, a two-point connection headgear may be suitable for use with the cushions 3050 of Figs. 5 to 10B. In these forms, the brace 3900 may enable a seal of sufficient quality and/or stability to be formed with the patient's face that the additional stability typically offered by four-point connection headgear may not be required, and a two-point connection headgear will suffice. This may be particularly advantageous in the case of the nose and mouth masks shown in Figs 5 to 8D, since this type of mask is commonly used with a greater number of headgear connection points, e.g. four-point connection.

In some forms, two-point connection headgear may be favoured by a patient over a four-point connection headgear because it may cause less discomfort when worn, cause less facial marking and reduce the feeling of claustrophobia, unease and/or obtrusiveness that is sometimes reported by patients when wearing headgear.

### 4.3.5.2 Rigidiser Arm

In certain forms, the positioning and stabilising structure 3300 may comprise one or more rigidiser arms 3340. The rigidiser arms 3340 may be provided to a headgear strap and/or a headgear tube 3350. For example, the rigidiser arms 3340 may be attached to a side of the headgear strap and/or headgear tube 3350, and may extend along a portion of their length. Alternatively, the rigidiser arms 3340 may be provided between the plenum chamber 3200 (which may be provided as part of a cushion module 3150) and the headgear straps.

As shown in Figs. 4C and 10B, a rigidiser arm 3340 may be an elongated, rigid member that assists in maintaining the cushion 3050 in an operative position. The rigidiser arm 3340 may contact a side of the patient's head and provide a force to limit slipping of the seal-forming structure 3100 from the patient's nose and/or mouth.

In some forms, the rigidiser arm 3340 is constructed from a rigid material (e.g., plastic). The rigid material may not permit the rigidiser arm 3340 to stretch. Additionally, the rigidiser arm 3340 may be substantially inflexible and may be unable to bend. The rigidiser arm 3340 may be pre-molded into a desired shape in order to fit a patient's head. For example, the rigidiser arms 3340 may be molded with a curved shape to substantially correspond to the shape of the side of the patient's head (e.g., overlaying the masseter muscle and/or the temporal bone).

In certain forms, the rigidiser arm 3340 may be molded in order to conform to a specific patient's head (e.g., the rigidiser arm 3340 is customized).

In some forms, the rigidiser arm 3340 may be flexible along at least one direction. For example, the rigidiser arm 3340 may be flexible about its width and may be inflexible along its length. In other words, the rigidiser arm 3340 may be bendable about an axis along the width of the rigidiser arm 3340, but may be unable to bend about an axis perpendicular to the rigidiser arm 3340. This may allow an individual patient to adjust the rigidiser arm 3340 in order to better fit their individual head.

In certain forms, the rigidiser arm 3340 may remain in the new position after being bent. This may allow a patient adjust the shape of the rigidiser arm 3340 for their specific head and then the rigidiser arm 3340 will keep the desired shape while in use in order to promote patient comfort.

In some forms, a first end 3342 of the rigidiser arm 3340 may be a free end and a second end 3344 (e.g., opposite of the first end 3342) of the rigidiser arm 3340 may be fixed. The first end 3342 may be curved in order to minimize sharp edges that could cause patient discomfort. The first end 3342 may also overlay the patient's head proximate to the temporal bone, in use. The second end 3344 may be fixed to an arm connection structure 3504. The arm connection structure 3504 may be configured to connect to the plenum chamber 3200, which may be provided as part of a cushion module 3150.

### 4.3.5.3 Superior portion

As in the forms of the technology shown in Figs. 13A to 17B, the positioning and stabilising structure 3300 comprises a superior portion 3370 configured to overlie a region of the patient's head superior to an otobasion superior of the patient's head in use.

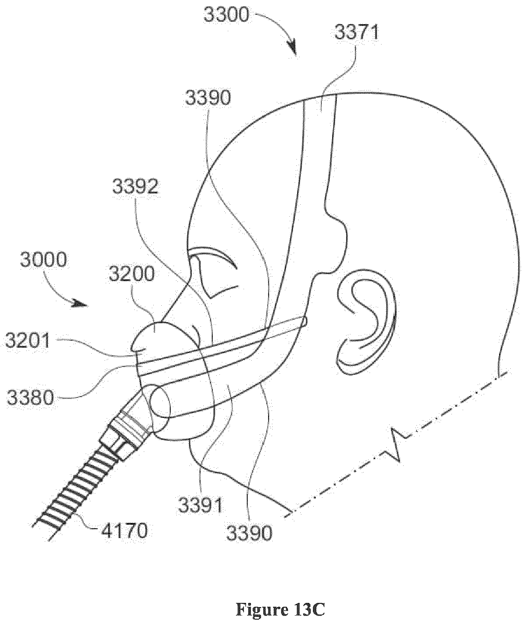

The superior portion 3370 may comprise a crown portion 3371 that overlies the crown of the patient's head in use, as shown in Figs. 13C, 14C, 15A, 15B, 16A, 17A, and 17B. The crown portion 3371 may overlie a portion of the patient's parietal bone in use. Alternatively, or additionally, the crown portion 3371 may overlie a portion of the patient's frontal bone proximate the top of the head in use.

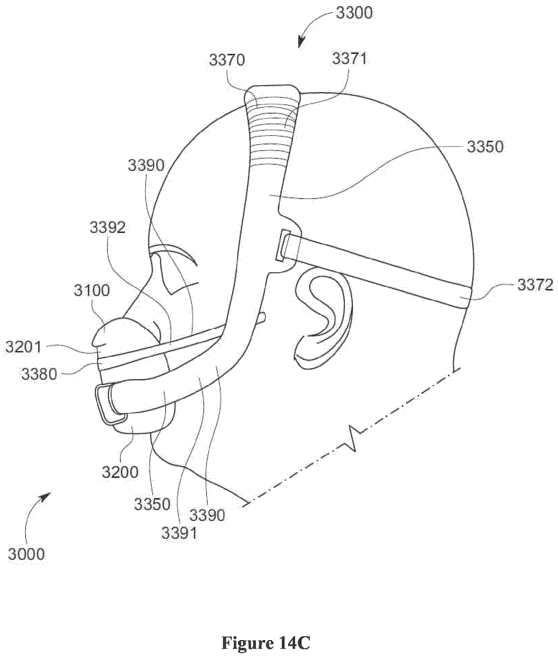

The superior portion 3370 may additionally or alternatively comprise a rear portion 3372 that overlies the rear of the patient's head in use, as shown in Figs. 14C, 15A, 15B, 16A, 17A, and 17B. The rear portion 3372 may overlay, or lie inferior to, the patient's occipital bone in use.

In some forms of the technology, such as those shown in Figs. 14C, 15A, 15B, 16A, 17A, and 17B, the superior portion 3370 comprises both a crown portion 3371 and a rear portion 3372. In other forms of the technology, the superior portion 3370 may comprise another configuration and/or combination of portions. In some forms, the positioning and stabilising structure includes a tie that is constructed and arranged to interconnect the crown portion and the rear portion to reduce a tendency of the crown portion and the rear portion to move apart from one another.

In some forms, for example as shown in Figs. 13C, 15A to 16A and 17A to 17B, the superior portion 3370 comprises one or more straps. In some forms, for example as shown in Fig. 14C, the superior portion 3370 comprises one or more headgear tubes 3350 that deliver pressurised air received from a conduit forming part of the air circuit 4170 from the RPT device to the patient's airways, for example through the plenum chamber 3200 and seal-forming structure 3100. The superior portion 3370 may comprise a connection port 3600 that connects to the air circuit 4170 to receive the flow of air into the headgear tubes 3350. The connection port 3600 may be positioned on top of the patient's head in use. The headgear tubes comprised as part of the superior portion 3370 may be joined to, for example may be integrally connected with, headgear tubes comprised as part of the side portions 3390. Forms of the technology in which the positioning and stabilising structure 3300 comprises headgear tubes 3350 (which may be referred to as "conduit headgear") are described in more detail below.

### 4.3.5.4 Anterior engagement portion

As in the forms of the technology shown in Figs. 13A to 17B, the positioning and stabilising 3300 structure further comprises an anterior engagement portion 3380 configured to overlie an anterior portion 3201 of the plenum chamber 3200 in use. The anterior engagement portion 3380 may engage with and provide a force to the plenum chamber 3200 in use to hold the plenum chamber 3200 and seal-forming structure 3100 in a therapeutically effective position on the patient's head.

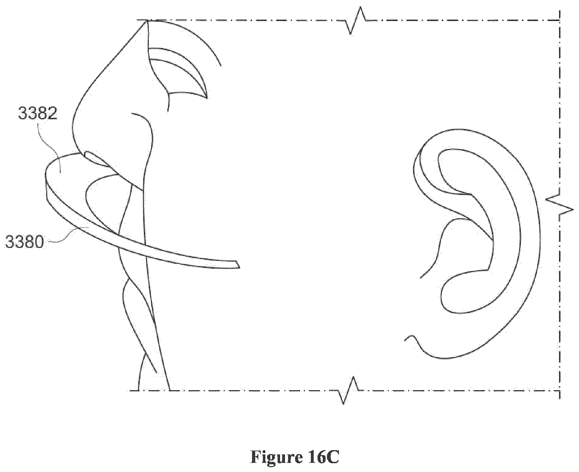

In some forms the anterior engagement portion 3380 may be formed such that it has a non-planar cross-sectional shape and is not substantially flat or planar as in the form of a strap, as shown in the figures, in particular Fig. 16C. For example, the anterior engagement portion 3380 may be a moulded component. In other forms, the anterior engagement portion 3380 may comprise a strap and one or more members provided to the strap, for example wedge-shaped members. More particularly, in some forms the anterior engagement portion comprises a central portion of the strap and the two side portions 3390 may each comprise end portions of the strap. A wedge may be mounted on a patient-facing side of the central portion of the strap. The wedge may comprise a patient-facing surface 3381 of the anterior engagement portion 3380, as described below. The wedge may be formed from a silicone, rubber, polycarbonate, or foam.

In the illustrated forms, the anterior engagement portion 3380 is not a component that is able to convey a flow of breathable gas, i.e. the anterior engagement portion 3380 is not comprised as part of headgear tubes 3350. More generally, the anterior engagement portion 3380 may not be in contact with air being delivered to the patient for inhaling or in contact with air exhaled by the patient. For example, the anterior engagement portion 3380 may be located entirely outside the plenum chamber 3200 in use.

In some forms, the anterior engagement portion 3380 may be customized to the patient. The shape of the anterior engagement portion 3380 may alter how the anterior engagement portion 3380 engages with and exerts a force on the plenum chamber 3200 in use to hold the plenum chamber 3200 and seal-forming structure 3100 in a therapeutically effective position on the patient's head. In some forms, the anterior engagement portion 3380 may be 3D printed with an optimised shape, i.e. with a specific cross-sectional shape and/or curvature, to assist with providing a good fit and seal with a particular patient's face, including their nose shape.

### 4.3.5.4.1 Direction of contact force between the headgear and the plenum chamber

As mentioned above the anterior engagement portion 3380 of the positioning and stabilising structure 3300 may be configured to engage with the plenum chamber 3200.

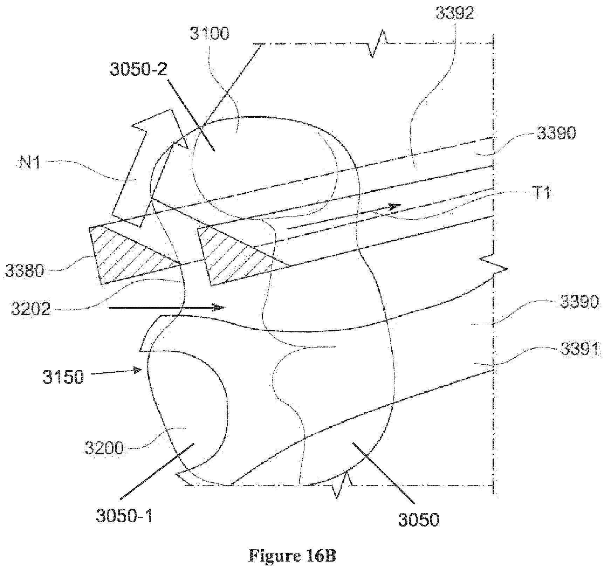

As, shown in Fig. 16B, the anterior engagement portion 3380 may be configured such that when, in use, a patient-facing surface 3381 of the anterior engagement portion 3380 contacts a non-patient-facing (e.g. anterior) surface 3202 of the plenum chamber 3200, a normal component N1 of the contact force between the patient-facing surface 3381 of the anterior engagement portion 3380 and the non-patient-facing surface 3202 of the plenum chamber 3200 may be in a direction superior to the direction of the tension force T1 provided by side portions 3390 on the anterior engagement portion 3380. In some forms, the angle between the normal component N1 of the contact force and a plane parallel to the Frankfort horizontal (shown in Fig 2E) may be greater than the angle between the tension force T1 and the same plane parallel to the Frankfort horizontal. In some forms, the direction of the normal component N1 of the contact force may be in a general direction towards the nares of the patient. In some forms, the direction of the tension force T1 is in a direction lengthwise along the side portions 3390. The configuration of the positioning and stabilising structure 3300, particularly the shape, position, and angle of the patient-facing surface 3381, has the effect of altering the direction of the force applied to the plenum chamber 3200 by the positioning and stabilising structure 3300 in such a way that assists the seal-forming structure with forming a seal with the patient's face, particularly against the underside of the patient's nose. The shape, position, and angle of the patient-facing surface 3381 allows the direction of the force of the positioning and stabilising structure 3300 on the plenum chamber 3200 to be different to the direction of the tension force T1 in the straps.

In some forms, as shown in Figs. 16B to 16E, the patient-facing surface 3381 of the anterior engagement portion 3380 comprises a surface portion 3382 oriented such that a normal direction to the superior-facing surface portion 3382 is superior to the direction of the tension force. The surface portion 3382 may be substantially smooth. As shown in Fig. 16C, the surface portion 3382 may also be curved along its length such that it generally corresponds to the shape of the anterior portion 3201 of the plenum chamber 3200 in the lateral direction (i.e. across the face).

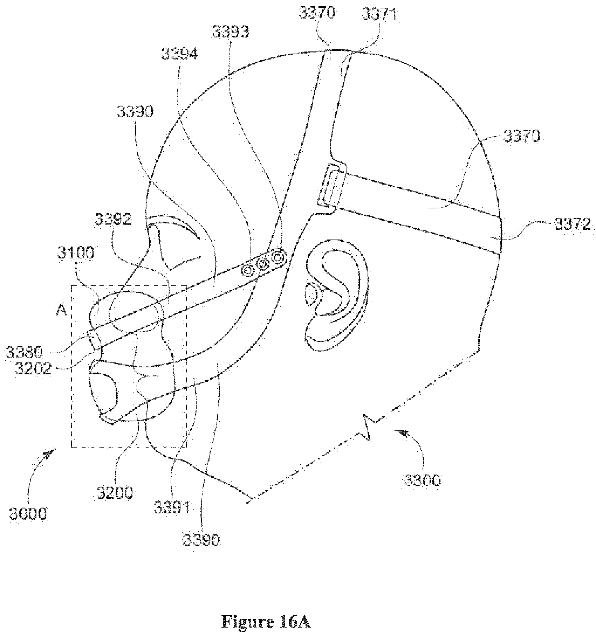

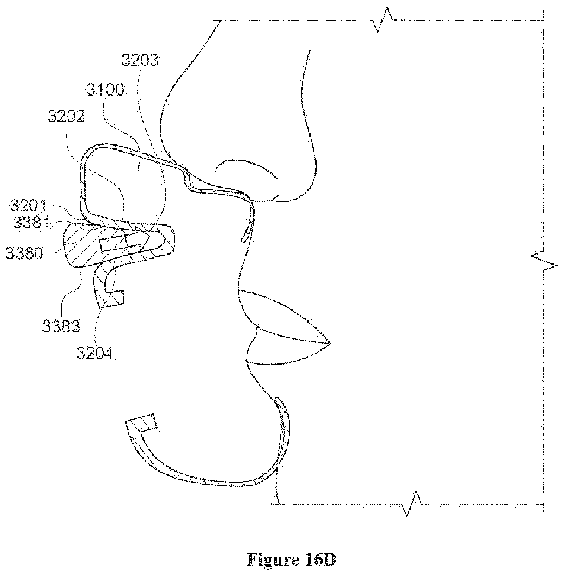

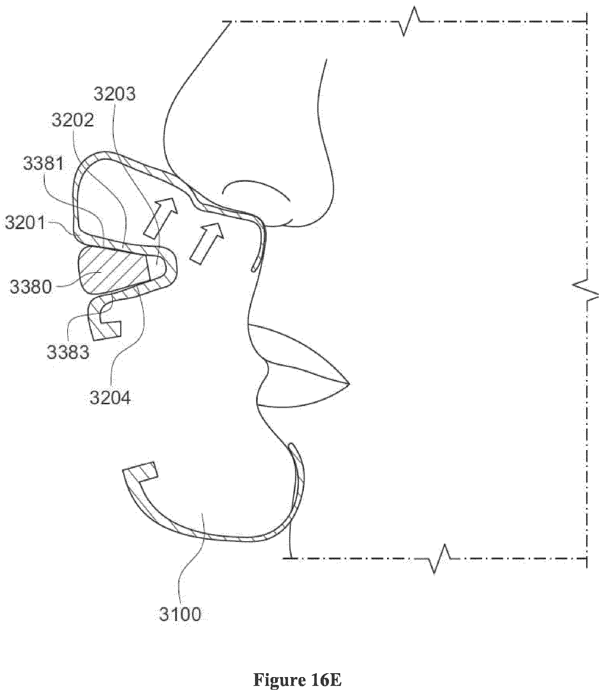

In some forms, as shown in Figs. 16D and 16E, a cross-section of the anterior engagement portion 3380 in the mid-sagittal plane when in use is non-rectangular and non-square in shape. In some forms, a cross-section of the anterior engagement portion 3380 in the mid-sagittal plane when in use is substantially wedge-shaped, as shown in Figs. 16D and 16E. The cross-sectional shape of the anterior engagement portion 3380 may taper from a wider side furthest from the patient to a narrower side closer to the patient when in use. In some forms the cross-sectional shape of the anterior engagement portion 3380 is generally trapezoidal or triangular. In some forms, as shown in Figs. 16D and 16E, the cross-sectional shape has four sides, two opposite sides being substantially parallel with one further from the patient being longer than that closer to the patient, the other two opposite sides being generally sloped from the longer side to the shorter side. In some forms the cross-sectional shape has rounded corners, i.e. the edges of the anterior engagement portion 3380 are rounded and/or smooth. In some forms, the cross-sectional shape of the anterior engagement portion 3380 may vary along the length of the anterior engagement portion 3380 such that the patient-facing surface of the anterior engagement portion 3380 has contours corresponding with the contours of the anterior portion 3201 of the plenum chamber 3200. In some forms, the taper or wedge-shape of the cross-section of the anterior engagement portion 3380 may vary along the length of the anterior engagement portion 3380.

### 4.3.5.4.2 Plenum chamber groove

In some forms, such as that shown in Figs. 16D and 16E, the plenum chamber 3200 comprises a groove 3203 or recess in an anterior surface 3204 of the plenum chamber 3200, wherein the groove 3203 comprises the non-patient-facing surface 3202 contacted in use by the patient-facing surface 3381 of the anterior engagement portion 3380 of the positioning and stabilising structure 3300. The groove 3203 may be sized to allow a substantial part of the wedge to be received within the groove 3203. The groove 3203 when viewed in the mid-sagittal plane may be substantially V-shaped, as shown in Figs. 16D and 16E, or in other forms may be substantially U-shaped. The groove 3203 may be oriented such that the open-end of the groove 3203 is facing away from the patient 1000. The length of the groove 3203 may be substantially parallel with the Frankfort horizontal when in use.

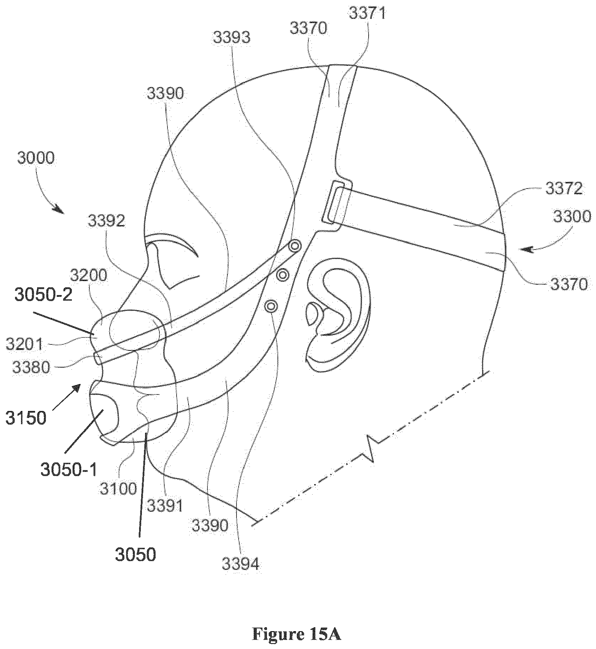

In some forms, the groove 3203 is located in a part of the plenum chamber 3200 positioned, in use, inferior to a part of the seal-forming structure 3100 in sealing contact with a region of the patient's face surrounding the patient's nasal airways. In some forms, for instance when the patient interface 3000 comprises a seal-forming structure 3100 configured to seal around an entrance to the patient's nasal airways and also around the patient's mouth as in Figs 15A to 17B, the groove 3203 is also located in a part of the plenum chamber 3200 positioned, in use, superior to a part of the seal-forming structure 3100 in sealing contact with a region of the patient's face surrounding the patient's mouth.

In some forms, the plenum chamber 3200, or a portion thereof, is flexible and a portion of the plenum chamber 3200 superior to the groove 3203 is able to move relative to a portion of the plenum chamber 3200 inferior to the groove 3203. The movement of the plenum chamber 3200 may provide a force on the seal-forming structure 3100 such that it forms a better seal with the patient's face surrounding the patient's nasal airways. The movement of the plenum chamber 3200 may be caused by the force of the anterior engagement portion 3380 acting on the surfaces of the plenum chamber 3200 within the groove 3203. The anterior engagement portion 3380 may be positioned within the groove 3203 closer to, such as shown in Fig. 16E, or further from, such as shown in Fig. 16D, the patient 1000 by altering the tension force provided by the side portions 3390 on the anterior engagement portion 3380. Moving the anterior engagement portion 3380 further into the groove 3203 causes the opening of the groove 3203 to widen, i.e. the groove 3203 "splays out". This provides a greater force on the seal-forming structure 3100 such that it moves towards the patient 1000, enhancing the seal against the patient's face.

In some forms, the plenum chamber 3200, or a portion thereof, is flexible and a portion of the plenum chamber 3200 inferior to the groove 3203 is able to move relative to a portion of the plenum chamber 3200 superior to the groove 3203. The movement of the plenum chamber 3200 may provide a force on the seal-forming structure 3100 such that it forms a better seal with the patient's face surrounding the patient's mouth. The anterior engagement portion 3380 may be configured such that when, in use, a second patient-facing surface 3383 of the anterior engagement portion 3380 contacts a second non-patient-facing surface 3204 of the plenum chamber 3200, a normal component of the contact force between the second patient-facing surface 3381 of the anterior engagement portion 3380 and the second non-patient-facing surface 3202 of the plenum chamber 3200 may be in a direction inferior to the direction of the tension force provided by side portions 3390 on the anterior engagement portion 3380. In some forms, the angle between the normal component of the contact force and a plane parallel to the Frankfort horizontal (shown in Fig 2E) may be greater than the angle between the tension force and the same plane parallel to the Frankfort horizontal. In some forms, the direction of the normal component of the contact force may be in a general direction towards the mouth of the patient.

In some forms, the material and thickness of the non-patient-facing surface 3202 and/or the second non-patient-facing surface 3204 may be different to the other parts of the plenum chamber 3200. In some forms, the material of the non-patient-facing surface 3202 and/or the second non-patient-facing surface 3204 are harder than the other parts of the plenum chamber 3200. The non-patient-facing surface 3202 and/or the second non-patient-facing surface 3204 may be formed from a polycarbonate material. In other forms the non-patient-facing surface 3202 and/or the second non-patient-facing surface 3204 may be formed from a silicone of different hardness to the other parts of the plenum chamber 3200.

### 4.3.5.5 Side portions

The positioning and stabilising 3300 structure may further comprise two side portions 3390. Each of the side portions may be connected between the superior portion 3370 and the anterior engagement portion 3380. The side portions 3390 may be configured to overlie respective opposite cheek regions of the patient's head in use.

In use the side portions 3390 provide a tension force to the anterior engagement portion 3380. Furthermore, the superior portion 3370 may provide a tension force on the side portions 3390. In use, the forces acting on and the position of the superior, anterior engagement and side portions on the patient's head may result in an equilibrium with the plenum chamber 3200 and seal-forming structure 3100 being maintained in a therapeutically effective position on the patient's head.

Each side portion 3390 may comprise one portion or an assembly of portions. The portions comprised as part of each side portion 3390 may include straps, rigidiser arms, headgear tubes or a combination of these, as described below. The portions comprised as part of each side portion 3390 may be integrally formed with parts of the anterior engagement portion 3380 and/or superior portion 3370.

In one, non-illustrated form of the technology each side portion comprises only a single portion or strap connected between the superior portion 3370 and the anterior engagement portion 3380. In such forms the only portion of the positioning and stabilising structure 3300 that contacts the plenum chamber 3200 in use is the anterior engagement portion 3380 which may also be formed by a single portion or strap.

### 4.3.5.5.1 Four-point headgear

In some forms of the technology, such as those shown in Figs. 13A to 17B, the positioning and stabilising structure 3300 may engage the plenum chamber 3200 at multiple points, for example a "four-point" headgear arrangement. In such a form, the positioning and stabilising structure 3300 may engage a superior part of the plenum chamber and an inferior part of the plenum chamber on both a left and right side.

Each of the side portions 3390 of the positioning and stabilising structure 3300 may comprise a first side portion 3391 and a second side portion 3392. In certain forms, a first side portion 3391 may be connected between the superior portion 3370 and the plenum chamber 3200, and a second side portion 3392 may extend to the anterior engagement portion 3380, as shown in Figs. 14C, 15A, 15B, 16A, 17A, and 17B.

The first side portion 3391 may connect to a region of the plenum chamber 3200 that is inferior in use to the anterior portion 3201 of the plenum chamber 3200 overlaid by the anterior engagement portion 3380.

The first side portions 3391 may be configured to provide a tension force on an inferior part of the plenum chamber to urge an inferior part of the seal-forming structure into sealing contact with a region of the patient's face surrounding the patient's mouth in use.

The second side portions 3392 may be configured to provide a tension force on a superior part of the plenum chamber to urge a superior part of the seal-forming structure into sealing contact with a region of the patient's face surrounding the patient's nasal airways in use.

In some forms, an end of each of the second side portions 3392 is connected to a part of the respective first side portion 3391 at a connection 3393. Each of the connections 3393 between the end of each of the second side portion 3392 and the respective first side portion 3391 may be located to overlie a region of the patient's head adjacently anterior to one of the patient's ears in use. In some forms, the connections 3393 are located over a cheek region of the patient in use.

In some forms, the second side portions 3392 each comprise end portions of the strap as described above. In some forms, the first side portions 3391 each comprise a strap or rigidiser arm. Rigidiser arms are rigid or semi-rigid components, or assemblies of components. The rigidiser arms may be formed from polycarbonate and/or nylon in some forms. Rigidiser arms may be shaped such that when the patient interface 3000 and the positioning and stabilising structure 3300 are donned by the patient 1000 the rigidiser arms do not pass over the eyes. The rigidiser arms may also be shaped to avoid the ears and the temples. For example, each rigidiser arm may be shaped to pass along and conform to the patient's cheek and extend between the eye and ear while avoiding the temple such that the rigidiser arm connects to the superior portion 3370 at an attachment point above the patient's ear. The rigidiser arms may be relatively wider along their width than the dimension perpendicular to the patient's skin in use. By forming the rigidiser arms in this way may allow for targeted flexing of the rigidiser arms. In other words, flexing is resisted more greatly in the direction of the wider cross-section while it is resisted less in the direction of the narrower cross-section. This may be advantageous in that the rigidiser arms may be allowed to flex toward the patient's face and cheeks in conformity therewith when placed in tension by the positioning and stabilising structure 3300, while flexing upward into the patient's eyes or downward toward the patient's ears is resisted.

In some forms, such as that shown in Fig. 14C, the first side portions 3391 each comprise a tube 3350 configured to deliver pressurised air from the superior portion to the plenum chamber 3200 for delivery to the patient's airways, and wherein the superior portion 3370 comprises a connection port 3600 for receiving a supply of pressurised air and at least one tube 3350 for delivering the supply of pressurised air to the tubes 3350 of the second side portions 3392. In some forms, the first side portions 3391 and the superior portion 3380 form part of a conduit headgear as described below.

### 4.3.5.5.1.1 Conduit headgear

### 4.3.5.5.1.1.1 Conduit headgear tubes

In some forms of the present technology, the positioning and stabilising structure 3300 comprises one or more headgear tubes 3350 that deliver pressurised air received from a conduit forming part of the air circuit 4170 from the RPT device to the patient's airways, for example through the plenum chamber 3200 and seal-forming structure 3100. In the forms of the present technology illustrated in Figs. 3I, 10A, and 14C, the positioning and stabilising structure 3300 comprises two tubes 3350 that deliver air to the plenum chamber 3200 from the air circuit 4170. The tubes 3350 are configured to position and stabilise the seal-forming structure 3100 of the patient interface 3000 at the appropriate part of the patient's face (for example, the nose and/or mouth) in use. This allows the conduit of air circuit 4170 providing the flow of pressurised air to connect to a connection port 3600 of the patient interface in a position other than in front of the patient's face, for example on top of the patient's head.

In the forms of the present technology illustrated in Figs. 3I, 10A and 14C, the positioning and stabilising structure 3300 comprises two tubes 3350, each tube 3350 being positioned in use on a different side of the patient's head and extending across the respective cheek region, above the respective ear (superior to the otobasion superior on the patient's head) to the elbow 3610 on top of the head of the patient 1000. This form of technology may be advantageous because, if a patient sleeps with their head on its side and one of the tubes 3350 is compressed to block or partially block the flow of gas along the tube 3350, the other tube 3350 remains open to supply pressurised gas to the patient. In other examples of the technology, the patient interface 3000 may comprise a different number of tubes, for example one tube, or two or more tubes. In one example in which the patient interface has one tube 3350, the single tube 3350 is positioned on one side of the patient's head in use (e.g. across one cheek region) and a strap forms part of the positioning and stabilising structure 3300 and is positioned on the other side of the patient's head in use (e.g. across the other region) to assist in securing the patient interface 3000 on the patient's head.

In the forms of the technology shown in Figs. 3I, 10A and 14C the two tubes 3350 are fluidly connected at superior ends to each other and to the connection port 3600. In some examples, the two tubes 3350 are integrally formed while in other examples the tubes 3350 are formed separately but are connected in use and may be disconnected, for example for cleaning or storage. Where separate tubes are used they may be indirectly connected together, for example each may be connected to a T-shaped connector having two arms/branches each fluidly connectable to a respective one of the tubes 3350 and a third arm or opening in the T-shaped connector providing the connection port 3600 for fluid connection to the air circuit 4170 in use.

The tubes 3350 may be formed from a flexible material, such as an elastomer, e.g. silicone or TPE, and/or from one or more textile and/or foam materials. The tubes 3350 may have a preformed shape and may be able to be bent or moved into another shape upon application of a force but may return to the original preformed shape in the absence of said force. The tubes 3350 may be generally arcuate or curved in a shape approximating the contours of a patient's head between the top of the head and the nasal or oral region.

In some examples, the one or more tubes 3350 are crush resistant to resist being blocked if crushed during use, for example if squashed between a patient's head and pillow, especially if there is only one tube 3350. The tubes 3350 may be formed with a sufficient structural stiffness to resist crushing or may be as described in US Patent no. 6044844.

Each tube 3350 may be configured to receive a flow of air from the connection port 3600 on top of the patient's head and to deliver the flow of air to the seal-forming structure 3100 at the entrance of the patient's airways. In the examples shown in Figs. 3I, 10A and 14C, each tube 3350 lies in use on a path extending from the plenum chamber 3200 across the patient's cheek region and superior to the patient's ear to the elbow 3610. For example, a portion of each tube 3350 proximate the plenum chamber 3200 may overlie a maxilla region of the patient's head in use. Another portion of each tube 3350 may overlie a region of the patient's head superior to an otobasion superior of the patient's head. Each of the tubes 3350 may also lie over the patient's sphenoid bone and/or temporal bone and either or both of the patient's frontal bone and parietal bone. The elbow 3610 may be located in use over the patient's parietal bone, over the frontal bone and/or over the junction therebetween (e.g. the coronal suture).

In certain forms of the present technology the patient interface 3000 is configured such that the connection port 3600 can be positioned in a range of positions across the top of the patient's head so that the patient interface 3000 can be positioned as appropriate for the comfort or fit of an individual patient. In some examples, the headgear tubes 3350 are configured to allow movement of an upper portion of the patient interface 3000 (e.g. a connection port 3600) with respect to a lower portion of the patient interface 3000 (e.g. a plenum chamber 3200). That is, the connection port 3600 may be at least partially decoupled from the plenum chamber 3200. In this way, the seal-forming structure 3100 may form an effective seal with the patient's face irrespective of the position of the connection port 3600 (at least within a predetermined range of positions) on the patient's head.

As described above, in some examples of the present technology the patient interface 3000 comprises a seal-forming structure 3100 in the form of a cradle cushion which lies generally under the nose and seals to an inferior periphery of the nose (e.g. an under-the-nose cushion). The positioning and stabilising structure 3300, including the tubes 3350 may be structured and arranged to pull the seal-forming structure 3100 into the patient's face under the nose with a sealing force vector in a posterior and superior direction (e.g. a posterosuperior direction). A sealing force vector with a posterosuperior direction may cause the seal-forming structure 3100 to form a good seal to both the inferior periphery of the patient's nose and anterior-facing surfaces of the patient's face, for example on either side of the patient's nose and the patient's lip superior.

### 4.3.5.5.1.1.2 Extendable and non-extendable tube portions

In some examples of the present technology, one or both of the tubes 3350 are not extendable in length. However, in some forms, the tubes 3350 may comprise one or more extendable tube sections, for example formed by an extendable concertina structure. In some forms, the patient interface 3000 may comprise a positioning and stabilising structure 3300 including at least one gas delivery tube comprising a tube wall having an extendable concertina structure. The patient interface 3000 shown in Figs. 3I and 14C comprises tubes 3350, the superior portions of which comprise extendable tube sections each in the form of an extendable concertina structure 3362.

The cross-sectional shape of the non-extendable tube sections 3363 of the tubes 3350 may be circular, elliptical, oval, D-shaped or a rounded rectangle, for example as described in US Patent No. 6,044,844. A cross-sectional shape that presents a flattened surface of tube on the side that faces and contacts the patient's face or other part of the head may be more comfortable to wear than, for example a tube with a circular cross-section.

In some examples of the present technology, the non-extendable tube sections 3363 connects to the plenum chamber 3200 from a low angle. The headgear tubes 3350 may extend inferiorly down the sides of the patient's head and then curve anteriorly and medially to connect to the plenum chamber 3200 in front of the patient's face. The tubes 3350, before connecting to the plenum chamber 3200, may extend to a location at the same vertical position as (or, in some examples, inferior to) the connection with the plenum chamber 3200. That is, the tubes 3350 may project in an at least partially superior direction before connecting with the plenum chamber 3200. A portion of the tubes 3350 may be located inferior to the cushion module 3150 and/or the seal forming structure 3100. The tubes 3350 may contact the patient's face below the patient's cheekbones, which may be more comfortable than contact on the patient's cheekbones and may avoid excessively obscuring the patient's peripheral vision.

### 4.3.5.5.1.1.3 Conduit headgear connection port

In certain forms of the present technology, the patient interface 3000 may comprise a connection port 3600 located proximal to a superior, lateral or posterior portion of a patient's head. For example, in the forms of the present technology illustrated in Figs. 3I and 14C, the connection port 3600 is located on top of the patient's head (e.g. at a superior location with respect to the patient's head). In this example the patient interface 3000 comprises an elbow 3610 forming the connection port 3600. The elbow 3610 may be configured to fluidly connect with a conduit of an air circuit 4170. The elbow 3610 may be configured to swivel with respect to the positioning and stabilising structure 3300 to at least partially decouple the conduit from the positioning and stabilising structure 3300. In some examples the elbow 3610 may be configured to swivel by rotation about a substantially vertical axis and, in some particular examples, by rotation about two or more axes. In some examples the elbow may comprise or be connected to the tubes 3350 by a ball-and-socket j oint. The connection portion 3600 may be located in the sagittal plane of the patient's head in use.

Patient interfaces having a connection port that is not positioned anterior to the patient's face may be advantageous as some patients may find a conduit that connects to a patient interface anterior to their face to be unsightly and/or obtrusive. For example, a conduit connecting to a patient interface anterior to the patient's face may be prone to interference with bedclothes or bed linen, particularly if the conduit extends inferiorly from the patient interface in use. Forms of the present technology comprising a patient interface having a connection port positioned superiorly to the patient's head in use may make it easier or more comfortable for a patient to lie or sleep in one or more of the following positions: a side-sleeping position, a supine position (e.g. on their back, facing generally upwards) or in a prone position (e.g. on their front, facing generally downwards). Moreover, connecting a conduit to an anterior portion of a patient interface may exacerbate a problem known as tube drag in which the conduit exerts an undesired force upon the patient interface during movement of the patient's head or the conduit, thereby causing dislodgement away from the face. Tube drag may be less of a problem when force is received at a superior location of the patient's head than anterior to the patient's face proximate to the seal-forming structure (where tube drag forces may be more likely to disrupt the seal).

### 4.3.5.5.1.1.4 Headgear Tube Fluid Connections

The two tubes 3350 are fluidly connected at their inferior ends to the plenum chamber 3200. In certain forms of the technology, the connection between the tubes 3350 and the plenum chamber 3200 is achieved by connection of two rigid connectors. The tubes 3350 and plenum chamber 3200 may be configured to enable the patient to easily connect the two components together in a reliable manner. The tubes 3350 and plenum chamber 3200 may be configured to provide tactile and/or audible feedback in the form of a 're-assuring click' or a similar sound, so that the patient may easily know that each tube 3350 has been correctly connected to the plenum chamber 3200. In one form, the tubes 3350 are formed from a silicone or textile material and the inferior end of each of the silicone tubes 3350 is overmolded to a rigid connector made, for example, from polypropylene, polycarbonate, nylon or the like. The rigid connector on each tube 3350 may comprise a female mating feature configured to connect with a male mating feature on the plenum chamber 3200. Alternatively, the rigid connector on each tube 3350 may comprise a male mating feature configured to connect to a female mating feature on the plenum chamber 3200. In other examples the tubes 3350 may each comprise a male or female connector formed from a flexible material, such as silicone or TPE, for example the same material from which the tubes 3350 are formed.

In other examples a compression seal is used to connect each tube 3350 to the plenum chamber 3200. For example, a resiliently flexible (e.g. silicone) tube 3350 without a rigid connector may be configured to be squeezed to reduce its diameter so that it can be compressed into a port in the plenum chamber 3200 and the inherent resilience of the silicone pushes the tube 3350 outwards to seal the tube 3350 in the port in an air-tight manner. Alternatively, in a hard-to-hard type engagement between the tube 3350 and the plenum chamber 3200, each tube 3350 and/or plenum chamber 3200 may comprise a pressure activated seal, for example a peripheral sealing flange. When pressurised gas is supplied through the tubes 3350 the sealing flange may be urged against the join between the tubes and a circumferential surface around a port or connector of the plenum chamber 3200 to form or enhance a seal between the tube 3350 and plenum chamber 3200.

### 4.3.5.5.1.1.5 Conduit headgear straps

In certain forms of the present technology, the positioning and stabilising structure 3300 comprises at least one headgear portion or strap acting in addition to the tubes 3350 to position and stabilise the seal-forming structure 3100 at the entrance to the patient's airways. As shown in Figs. 3I and 14C, the patient interface 3000 comprises a strap 3310 forming part of the positioning and stabilising structure 3300. The strap 3310 may be known as a back strap or a rear headgear strap, for example. In other examples of the present technology, one or more further straps may be provided. For example, patient interfaces 3000 according to examples of the present technology having a full face cushion may have a second, lower, strap configured to lie against the patient's head proximate the patient's neck and/or against posterior surfaces of the patient's neck.

In the examples shown in Figs. 3I and 14C, strap 3310 of the positioning and stabilising structure 3300 is connected between the two tubes 3350 positioned on each side of the patient's head and passing around the back of the patient's head, for example overlying or lying inferior to the occipital bone of the patient's head in use. The strap 3310 connects to each tube above the patient's ears. With reference to Figs. 3I and 14C, the positioning and stabilising structure 3300 comprises a pair of tabs 3320. In use a strap 3310 may be connected between the tabs 3320. The strap 3310 may be sufficiently flexible to pass around the back of the patient's head and lie comfortably against the patient's head, even when under tension in use.

### 4.3.5.5.2 Headgear with adjustable portions

In some forms of the technology, altering the tension force provided by the side portions 3390 on the anterior engagement portion 3380 alters the contact force between the patient-facing surface 3381 and the non-patient-facing surface 3202. In some forms, increasing the tension force increases the contact force and decreasing the tension force decreases the contact force. In some forms of the technology, altering the direction of the tension force alters the angle of the contact force. The ability to alter the angle of the contact force may assist in achieving an adequate seal between the seal-forming structure 3100 and the patient's face.

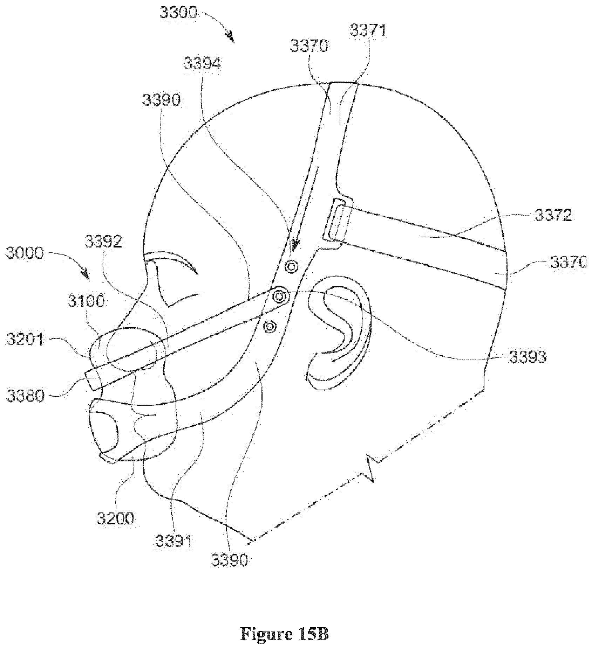

In some forms, altering the tension force may be achieved by shortening or lengthening the side portions 3390. In other forms, as shown in Figs. 15A, 15B, and 16A, altering the tension force may be achieved by altering the connection between the side portions 3390 and the superior portion 3380. In some forms there may be a plurality of possible positions that the connection between the side portions 3390 and the superior portion 3380 may be connected at. The patient or some other person may be able to choose which of the plurality of the positions to locate the connection.

In the forms of the technology described above comprising first 3391 and second 3392 side portions, the positioning and stabilising structure 3300 is configured so that the ends of each of the second side portions 3392 may be connected to the respective first side portions 3391 at a plurality of different positions to adjust the direction of the tension force on the anterior engagement portion. In some forms, such as those shown in Figs. 15A and 15B, the first side portions 3391 may comprise a plurality of connection points 3394 that can receive a connector mounted at or proximate to the end of the second side portion 3392. In other forms, such as that shown in Fig 16A, the end of the second side portion 3392 may comprise a plurality of connectors establishing a plurality of connection points 3394 that can connect to a position on the first side portion 3391. In other forms, both the end of the second side portion 3392 and the first side portion may comprise connectors that establish connection points 3394.

The connection points 3394 may be in the form of clips, buttons, hook and loop, or snap-fit engagements.

### 4.3.5.6 Textile cover

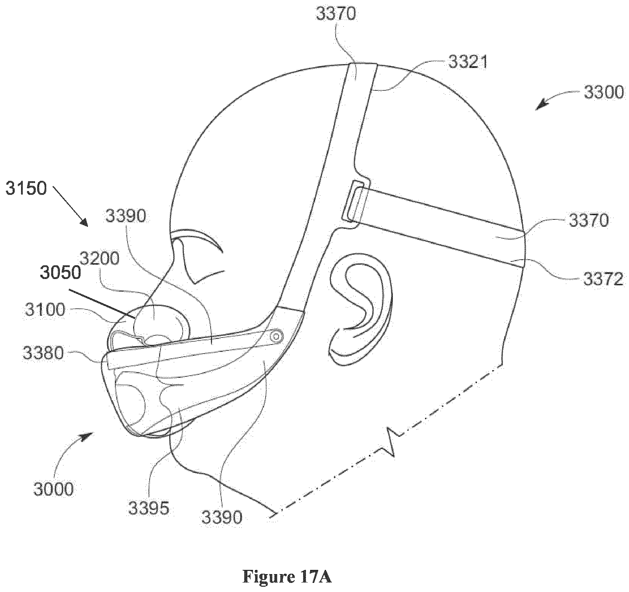

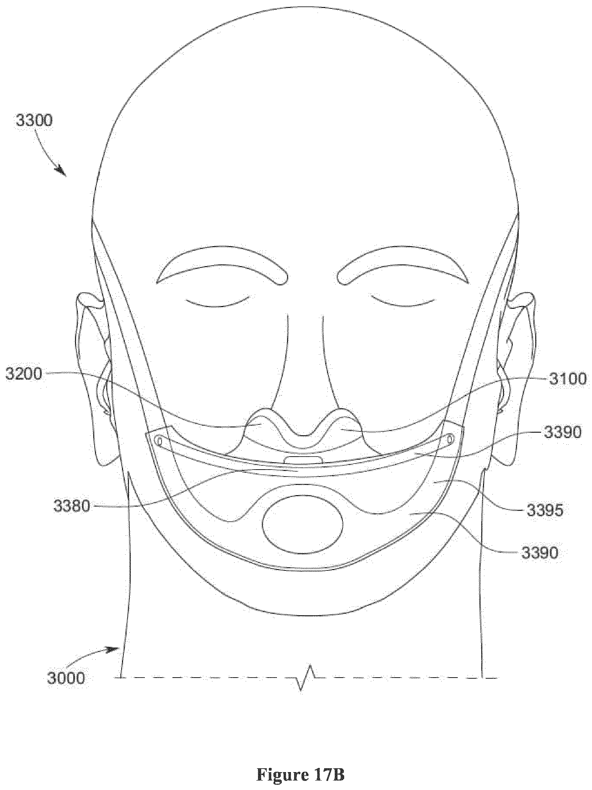

In the forms of the technology shown in Figures 17A and 17B, the positioning and stabilising structure 3300 may comprise a cover to cover one or more portions of the patient interface in use. For example, the cover may cover the anterior engagement portion 3380 and the side portions 3390. The positioning and stabilising structure 3300 may comprise a textile sheath 3395. The textile sheath 3395 may encapsulate or encase all or part of the anterior engagement portion 3390 and the side portions 3390. The textile sheath may attach to one or more non-patient facing surfaces of the anterior engagement portion 3380 and/or side portions 3390. In some forms this may be achieved by stitching or gluing the textile sheath to the non-patient facing surfaces, or via some other attachment mechanism. The cover or sheath may be removable or permanently attached. In some forms, the attachment mechanism may comprise one or more sections of hook and loop material. In some forms, the sections of hook and loop material are located at lateral regions of the textile sheath 3395 that overlie the cheek regions of the patient in use. The textile sheath 3395 may be formed from a single piece of material. The textile sheath 3395 may be flexible, for example the material from which it is formed may be relatively soft. The textile sheath 3395 may improve the comfort of the patient 1000 when using the patient interface 3000.

### 4.3.6 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO₂ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. For example, the vent 3400 may be located in a part of the cushion module 3150, for example in the cushion 3050 or the frame 3270. Alternatively, the vent 3400 is located in a decoupling structure, e.g., a swivel. Alternatively, the patient interface 3000 may comprise a plurality of vents 3400 located in a plurality of components.

### 4.3.7 Decoupling structure(s)

In one form the patient interface 3000 includes at least one decoupling structure, for example, a swivel or a ball and socket. The decoupling structure may take the form of an elbow 3610 adjustably, for example rotatably, connected to the plenum chamber inlet port 3254.

### 4.3.8 Connection port

Connection port 3600 allows for the plenum chamber 3200 to be fluidly connected to the air circuit 4170. In some forms, the plenum chamber inlet port 3254 and connection port 3600 may be provided by the same component, and may be the same opening. In other forms, the plenum chamber inlet port 3254 and connection port 3600 may be provided by different components and/or may be provided by different openings, but be fluidly connected.

### 4.3.9 Forehead support

In one form, the patient interface 3000 includes a forehead support 3700. In other forms, no forehead support is provided. For example, some forms of the technology may be sufficiently stable on the patient's face without a forehead support.

### 4.3.10 Anti-asphyxia valve

In one form, the patient interface 3000 includes an anti-asphyxia valve.

### 4.3.11 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 4.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

### 4.4.1 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

In other forms of the present technology, some portion or all of the algorithms 4300 may be implemented by a controller of an external device such as the local external device 4288 or the remote external device 4286. In such forms, data representing the input signals and / or intermediate algorithm outputs necessary for the portion of the algorithms 4300 to be executed at the external device may be communicated to the external device via the local external communication network 4284 or the remote external communication network 4282. In such forms, the portion of the algorithms 4300 to be executed at the external device may be expressed as computer programs, such as with processor control instructions to be executed by one or more processor(s), stored in a non-transitory computer readable storage medium accessible to the controller of the external device. Such programs configure the controller of the external device to execute the portion of the algorithms 4300.

In such forms, the therapy parameters generated by the external device via the therapy engine module 4320 (if such forms part of the portion of the algorithms 4300 executed by the external device) may be communicated to the central controller 4230 to be passed to the therapy control module 4330.

### 4.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

### 4.6 HUMIDIFIER

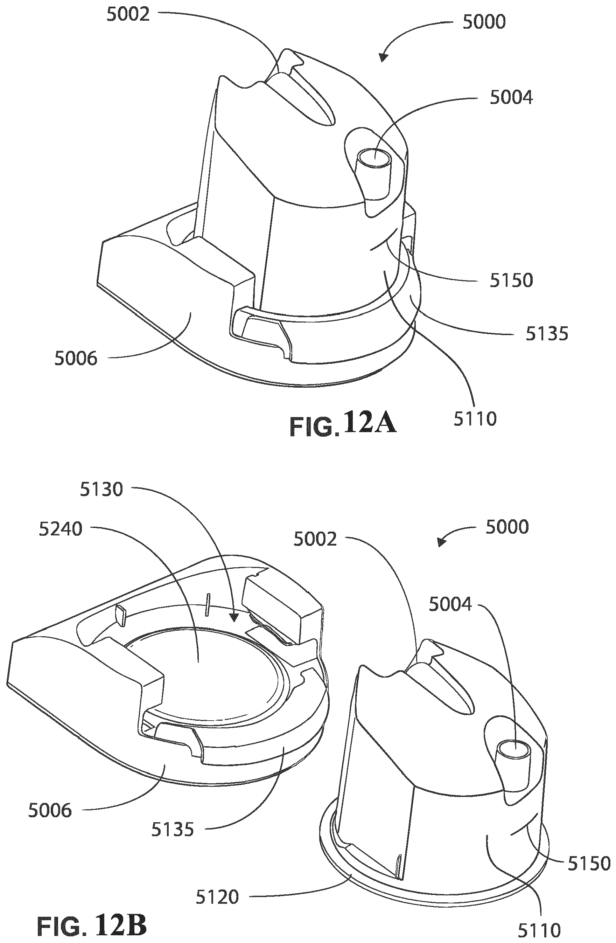

In one form of the present technology there is provided a humidifier 5000 (e.g. as shown in Figs. 1A, 1B, 12A and 12B) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

### 4.7 GLOSSARY

For the purposes of the present technology disclosure, in certain forms of the present technology, one or more of the following definitions may apply. In other forms of the present technology, alternative definitions may apply.

### 4.7.1 General

*Air:* In certain forms of the present technology, air may be taken to mean atmospheric air, and in other forms of the present technology air may be taken to mean some other combination of breathable gases, e.g. oxygen enriched air.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql*, is the flow rate of leak from a patient interface system or elsewhere. Respiratory flow rate, *Qr*, is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Oxygen enriched air:* Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

*Medical Oxygen:* Medical oxygen is defined as oxygen enriched air with an oxygen concentration of 80% or *greater.Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy:* The application of a supply of air to an entrance to the airways at a treatment pressure that is typically positive with respect to atmosphere.

*Ventilator:* A mechanical device that provides pressure support to a patient to perform some or all of the work of breathing.

### 4.7.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Coming. Another manufacturer of LSR is Wacker. Unless otherwise specified to the contrary, an exemplary form of LSR has a Shore A (or Type A) indentation hardness in the range of about 35 to about 45 as measured using ASTM D2240.

### Polycarbonate: a thermoplastic polymer of Bisphenol-A Carbonate.

### 4.7.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material per se to resist deformation (e.g. described by a Young's Modulus, or an indentation hardness scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness* (or *rigidity*) of a structure or component: The ability of the structure or component to resist deformation in response to an applied load. The load may be a force or a moment, e.g. compression, tension, bending or torsion. The structure or component may offer different resistances in different directions. The inverse of stiffness is flexibility.

*Floppy* structure or component: A structure or component that will change shape, e.g. bend, when caused to support its own weight, within a relatively short period of time such as 1 second.

*Rigid* structure or component: A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 4.7.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, Ti to total breath time, Ttot.

*Effort* (breathing): The work done by a spontaneously breathing person attempting to breathe.

*Expiratory portion* of a breathing cycle: The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) Flattened: Having a rise followed by a relatively flat portion, followed by a fall.
(ii) M-shaped: Having two local peaks, one at the leading edge, and one at the trailing edge, and a relatively flat portion between the two peaks.
(iii) Chair-shaped: Having a single local peak, the peak being at the leading edge, followed by a relatively flat portion.
(iv) Reverse-chair shaped: Having a relatively flat portion followed by single local peak, the peak being at the trailing edge.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea:* An increase in flow to a level higher than normal.

*Inspiratory portion* of a breathing cycle: The period from the start of inspiratory flow to the start of expiratory flow will be taken to be the inspiratory portion of a breathing cycle.

*Patency* (airway): The degree of the airway being open, or the extent to which the airway is open. A patent airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate* (*Qpeak*): The maximum value of flow rate during the inspiratory portion of the respiratory flow waveform.

*Respiratory flow rate, patient airflow rate, respiratory airflow rate* (*Qr*): These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume* (*Vt*): The volume of air inhaled or exhaled during normal breathing, when extra effort is not applied. In principle the inspiratory volume Vi (the volume of air inhaled) is equal to the expiratory volume *Ve* (the volume of air exhaled), and therefore a single tidal volume Vt may be defined as equal to either quantity. In practice the tidal volume *Vt* is estimated as some combination, e.g. the mean, of the inspiratory volume Vi and the expiratory volume *Ve.*

*Inhalation Time* (*Ti*): The duration of the inspiratory portion of the respiratory flow rate waveform.

*Exhalation Time* (*Te*): The duration of the expiratory portion of the respiratory flow rate waveform.

*Total Time* (*Ttot*): The total duration between the start of one inspiratory portion of a respiratory flow rate waveform and the start of the following inspiratory portion of the respiratory flow rate waveform.

*Typical recent ventilation:* The value of ventilation around which recent values of ventilation Vent over some predetermined timescale tend to cluster, that is, a measure of the central tendency of the recent values of ventilation.

*Upper airway obstruction* (*UAO*): includes both partial and total upper airway obstruction. This may be associated with a state of flow limitation, in which the flow rate increases only slightly or may even decrease as the pressure difference across the upper airway increases (Starling resistor behaviour).

*Ventilation* (*Vent*): A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 4.7.3 Anatomy

### 4.7.3.1 Anatomy of the face

### Ala: the external outer wall or "wing" of each nostril (plural: alar)

### Alar angle:

Alare: The most lateral point on the nasal ala.

Alar curvature (or alar crest) point: The most posterior point in the curved base line of each ala, found in the crease formed by the union of the ala with the cheek.

Auricle: The whole external visible part of the ear.

(nose) Bony framework: The bony framework of the nose comprises the nasal bones, the frontal process of the maxillae and the nasal part of the frontal bone.

(nose) Cartilaginous framework: The cartilaginous framework of the nose comprises the septal, lateral, major and minor cartilages.

Columella: the strip of skin that separates the nares and which runs from the pronasale to the upper lip.

Columella angle: The angle between the line drawn through the midpoint of the nostril aperture and a line drawn perpendicular to the Frankfort horizontal while intersecting subnasale.

Frankfort horizontal plane: A line extending from the most inferior point of the orbital margin to the left tragion. The tragion is the deepest point in the notch superior to the tragus of the auricle.

Glabella: Located on the soft tissue, the most prominent point in the midsagittal plane of the forehead.

Lateral nasal cartilage: A generally triangular plate of cartilage. Its superior margin is attached to the nasal bone and frontal process of the maxilla, and its inferior margin is connected to the greater alar cartilage.

### Lip, lower (labrale inferius):

### Lip, upper (labrale superius):

Greater alar cartilage: A plate of cartilage lying below the lateral nasal cartilage. It is curved around the anterior part of the naris. Its posterior end is connected to the frontal process of the maxilla by a tough fibrous membrane containing three or four minor cartilages of the ala.

Nares (Nostrils): Approximately ellipsoidal apertures forming the entrance to the nasal cavity. The singular form of nares is naris (nostril). The nares are separated by the nasal septum.

Naso-labial sulcus or Naso-labial fold: The skin fold or groove that runs from each side of the nose to the corners of the mouth, separating the cheeks from the upper lip.

Naso-labial angle: The angle between the columella and the upper lip, while intersecting subnasale.

Otobasion inferior: The lowest point of attachment of the auricle to the skin of the face.

Otobasion superior: The highest point of attachment of the auricle to the skin of the face.

Pronasale: the most protruded point or tip of the nose, which can be identified in lateral view of the rest of the portion of the head.

Philtrum: the midline groove that runs from lower border of the nasal septum to the top of the lip in the upper lip region.

Pogonion: Located on the soft tissue, the most anterior midpoint of the chin.

Ridge (nasal): The nasal ridge is the midline prominence of the nose, extending from the Sellion to the Pronasale.

Sagittal plane: A vertical plane that passes from anterior (front) to posterior (rear). The midsagittal plane is a sagittal plane that divides the body into right and left halves.

Sellion: Located on the soft tissue, the most concave point overlying the area of the frontonasal suture.

Septal cartilage (nasal): The nasal septal cartilage forms part of the septum and divides the front part of the nasal cavity.

Subalare: The point at the lower margin of the alar base, where the alar base joins with the skin of the superior (upper) lip.

Subnasal point: Located on the soft tissue, the point at which the columella merges with the upper lip in the midsagittal plane.

Supramenton: The point of greatest concavity in the midline of the lower lip between labrale inferius and soft tissue pogonion

### Anatomy of the skull

Frontal bone: The frontal bone includes a large vertical portion, the squama frontalis, corresponding to the region known as the forehead.

Mandible: The mandible forms the lower jaw. The mental protuberance is the bony protuberance of the jaw that forms the chin.

Maxilla: The maxilla forms the upper jaw and is located above the mandible and below the orbits. The frontal process of the maxilla projects upwards by the side of the nose, and forms part of its lateral boundary.

Nasal bones: The nasal bones are two small oblong bones, varying in size and form in different individuals; they are placed side by side at the middle and upper part of the face, and form, by their junction, the "bridge" of the nose.

Nasion: The intersection of the frontal bone and the two nasal bones, a depressed area directly between the eyes and superior to the bridge of the nose.

Occipital bone: The occipital bone is situated at the back and lower part of the cranium. It includes an oval aperture, the foramen magnum, through which the cranial cavity communicates with the vertebral canal. The curved plate behind the foramen magnum is the squama occipitalis.

Orbit: The bony cavity in the skull to contain the eyeball.

Parietal bones: The parietal bones are the bones that, when joined together, form the roof and sides of the cranium.

Temporal bones: The temporal bones are situated on the bases and sides of the skull, and support that part of the face known as the temple.

Zygomatic bones: The face includes two zygomatic bones, located in the upper and lateral parts of the face and forming the prominence of the cheek.

### 4.7.4 Patient interface

Anti-asphyxia valve (AAV): The component or sub-assembly of a mask system that, by opening to atmosphere in a failsafe manner, reduces the risk of excessive CO2 rebreathing by a patient.

Elbow: An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

Frame: Frame will be taken to mean a mask structure that bears the load of tension between two or more points of connection with a headgear. A mask frame may be a non-airtight load bearing structure in the mask. However, some forms of mask frame may also be air-tight.

### Functional dead space: (description to be inserted here)

Headgear: Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

Membrane: Membrane will be taken to mean a typically thin element that has, preferably, substantially no resistance to bending, but has resistance to being stretched.

Plenum chamber: a mask plenum chamber will be taken to mean a portion of a patient interface having walls at least partially enclosing a volume of space, the volume having air therein pressurised above atmospheric pressure in use. A shell may form part of the walls of a mask plenum chamber.

Seal: May be a noun form ("a seal") which refers to a structure, or a verb form ("to seal") which refers to the effect. Two elements may be constructed and/or arranged to 'seal' or to effect 'sealing' therebetween without requiring a separate 'seal' element per se.

Shell: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

Stiffener: A stiffener will be taken to mean a structural component designed to increase the bending resistance of another component in at least one direction.

Strut: A strut will be taken to be a structural component designed to increase the compression resistance of another component in at least one direction.

Swivel (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

Tie (noun): A structure designed to resist tension.

Vent: (noun): A structure that allows a flow of air from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 4.7.5 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 4.7.5.1 Curvature in one dimension

The curvature of a plane curve at p may be described as having a sign (e.g. positive, negative) and a magnitude (e.g. 1/radius of a circle that just touches the curve at p).

Positive curvature: If the curve at p turns towards the outward normal, the curvature at that point will be taken to be positive (if the imaginary small person leaves the point p they must walk uphill). See Fig. 3B (relatively large positive curvature compared to Fig. 3C) and Fig. 3C (relatively small positive curvature compared to Fig. 3B). Such curves are often referred to as concave.

Zero curvature: If the curve at p is a straight line, the curvature will be taken to be zero (if the imaginary small person leaves the point p, they can walk on a level, neither up nor down). See Fig. 3D.

Negative curvature: If the curve at p turns away from the outward normal, the curvature in that direction at that point will be taken to be negative (if the imaginary small person leaves the point p they must walk downhill). See Fig. 3E (relatively small negative curvature compared to Fig. 3F) and Fig. 3F (relatively large negative curvature compared to Fig. 3E). Such curves are often referred to as convex.

### 4.7.5.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

Principal curvatures and directions: The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at p are the curvatures in the principal directions.

Region of a surface: A connected set of points on a surface. The set of points in a region may have similar characteristics, e.g. curvatures or signs.

Saddle region: A region where at each point, the principal curvatures have opposite signs, that is, one is positive, and the other is negative (depending on the direction to which the imaginary person turns, they may walk uphill or downhill).

Dome region: A region where at each point the principal curvatures have the same sign, e.g. both positive (a "concave dome") or both negative (a "convex dome").

Cylindrical region: A region where one principal curvature is zero (or, for example, zero within manufacturing tolerances) and the other principal curvature is non-zero.

Planar region: A region of a surface where both of the principal curvatures are zero (or, for example, zero within manufacturing tolerances).

Edge of a surface: A boundary or limit of a surface or region.

Path: In certain forms of the present technology, 'path' will be taken to mean a path in the mathematical - topological sense, e.g. a continuous space curve from f(0) to f(1) on a surface. In certain forms of the present technology, a 'path' may be described as a route or course, including e.g. a set of points on a surface. (The path for the imaginary person is where they walk on the surface, and is analogous to a garden path).

Path length: In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

Straight-line distance: The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 4.7.5.3 Space curves

Space curves: Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

Tangent unit vector (or unit tangent vector): For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

Unit normal vector: As the imaginary person moves along the curve, this tangent vector itself changes. The unit vector pointing in the same direction that the tangent vector is changing is called the unit principal normal vector. It is perpendicular to the tangent vector.

Binormal unit vector: The binormal unit vector is perpendicular to both the tangent vector and the principal normal vector. Its direction may be determined by a right-hand rule (see e.g. Fig. 3P), or alternatively by a left-hand rule (Fig. 3O).

Osculating plane: The plane containing the unit tangent vector and the unit principal normal vector. See Figures 3O and 3P.

Torsion of a space curve: The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 4.7.5.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 4.8 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein are incorporated herein by reference in their entirety to disclose and describe the methods and/or materials which are the subject of those publications. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

### 4.9 REFERENCE SIGNS LIST

| | |
|---|---|
| patient | 1000 |
| bed partner | 1100 |
| patient interface | 3000 |
| cushion | 3050 |
| mouth portion | 3050-1 |
| nasal portion | 3050-2 |
| posterior wall portion | 3052 |
| anterior wall portion | 3054 |
| recess | 3060 |
| opening | 3070 |
| seal-forming structure | 3100 |
| cushion module | 3150 |
| plenum chamber | 3200 |
| plenum chamber | 3200-1 |
| plenum chamber | 3200-2 |
| anterior portion | 3201 |
| non-patient-facing surface | 3202 |
| groove | 3203 |
| second non-patient-facing surface | 3204 |
| plenum chamber inlet port | 3254 |
| plenum chamber inlet port | 3254-1 |
| plenum chamber inlet port | 3254-2 |
| groove | 3266-1 |
| groove | 3266-2 |
| frame | 3270 |
| positioning and stabilising structure | 3300 |
| headgear | 3302 |
| headgear | 3302-1 |
| headgear | 3302-2 |
| rear strap | 3307-1 |
| rear strap | 3307-2 |
| slit | 3309-2 |
| strap | 3310 |
| tab | 3320 |
| rigidiser arm | 3340 |
| first end | 3342 |
| second end | 3344 |
| tube | 3350 |
| sleeve | 3351 |
| extendable concertina structure | 3362 |
| non-extendable tube sections | 3363 |
| superior portion | 3370 |
| crown portion | 3371 |
| rear portion | 3372 |
| anterior engagement portion | 3380 |
| patient-facing surface | 3381 |
| surface portion | 3382 |
| second patient-facing surface | 3383 |
| side portions | 3390 |
| first side portion | 3391 |
| second side portion | 3392 |
| connection | 3393 |
| connection points | 3394 |
| textile sheath | 3395 |
| vent | 3400 |
| vent opening | 3402 |
| vent opening | 3402-1 |
| vent opening | 3402-2 |
| arm connection structure | 3504 |
| connection port | 3600 |
| elbow | 3610 |
| forehead support | 3700 |
| brace | 3900 |
| patient-facing surface | 3905 |
| non-patient-facing surface | 3910 |
| superior surface | 3912 |
| inferior surface | 3914 |
| first end | 3920 |
| second end | 3925 |
| line | 3950 |
| RPT device | 4000 |
| external housing | 4010 |
| upper portion | 4012 |
| lower portion | 4014 |
| panel(s) | 4015 |
| chassis | 4016 |
| handle | 4018 |
| pneumatic block | 4020 |
| pneumatic components | 4100 |
| air filters | 4110 |
| inlet air filter | 4112 |
| outlet air filter | 4114 |
| muffler | 4120 |
| inlet muffler | 4122 |
| outlet muffler | 4124 |
| pressure generator | 4140 |
| blower | 4142 |
| brushless DC motor | 4144 |
| anti-spill back valve | 4160 |
| air circuit | 4170 |
| supplemental oxygen | 4180 |
| electrical components | 4200 |
| Printed Circuit Board Assembly (PCBA) | 4202 |
| power supply | 4210 |
| input devices | 4220 |
| transducers | 4270 |
| humidifier | 5000 |
| humidifier inlet | 5002 |
| humidifier outlet | 5004 |
| humidifier base | 5006 |
| reservoir | 5110 |
| conductive portion | 5120 |
| humidifier reserve dock | 5130 |
| locking lever | 5135 |
| water level indicator | 5150 |
| heating element | 5240 |

## Claims

1. A patient interface (3000) for supplying a flow of air at a therapeutic pressure to a patient's airways, the patient interface (3000) comprising:
a cushion module (3150) comprising a cushion (3050), wherein the cushion (3050) is constructed and configured to be substantially flexible; and
a brace (3900),
wherein the cushion module (3150) at least in part defines a plenum chamber (3200) pressurisable to the therapeutic pressure and comprising a plenum chamber inlet port (3254) for receiving the flow of air into the plenum chamber (3200),
wherein the cushion (3050) comprises a seal-forming structure (3100) constructed and configured to form a seal with a region of the patient's face surrounding an entrance to the patient's nares, said seal-forming structure (3100) having an opening therein such that the flow of air at said therapeutic pressure is delivered to the patient's nares in use, the seal-forming structure (3100) constructed and configured to maintain said therapeutic pressure in the plenum chamber (3200) throughout the patient's respiratory cycle in use, and
wherein the cushion (3050) comprises a nasal portion (3050-2) configured to be positioned, in use, proximate and anterior to a nasal and/or lip superior region of the patient's face,
wherein the cushion module (3150) is configured to connect, in use, to a positioning and stabilising structure (3300) to provide a force to hold the seal-forming structure (3100) in a therapeutically effective position on the patient's head,
wherein the brace (3900) is constructed and configured to be more rigid than the nasal portion (3050-2) of the cushion (3050),
wherein the brace (3900) is configured to mount to the nasal portion (3050-2) of the cushion (3050) and, in use, to brace against movement of portions of the seal-forming structure (3100) in sealing engagement with regions of the patient's face proximate the patient's nares and
**characterized in that** the brace (3900) is configured to deform a seal-forming surface of the nasal portion (3050-2) relative to a natural shape of the seal-forming surface of the nasal portion (3050-2).

2. A patient interface (3000) as claimed in claim 1, wherein the brace (3900) comprises a concave patient-facing surface (3905) facing towards the patient in use.

3. A patient interface (3000) as claimed in claim 2, wherein the brace (3900) comprises a convex non-patient-facing surface (3910) facing away from the patient in use.

4. A patient interface (3000) as claimed in claim 3, wherein the brace (3900) is arc-shaped when viewed in a direction perpendicular to the transverse plane of the patient.

5. A patient interface (3000) as claimed in any one of claims 1 to 4, wherein the brace (3900) is elongate and the brace (3900) is configured to mount to the nasal portion (3050-2) of the cushion (3050) in an orientation such that the brace (3900) is elongate in the lateral direction with respect to the patient in use.

6. A patient interface (3000) as claimed in any one of claims 1 to 5, wherein the brace (3900) is shaped as an arc-shaped rectangular prism.

7. A patient interface (3000) as claimed in any one of claims 1 to 6, wherein the seal-forming structure (3100) is constructed and configured to form a seal with a region of the patient's face surrounding an entrance to the patient's mouth such that the flow of air is delivered to the patient's mouth in use.

8. A patient interface (3000) as claimed in any one of claims 1 to 7, wherein the cushion module (3150) comprises a frame (3270), wherein the frame (3270) is constructed and configured to be more rigid than the nasal portion (3050-2) of the cushion (3050).

9. A patient interface (3000) as claimed in claim 8, wherein the frame (3270) at least in part defines the plenum chamber (3200).

10. A patient interface (3000) as claimed in any one of claims 8 to 9, wherein the frame (3270) comprises a frame opening forming the plenum chamber inlet port (3254).

11. A patient interface (3000) as claimed in claim 10, wherein the frame opening is positioned in an anterior central region of the patient interface (3000) when worn.

12. A patient interface (3000) as claimed in any one of claims 1 to 11, wherein the patient interface (3000) comprises the positioning and stabilising structure (3300) to provide a force to hold the seal-forming structure (3100) in a therapeutically effective position on the patient's head.

13. A patient interface (3000) as claimed in claim 12, wherein the positioning and stabilising structure (3300) comprises one or more tubes (3350) configured to convey the flow of air at the therapeutic pressure from a connection port (3600) to the plenum chamber inlet port (3254), wherein the connection port (3600) is positioned, in use, in a region of the patient's head superior to an otobasion superior of the patient's head an otobasion superior of the patient's head.

14. A patient interface assembly, the patient interface assembly comprising:
a patient interface (3000) according to any one of claims 1 to 13; and
at least one additional brace (3900) constructed and configured to be more rigid than the nasal portion (3050-2) of the cushion (3050);
wherein the additional brace (3900) is configured to mount to the nasal portion (3050-2) of the cushion (3050) and, in use, to brace against movement of portions of the seal-forming structure (3100) in sealing engagement with regions of the patient's face proximate the patient's nares;
wherein the brace (3900) and the additional brace (3900) may be interchangeably mounted to the cushion module (3150); and
wherein the brace (3900) and the additional brace (3900) are shaped and/or sized differently from each other.

## Patentansprüche

1. Patientenschnittstelle (3000) zum Zuführen eines Luftstroms mit einem therapeutischen Druck zu den Atemwegen eines Patienten, wobei die PatientenSchnittstelle (3000) aufweist:
ein Polstermodul (3150) mit einem Polster (3050), wobei das Polster (3050) derart konstruiert und konfiguriert ist, dass es im Wesentlichen flexibel ist; und
ein Versteifungselement (3900),
wobei das Polstermodul (3150) zumindest teilweise eine Plenumkammer (3200) definiert, die auf den therapeutischen Druck druckbeaufschlagbar ist, und eine Plenumkammereinlassöffnung (3254) zum Empfangen des in die Plenumkammer (3200) strömenden Luftstroms aufweist,
wobei das Polster (3050) eine dichtungsbildende Struktur (3100) aufweist, die derart konstruiert und konfiguriert ist, dass eine Dichtung mit einem Bereich des Gesichts des Patienten gebildet wird, der einen Eingang zu den Nasenlöchern des Patienten umgibt, wobei die dichtungsbildende Struktur (3100) eine darin ausgebildete Öffnung aufweist, so dass der Luftstrom mit dem therapeutischen Druck im Gebrauch den Nasenlöchern des Patienten zugeführt wird, wobei die dichtungsbildende Struktur (3100) derart konstruiert und konfiguriert ist, dass der therapeutische Druck in der Plenumkammer (3200) während des gesamten Atemzyklus des Patienten im Gebrauch aufrechterhalten wird, und
wobei das Polster (3050) einen Nasenabschnitt (3050-2) aufweist, der dafür konfiguriert ist, im Gebrauch in der Nähe und vor einem Nasenbereich und/oder einem Bereich über den Lippen des Gesichts des Patienten angeordnet zu werden,
wobei das Polstermodul (3150) dafür konfiguriert ist, im Gebrauch mit einer Positionierungs- und Stabilisierungsstruktur (3300) verbunden zu werden, um eine Kraft zum Halten der dichtungsbildenden Struktur (3100) in einer therapeutisch wirksamen Position auf dem Kopf des Patienten bereitzustellen,
wobei das Versteifungselement (3900) derart konstruiert und konfiguriert ist, dass es steifer ist als der Nasenabschnitt (3050-2) des Polsters (3050),
wobei das Versteifungselement (3900) dafür konfiguriert ist, am Nasenabschnitt (3050-2) des Polsters (3050) befestigt zu werden und im Gebrauch eine Absteifung gegen eine Bewegung von Abschnitten der dichtungsbildenden Struktur (3100) bereitzustellen, die im Dichteingriff mit Bereichen des Gesichts des Patienten in der Nähe der Nasenlöcher des Patienten stehen, und
**dadurch gekennzeichnet, dass**
das Versteifungselement (3900) dafür konfiguriert ist, eine dichtungsbildende Oberfläche des Nasenabschnitts (3050-2) relativ zu einer natürlichen Form der dichtungsbildenden Oberfläche des Nasenabschnitts (3050-2) zu verformen.

2. Patientenschnittstelle (3000) nach Anspruch 1, wobei das Versteifungselement (3900) eine konkave, dem Patienten zugewandte Oberfläche (3905) aufweist, die im Gebrauch zum Patienten hin gewandt ist.

3. Patientenschnittstelle (3000) nach Anspruch 2, wobei das Versteifungselement (3900) eine konvexe, nicht dem Patienten zugewandte Oberfläche (3910) aufweist, die im Gebrauch vom Patienten weg gewandt ist.

4. Patientenschnittstelle (3000) nach Anspruch 3, wobei das Versteifungselement (3900) in einer Richtung senkrecht zur Transversalebene des Patienten betrachtet bogenförmig ist.

5. Patientenschnittstelle (3000) nach einem der Ansprüche 1 bis 4, wobei das Versteifungselement (3900) länglich ist und das Versteifungselement (3900) dafür konfiguriert ist, am Nasenabschnitt (3050-2) des Polsters (3050) in einer derartigen Ausrichtung montiert zu werden, dass das Versteifungselement (3900) im Gebrauch in der lateralen Richtung in Bezug auf den Patienten länglich ist.

6. Patientenschnittstelle (3000) nach einem der Ansprüche 1 bis 5, wobei das Versteifungselement (3900) als ein bogenförmiges rechteckiges Prisma geformt ist.

7. Patientenschnittstelle (3000) nach einem der Ansprüche 1 bis 6, wobei die dichtungsbildende Struktur (3100) derart konstruiert und konfiguriert ist, dass eine Abdichtung mit einem Bereich des Gesichts des Patienten gebildet wird, der einen Eingang zum Mund des Patienten umgibt, so dass der Luftstrom im Gebrauch dem Mund des Patienten zugeführt wird.

8. Patientenschnittstelle (3000) nach einem der Ansprüche 1 bis 7, wobei das Polstermodul (3150) einen Rahmen (3270) aufweist, wobei der Rahmen (3270) derart konstruiert und konfiguriert ist, dass er steifer ist als der Nasenabschnitt (3050-2) des Polsters (3050).

9. Patientenschnittstelle (3000) nach Anspruch 8, wobei der Rahmen (3270) zumindest teilweise die Plenumkammer (3200) definiert.

10. Patientenschnittstelle (3000) nach Anspruch 8 oder 9, wobei der Rahmen (3270) eine Rahmenöffnung aufweist, die die Plenumkammereinlassöffnung (3254) bildet.

11. Patientenschnittstelle (3000) nach Anspruch 10, wobei die Rahmenöffnung in einem vorderen mittleren Bereich der Patientenschnittstelle (3000) angeordnet ist, wenn sie getragen wird.

12. Patientenschnittstelle (3000) nach einem der Ansprüche 1 bis 11, wobei die Patientenschnittstelle (3000) die Positionierungs- und Stabilisierungsstruktur (3300) zum Bereitstellen einer Kraft aufweist, die die dichtungsbildende Struktur (3100) in einer therapeutisch wirksamen Position an dem Kopf des Patienten hält.

13. Patientenschnittstelle (3000) nach Anspruch 12, wobei die Positionierungs- und Stabilisierungsstruktur (3300) ein oder mehrere Rohre (3350) aufweist, die dafür konfiguriert sind, den Luftstrom mit dem therapeutischen Druck von einer Verbindungsöffnung (3600) zur Plenumkammereinlassöffnung (3254) zu leiten, wobei die Verbindungsöffnung (3600) im Gebrauch in einem Bereich des Kopfs des Patienten oberhalb einer Otobasion superior des Kopfs des Patienten angeordnet ist.

14. Patientenschnittstellenanordnung, wobei die Patientenschnittstellenanordnung aufweist:
eine Patientenschnittstelle (3000) nach einem der Ansprüche 1 bis 13; und
mindestens ein zusätzliches Versteifungselement (3900), das derart konstruiert und konfiguriert ist, dass es steifer ist als der Nasenabschnitt (3050-2) des Polsters (3050),
wobei das zusätzliche Versteifungselement (3900) dafür konfiguriert ist, am Nasenabschnitt (3050-2) des Polsters (3050) montiert zu werden und im Gebrauch eine Absteifung gegen eine Bewegung von Abschnitten der dichtungsbildenden Struktur (3100) bereitstellt, die mit Bereichen des Gesichts des Patienten in der Nähe der Nasenlöcher des Patienten in Dichteingriff stehen,
wobei das Versteifungselement (3900) und das zusätzliche Versteifungselement (3900) austauschbar am Polstermodul (3150) montiert sein können, und
wobei das Versteifungselement (3900) und das zusätzliche Versteifungselement (3900) unterschiedlich geformt und/oder dimensioniert sind.

## Revendications

1. Interface patient (3000) pour fournir un flux d'air à une pression thérapeutique aux voies aériennes d'un patient, l'interface patient (3000) comprenant :
un module de coussin (3150) comprenant un coussin (3050), dans laquelle le coussin (3050) est construit et configuré pour être sensiblement flexible ; et
une entretoise (3900),
dans laquelle le module de coussin (3150) définit au moins en partie une chambre de répartition (3200) pressurisable à la pression thérapeutique et comprenant un orifice d'entrée de chambre de répartition (3254) pour recevoir le flux d'air dans la chambre de répartition (3200),
dans laquelle le coussin (3050) comprend une structure formant étanchéité (3100) construite et configurée pour former une étanchéité par rapport à une région du visage du patient entourant une entrée des narines du patient, ladite structure formant étanchéité (3100) comportant une ouverture de sorte que le flux d'air à ladite pression thérapeutique soit distribué aux narines du patient lors de l'utilisation, la structure formant étanchéité (3100) étant construite et configurée pour maintenir ladite pression thérapeutique dans la chambre de répartition (3200) tout au long du cycle respiratoire du patient lors de l'utilisation, et
dans laquelle le coussin (3050) comprend une partie nasale (3050-2) configurée pour être positionnée, lors de l'utilisation, proximale et antérieure à une région nasale et/ou supérieure labiale du visage du patient,
dans laquelle le module de coussin (3150) est configuré pour se relier, lors de l'utilisation, à une structure de positionnement et de stabilisation (3300) pour fournir une force permettant de maintenir la structure formant étanchéité (3100) dans une position thérapeutiquement efficace sur la tête du patient,
dans laquelle l'entretoise (3900) est construite et configurée pour être plus rigide que la partie nasale (3050-2) du coussin (3050),
dans laquelle l'entretoise (3900) est configurée pour se monter sur la partie nasale (3050-2) du coussin (3050) et, lors de l'utilisation, pour s'opposer au mouvement de parties de la structure formant étanchéité (3100) en prise étanche avec des régions du visage du patient proches des narines du patient et
**caractérisée en ce que** l'entretoise (3900) est configurée pour déformer une surface formant étanchéité de la partie nasale (3050-2) par rapport à une forme naturelle de la surface formant étanchéité de la partie nasale (3050-2).

2. Interface patient (3000) selon la revendication 1, dans laquelle l'entretoise (3900) comprend une surface concave orientée vers le patient (3905) orientée vers le patient lors de l'utilisation.

3. Interface patient (3000) selon la revendication 2, dans laquelle l'entretoise (3900) comprend une surface convexe non orientée vers le patient (3910) orientée à l'opposé du patient lors de l'utilisation.

4. Interface patient (3000) selon la revendication 3, dans laquelle l'entretoise (3900) est en forme d'arc lorsqu'elle est vue dans une direction perpendiculaire au plan transversal du patient.

5. Interface patient (3000) selon l'une quelconque des revendications 1 à 4, dans laquelle l'entretoise (3900) est allongée et l'entretoise (3900) est configurée pour se monter sur la partie nasale (3050-2) du coussin (3050) dans une orientation telle que l'entretoise (3900) soit allongée dans la direction latérale par rapport au patient lors de l'utilisation.

6. Interface patient (3000) selon l'une quelconque des revendications 1 à 5, dans laquelle l'entretoise (3900) est en forme de prisme rectangulaire arqué.

7. Interface patient (3000) selon l'une quelconque des revendications 1 à 6, dans laquelle la structure formant étanchéité (3100) est construite et configurée pour former une étanchéité avec une région du visage du patient entourant une entrée dans la bouche du patient de sorte que le flux d'air soit délivré à la bouche du patient lors de l'utilisation.

8. Interface patient (3000) selon l'une quelconque des revendications 1 à 7, dans laquelle le module de coussin (3150) comprend un cadre (3270), dans laquelle le cadre (3270) est construit et configuré pour être plus rigide que la partie nasale (3050-2) du coussin (3050).

9. Interface patient (3000) selon la revendication 8, dans laquelle le cadre (3270) définit au moins en partie la chambre de répartition (3200).

10. Interface patient (3000) selon l'une quelconque des revendications 8 et 9, dans laquelle le cadre (3270) comprend une ouverture de cadre formant l'orifice d'entrée de chambre de répartition (3254).

11. Interface patient (3000) selon la revendication 10, dans laquelle l'ouverture de cadre est positionnée dans une région centrale antérieure de l'interface patient (3000) lorsqu'elle est portée.

12. Interface patient (3000) selon l'une quelconque des revendications 1 à 11, dans laquelle l'interface patient (3000) comprend la structure de positionnement et de stabilisation (3300) pour fournir une force permettant de maintenir la structure formant étanchéité (3100) dans une position thérapeutiquement efficace sur la tête du patient.

13. Interface patient (3000) selon la revendication 12, dans laquelle la structure de positionnement et de stabilisation (3300) comprend un ou plusieurs tubes (3350) configurés pour transporter le flux d'air à la pression thérapeutique d'un orifice de connexion (3600) vers l'orifice d'entrée de chambre de répartition (3254), dans laquelle l'orifice de connexion (3600) est positionné, lors de l'utilisation, dans une région de la tête du patient supérieure à un otobasion supérieur de la tête du patient.

14. Ensemble d'interface patient, l'ensemble d'interface patient comprenant :
une interface patient (3000) selon l'une quelconque des revendications 1 à 13 ; et
au moins une entretoise supplémentaire (3900) construite et configurée pour être plus rigide que la partie nasale (3050-2) du coussin (3050) ;
dans lequel l'entretoise supplémentaire (3900) est configurée pour se monter sur la partie nasale (3050-2) du coussin (3050) et, lors de l'utilisation, pour s'opposer au mouvement de parties de la structure formant étanchéité (3100) en prise étanche avec des régions du visage du patient proches des narines du patient ;
dans lequel l'entretoise (3900) et l'entretoise supplémentaire (3900) peuvent être montées de manière interchangeable sur le module de coussin (3150) ; et
dans lequel l'entretoise (3900) et l'entretoise supplémentaire (3900) ont une forme et/ou une taille différentes l'une de l'autre.
